(19) Europäisches Patentamt
European Patent Office
Office européen des brevets

(11) EP 4 317 164 A1

(12) EUROPEAN PATENT APPLICATION
published in accordance with Art. 153(4) EPC

(43) Date of publication:
**07.02.2024 Bulletin 2024/06**

(21) Application number: **22778888.2**

(22) Date of filing: **28.03.2022**

(51) International Patent Classification (IPC):
***C07D 495/06*** (2006.01) ***C07D 491/052*** (2006.01)
***C07D 493/04*** (2006.01) ***A61K 31/35*** (2006.01)
***A61K 31/381*** (2006.01) ***A61P 25/18*** (2006.01)
***A61P 25/20*** (2006.01) ***A61P 25/22*** (2006.01)
***A61P 25/24*** (2006.01) ***A61P 25/28*** (2006.01)

(52) Cooperative Patent Classification (CPC):
**A61K 31/35; A61K 31/381; A61P 25/18; A61P 25/20; A61P 25/22; A61P 25/24; A61P 25/28; C07D 491/052; C07D 493/04; C07D 495/06;** Y02P 20/55

(86) International application number:
**PCT/CN2022/083485**

(87) International publication number:
**WO 2022/206706 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2021 CN 202110338429**
**29.09.2021 CN 202111151734**
**09.12.2021 CN 202111496634**

(71) Applicants:
- **Shujing Biopharma Co., Ltd**
  **Shanghai 201208 (CN)**
- **Jiangsu NHWA Pharmaceutical Co., Ltd**
  **Xuzhou, Jiangsu 221000 (CN)**

(72) Inventors:
- **QIN, Jun**
  **Shanghai 201208 (CN)**
- **WU, Yongqi**
  **Shanghai 201208 (CN)**
- **FENG, Jiaquan**
  **Shanghai 201208 (CN)**
- **HUANG, Wei**
  **Shanghai 201208 (CN)**
- **ZOU, Yuanhai**
  **Shanghai 201208 (CN)**
- **ZHOU, Bingcheng**
  **Shanghai 201208 (CN)**
- **HU, Zhijing**
  **Shanghai 201208 (CN)**
- **JIANG, Xinjian**
  **Shanghai 201208 (CN)**
- **WAN, Zehong**
  **Shanghai 201208 (CN)**

(74) Representative: **Lavoix**
**Bayerstraße 83**
**80335 München (DE)**

(54) **SPIRO-CONTAINING DERIVATIVE, AND PREPARATION METHOD THEREFOR AND USE THEREOF**

(57) Provided are a spiro-containing derivative, and a preparation method therefor and the use thereof. In particular, provided are a compound as shown in general formula (I), and a preparation method therefor, a pharmaceutical composition containing same, and the use thereof in the preparation of a drug for preventing and/or treating central nervous system diseases related to 5-hydroxytryptamine receptors and/or trace amine-related receptors and/or dopamine receptors in mammals.

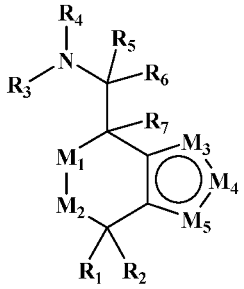

( I )

## Description

The present application claims the priority of the Chinese patent application No. 2021103384291 filed on March 29, 2021, the priority of the Chinese patent application No. 2021111517346 filed on September 29, 2021, and the priority of the Chinese patent application No. 2021114966347 filed on December 09, 2021. The full texts of the above-mentioned Chinese patent applications are cited by the present application.

## TECHNICAL FIELD

The present application belongs to the field of pharmaceutical chemistry, and particularly relates to a spirocycle-containing derivative, a preparation method and application thereof. More particularly, the present application relates to a spirocycle-containing derivative, a preparation method thereof, a pharmaceutical composition comprising the spirocycle-containing derivative, and use of the spirocycle-containing derivative or the pharmaceutical composition in the preparation of a medicament for preventing and/or treating a neuropsychiatric disease in mammals.

## BACKGROUND ART

Diseases related to central nervous system affect a large population in varying degrees. In general, the main features of such diseases include marked impairment of cognition or memory, and a marked deterioration in relative to the original level of function. Schizophrenia is a psychopathic disorder of unknown origin that usually begins by early adulthood, characterized by psychotic symptoms, stage progression and development, and/or regression in social behavior and professional ability. Symptoms of schizophrenia are generally manifested in three major categories: positive symptoms, negative symptoms, and cognitive symptoms. Positive symptoms are symptoms that manifest as "excessive" normal experiences, such as hallucinations and delusions. Negative symptoms are symptoms of a lack of normal experiences, such as anhedonia and a lack of social interaction. Cognitive symptoms, such as lack of sustained attention and poor decision-making, are associated with cognitive impairment in schizophrenia. Current anti-psychotic drugs can successfully treat positive symptoms, but are far from ideal for negative and cognitive symptoms.

Biogenic amines play important roles as neurotransmitters in the central and peripheral nervous systems. The synthesis and storage of biogenic amines, as well as their degradation and reabsorption after release, are tightly controlled. Imbalances in biogenic amine levels are known to be a major cause of brain function changes in many pathological conditions. Serotonin, norepinephrine, epinephrine, dopamine, and histamine have been extensively studied as classic biogenic amines. Particularly, the 5-hydroxytryptamine system plays an important role in regulating the functions of prefrontal cortex (PFC), including emotional control, cognitive behavior, and working memory. The pyramidal neurons and GABA interneurons of PFC comprise several 5-hydroxytryptamine receptor subtypes 5-HT1A and 5-HT2A with particularly high density. It has recently been demonstrated that PFC and NMDA receptor channels are targets of 5-HT1AR, and these two receptors regulate excitatory neurons in the cerebral cortex, thereby affecting cognitive function. In fact, various preclinical data indicate that 5-HT1AR may be a new target for the development of anti-psychotic drugs. The high affinity of atypical anti-psychotic drugs (such as olanzapine, aripiprazole, etc.) to 5-HT1AR and their low EPS side effects indicate that the 5-hydroxytryptamine system plays an important role in regulating the functions of PFC, including emotional control, cognitive behavior and working memory. The pyramidal neurons and GABA interneurons of PFC comprise several 5-hydroxytryptamine receptor subtypes 5-HT1A and 5-HT2A with particularly high density. Recent studies have shown that 5-HT1A agonists are associated with atypical anti-psychotic drugs treatment and can improve negative symptoms and cognitive disorder.

In recent years, with the gradual deepening of the research on classical biogenic amines, people have found a second type of endogenous amine compounds, namely trace amines TA, including p-tyramine, β-phenylethylamine, tryptamine and octopamine. The content level of these compounds in the mammalian nervous system is generally lower than that of classical biogenic amines, but they share similar characteristics with classical biogenic amines in terms of structure, metabolism, and subcellular localization. Trace amine-associated receptor TAAR, a new member of G protein-coupled receptors (GPCR), shares similar structure and consistent pharmacological data with the pharmacophore penetrating into GPCR. The phylogenetic relationship of this receptor gene indicates that these receptors form three distinct subfamilies, of which TAAR1 is the first subfamily of the four genes (TAAR1-4) highly conserved between humans and rodents. TA activates TAAR1 through Gαs and plays a role. Existing studies have shown that dysregulation of trace amine-associated receptors, especially TAAR1, is closely related to many psychiatric diseases such as schizophrenia and depression as well as other conditions such as attention deficit hyperactivity disorder, migraine, Parkinson's disease, substance abuse and eating disorder. Therefore, TAAR ligands have high potential for the treatment of these diseases.

Although there are many anti-schizophrenia drugs, there are still a variety of adverse reactions of schizophrenic drugs in clinical application. In addition, although the current anti-schizophrenia negative symptom drugs have been applied clinically and improved the negative symptoms in some patients, the overall effect is limited. There are still many

patients who cannot recover and repair normal social functions due to negative symptoms, resulting in difficulties in restoring normal social work. In addition, the treatment of cognitive disorder is also a key point in the treatment of schizophrenia at present, which affects the verbal memory, semantic processing ability and attention function of most patients with schizophrenia. However, the improvement of cognitive function by anti-psychotic drugs currently being researched or marketed is very limited. In addition, the treatment of refractory schizophrenia is still in a dilemma. Such patients have been treated with three anti-psychotic drugs with different active ingredients, with a sufficient quantity of drugs and sufficient treatment duration but poor treatment response or inability to tolerate adverse reactions of drugs, or even with sufficient maintenance or preventive treatment, the condition continues to recur or worsen. Therefore, therapeutic drugs for refractory schizophrenia have always been a challenge in clinical drug research and an urgent direction to be overcome.

Currently, Sunovion's SEP-363856, which is in phase III clinical research, as a 5-hydroxytryptamine and/or trace amine-associated receptor agonist, has significant effects on 5-HT1A and TAAR1 receptors and has shown good activity in present clinical study. Therefore, there is an urgent need to develop an anti-schizophrenia drug that has good, sustained and effective treatment effects for negative symptoms, can improve cognitive function of patients, can effectively treat refractory schizophrenia, and in addition, has low adverse drug reactions and acts on multiple targets, in order to meet the huge market demand.

## BRIEF DESCRIPTION

The technical problem to be solved by the present application is to provide a spirocycle-containing derivative, a preparation method and use thereof; the spirocycle-containing derivative can be used as a brandnew neuropsychiatric drug that acts on 5-hydroxytryptamine receptors and/or trace amine-associated receptors and/or dopamine receptors, particularly has a good agonistic effect on 5-HT1A receptors and/or TAAR1 receptors, and can effectively prevent and/or treat neuropsychiatric diseases in mammals.

The object of the present application is to provide a spirocycle-containing derivative capable of preventing and/or treating a neuropsychiatric disease in mammals, a preparation method thereof, a pharmaceutical composition comprising the same and use thereof in the medical field.

The present application provides a compound represented by general formula (I), a stereoisomer or a pharmaceutically acceptable salt thereof,

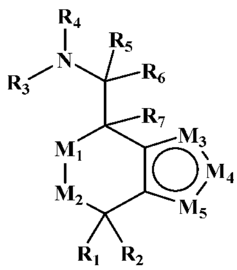

(I)

wherein

- $M_1$ and $M_2$ are each independently selected from $-(CR_AR_B)_n-$, O or S;
- $M_3$ is selected from $CR_a$, N or S;
- $M_4$ is selected from $CR_b$, N or S;
- $M_5$ is selected from $CR_c$, N or S;
- $R_A$, $R_B$, $R_a$, $R_b$ and $R_c$ are each independently selected from hydrogen, deuterium, halogen, hydroxyl, cyano, amino, $C_{1-6}$ alkyl, $C_{1-6}$ deuterated alkyl, $C_{1-6}$haloalkyl, $C_{1-6}$ alkoxy or $C_{1-6}$ alkylamine, preferably hydrogen, halogen or $C_{1-6}$ alkyl, more preferably hydrogen, fluorine, chlorine, bromine or $C_{1-3}$ alkyl, further preferably hydrogen, fluorine, chlorine, bromine or methyl;
- $R_1$ and $R_2$ together with the carbon atom to which they are connected are linked to form a $C_{3-8}$ cycloalkyl or a 3-8 membered heterocyclyl, preferably a $C_{3-6}$ cycloalkyl or a 3-6 membered heterocyclyl, more preferably a $C_{3-4}$ cycloalkyl or a 3-4 membered heterocyclyl, further preferably cyclopropyl, cyclobutyl or oxetanyl;
- $R_3$ and $R_4$ are each independently selected from hydrogen, deuterium, halogen, hydroxyl, cyano, $C_{1-6}$ alkyl, $C_{1-6}$ deuterated alkyl or $C_{1-6}$ haloalkyl, preferably hydrogen, $C_{1-6}$ alkyl, $C_{1-6}$ deuterated alkyl or $C_{1-6}$ haloalkyl, more preferably hydrogen, $C_{1-3}$ alkyl, $C_{1-3}$ deuterated alkyl or $C_{1-3}$ haloalkyl, further preferably hydrogen, methyl, ethyl, isopropyl, $-CD_3$ or $-CH_2CF_3$;

alternatively, $R_3$ and $R_4$ together with the nitrogen atom to which they are connected are linked to form a 3-8 membered nitrogen-containing heterocyclyl, preferably a 4-6 membered nitrogen-containing heterocyclyl, further preferably azetidinyl;

$R_5$ and $R_6$ are each independently selected from hydrogen, deuterium, halogen, hydroxyl, cyano, $C_{1-6}$ alkyl, $C_{1-6}$ deuterated alkyl or $C_{1-6}$ haloalkyl, preferably hydrogen or $C_{1-6}$ alkyl, more preferably hydrogen or $C_{1-3}$ alkyl, further preferably hydrogen or methyl;

alternatively, $R_5$ and $R_6$ together with the carbon atom to which they are connected are linked to form a $C_{3-8}$ cycloalkyl or a 3-8 membered heterocyclyl, preferably a $C_{3-5}$ cycloalkyl or a 3-5 membered heterocyclyl, further preferably cyclopropyl;

alternatively, $R_5$ or $R_6$ is linked with $R_3$ or $R_4$ to form a 3-8 membered nitrogen-containing heterocyclyl, preferably a 4-6 membered nitrogen-containing heterocyclyl, further preferably tetrahydropyrrolyl;

$R_7$ is selected from hydrogen, deuterium, halogen, hydroxyl, cyano, $C_{1-6}$ alkyl, $C_{1-6}$ deuterated alkyl or $C_{1-6}$ haloalkyl, preferably hydrogen or $C_{1-6}$ alkyl, more preferably hydrogen or $C_{1-3}$ alkyl, further preferably hydrogen or methyl;

alternatively, $R_7$ is linked with $R_3$ or $R_4$ to form a 4-8 membered nitrogen-containing heterocyclyl, preferably a 5-6 membered nitrogen-containing heterocyclyl, further preferably tetrahydropyrrolyl;

alternatively, $R_7$ is linked with $R_5$ or $R_6$ to form a $C_{3-8}$ cycloalkyl or a 3-8 membered heterocyclyl, preferably a $C_{4-6}$ cycloalkyl or a 4-6 membered heterocyclyl, further preferably cyclobutyl;

the cycloalkyl or heterocyclyl formed by linking $R_1$ and $R_2$ together with the carbon atom to which they are connected, the nitrogen-containing heterocyclyl formed by linking $R_3$ and $R_4$ together with the nitrogen atom to which they are connected, the cycloalkyl or heterocyclyl formed by linking $R_5$ and $R_6$ together with the carbon atom to which they are connected, the nitrogen-containing heterocyclyl formed by linking $R_5$ or $R_6$ with $R_3$ or $R_4$, the nitrogen-containing heterocyclyl formed by linking $R_7$ with $R_3$ or $R_4$, or the cycloalkyl or heterocyclyl formed by linking $R_7$with $R_5$ or $R_6$, optionally further substituted by one or more substituents selected from the group consisting of: hydrogen, deuterium, halogen, hydroxyl, cyano, nitro, amino, $C_{1-6}$ alkyl, $C_{1-6}$ alkoxy, $-(CH_2)_{n1}OR_{aa}$, $-(CH_2)_{n1}SR_{aa}$, $-(CH_2)_{n1}NR_{aa}R_{bb}$,$-(CH_2)_{n1}NR_{aa}C(O)R_{bb}$, $-(CH_2)_{n1}C(O)NR_{aa}R_{bb}$, $-(CH_2)_{n1}C(O)R_{aa}$,$-(CH_2)_{n1}C(O)OR_{aa}$, $-(CH_2)_{n1}S(O)_2R_{aa}$,$-(CH_2)_{n1}NR_{aa}S(O)_2R_{bb}$ and$-(CH_2)_{n1}S(O)_2NR_{aa}R_{bb}$;

$R_{aa}$ and $R_{bb}$ are each independently selected from hydrogen, deuterium, halogen, hydroxyl, cyano, nitro, amino, $C_{1-6}$ alkyl, $C_{1-6}$ deuterated alkyl or $C_{1-6}$ haloalkyl, preferably hydrogen, deuterium, halogen, hydroxyl, cyano, nitro, amino, $C_{1-3}$ alkyl, $C_{1-3}$ deuterated alkyl or $C_{1-3}$ haloalkyl;

n is an integer from 0 to 2; and

n1 is an integer from 0 to 2.

In a further preferred embodiment of the present application, wherein

$M_1$ and $M_2$ are each independently selected from $-(CR_AR_B)_n-$, O or S;

$M_3$ is selected from $CR_a$, N or S;

$M_4$ is selected from $CR_b$;

$M_5$ is selected from $CR_c$, N or S;

$R_A$, $R_B$, $R_a$, $R_b$ and $R_c$ are each independently selected from hydrogen, deuterium, halogen, hydroxyl, cyano, amino, $C_{1-6}$ alkyl, $C_{1-6}$ deuterated alkyl, $C_{1-6}$ haloalkyl, $C_{1-6}$ alkoxy or $C_{1-6}$alkylamine, preferably hydrogen, halogen or $C_{1-6}$ alkyl, more preferably hydrogen, fluorine, chlorine, bromine or $C_{1-3}$ alkyl, further preferably hydrogen, fluorine, chlorine or methyl;

$R_1$ and $R_2$ together with the carbon atom to which they are connected are linked to form a $C_{3-8}$ cycloalkyl or a 3-8 membered heterocyclyl, preferably a $C_{3-6}$ cycloalkyl or a 3-6 membered heterocyclyl, more preferably a $C_{3-4}$ cycloalkyl or 3-4 membered heterocyclyl, further preferably cyclopropyl, cyclobutyl or oxetanyl;

$R_3$ and $R_4$ are each independently selected from hydrogen, deuterium, halogen, hydroxyl, cyano, $C_{1-6}$ alkyl, $C_{1-6}$ deuterated alkyl or $C_{1-6}$ haloalkyl, preferably hydrogen, $C_{1-6}$ alkyl, $C_{1-6}$ deuterated alkyl or $C_{1-6}$ haloalkyl, more preferably hydrogen, $C_{1-3}$ alkyl, $C_{1-3}$ deuterated alkyl or $C_{1-3}$ haloalkyl, further preferably hydrogen, methyl, ethyl, isopropyl, $-CD_3$ or $-CH_2CF_3$;

alternatively, $R_3$ and $R_4$ together with the nitrogen atom to which they are connected are linked to form a 3-8 membered nitrogen-containing heterocyclyl, preferably a 4-6 membered nitrogen-containing heterocyclyl, further preferably azetidinyl;

$R_5$ and $R_6$ are each independently selected from hydrogen, deuterium, halogen, hydroxyl, cyano, $C_{1-6}$ alkyl, $C_{1-6}$ deuterated alkyl or $C_{1-6}$ haloalkyl, preferably hydrogen or $C_{1-6}$ alkyl, more preferably hydrogen or $C_{1-3}$ alkyl, further preferably hydrogen or methyl;

alternatively, $R_5$ and $R_6$ together with the carbon atom to which they are connected are linked to form a $C_{3-8}$ cycloalkyl or a 3-8 membered heterocyclyl, preferably a $C_{3-5}$ cycloalkyl or a 3-5 membered heterocyclyl, further preferably cyclopropyl;

alternatively, $R_5$ or $R_6$ is linked with $R_3$ or $R_4$ to form a 3-8 membered nitrogen-containing heterocyclyl, preferably a 4-6 membered nitrogen-containing heterocyclyl, further preferably tetrahydropyrrolyl;
$R_7$ is selected from hydrogen, deuterium, halogen, hydroxyl, cyano, $C_{1-6}$ alkyl, $C_{1-6}$ deuterated alkyl or $C_{1-6}$ haloalkyl, preferably hydrogen or $C_{1-6}$ alkyl, more preferably hydrogen or $C_{1-3}$ alkyl, further preferably hydrogen;
alternatively, $R_7$ is linked with $R_3$ or $R_4$ to form a 4-8 membered nitrogen-containing heterocyclyl, preferably a 5-6 membered nitrogen-containing heterocyclyl, further preferably tetrahydropyrrolyl;
alternatively, $R_7$ is linked with $R_5$ or $R_6$ to form a $C_{3-8}$ cycloalkyl or a 3-8 membered heterocyclyl, preferably a $C_{4-6}$ cycloalkyl or a 4-6 membered heterocyclyl, further preferably a cyclobutyl;
the cycloalkyl or heterocyclyl formed by linking $R_1$ and $R_2$ together with the carbon atom to which they are connected, the nitrogen-containing heterocyclyl formed by linking $R_3$ and $R_4$ together with the nitrogen atom to which they are connected, the cycloalkyl or heterocyclyl formed by linking $R_5$ and $R_6$ together with the carbon atom to which they are connected, the nitrogen-containing heterocyclyl formed by linking $R_5$ or $R_6$ with $R_3$ or $R_4$, the nitrogen-containing heterocyclyl formed by linking $R_7$ with $R_3$ or $R_4$, or the cycloalkyl or heterocyclyl formed by linking $R_7$ with $R_5$ or $R_6$, optionally furthersubstituted by one or more substituents selected from the group consisting of: hydrogen, deuterium, halogen, hydroxyl, cyano, nitro, amino, $C_{1-6}$ alkyl, $C_{1-6}$ alkoxy, $-(CH_2)_{n1}OR_{aa}$, $-(CH_2)_{n1}SR_{aa}$, $-(CH_2)_{n1}NR_{aa}R_{bb}$, $-(CH_2)_{n1}NR_{aa}C(O)R_{bb}$, $-(CH_2)_{n1}C(O)NR_{aa}R_{bb}$, $-(CH_2)_{n1}C(O)R_{aa}$, $-(CH_2)_{n1}C(O)OR_{aa}$, $-(CH_2)_{n1}S(O)_2R_{aa}$, $-(CH_2)_{n1}NR_{aa}S(O)_2R_{bb}$ and $-(CH_2)_{n1}S(O)_2NR_{aa}R_{bb}$;
$R_{aa}$ and $R_{bb}$ are each independently selected from hydrogen, deuterium, halogen, hydroxyl, cyano, nitro, amino, $C_{1-6}$ alkyl, $C_{1-6}$ deuterated alkyl or $C_{1-6}$ haloalkyl, preferably hydrogen, deuterium, halogen, hydroxyl, cyano, nitro, amino, $C_{1-3}$ alkyl, $C_{1-3}$ deuterated alkyl or $C_{1-3}$ haloalkyl;
n is an integer from 0 to 2; and
n1 is an integer from 0 to 2.

In a further preferred embodiment of the present application, the compound represented by general formula (I) can be:

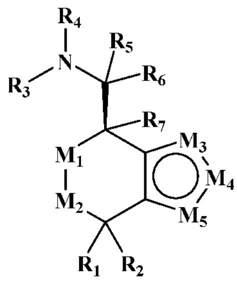
(I a) ,
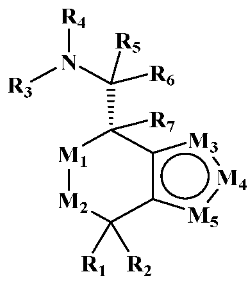
(I b)

or a mixture thereof.

In a further preferred embodiment of the present application, the following compounds are not included:

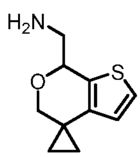 , 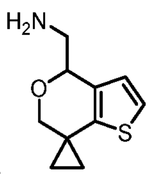 , 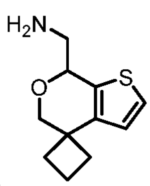 , 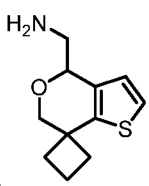 , 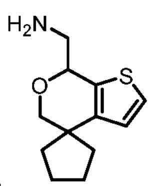 , 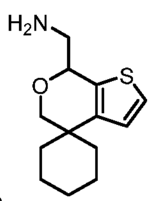 , 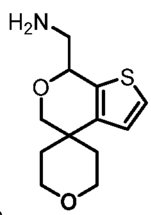 .

In a further preferred embodiment of the present application, $M_3$ is selected from S; $M_4$ is selected from $CR_b$ or N; $M_5$ is selected from $CR_c$ or N.

In a further preferred embodiment of the present application, $M_3$ is selected from $CR_a$ or N; $M_4$ is selected from S; $M_5$ is selected from $CR_c$ or N.

In a further preferred embodiment of the present application, $M_3$ is selected from $CR_a$ or N; $M_4$ is selected from $CR_b$ or N; $M_5$ is selected from S.

In a further preferred embodiment of the present application,

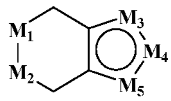

in general formula (I) is selected from the following groups:

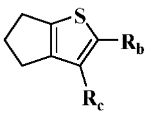, 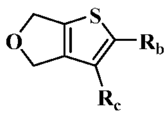, 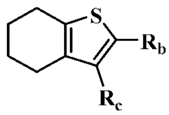, 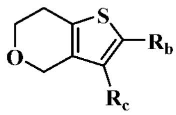, 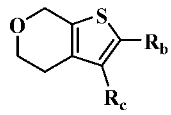,

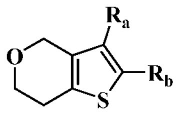, 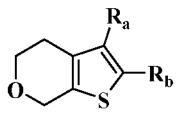, 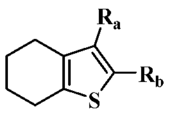, 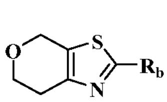 or 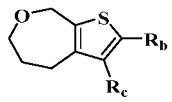,

preferably

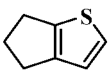, 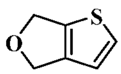, 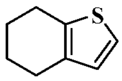, 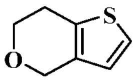, 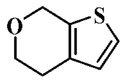, 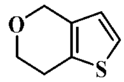,

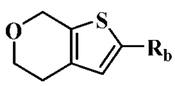, 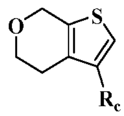, 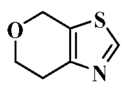 or 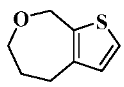;

Wherein $R_a$, $R_b$ and $R_c$ are each independently selected from hydrogen, deuterium, halogen, hydroxyl, cyano, amino, $C_{1-6}$ alkyl, $C_{1-6}$ deuterated alkyl, $C_{1-6}$ haloalkyl, $C_{1-6}$ alkoxy or $C_{1-6}$ alkylamine, preferably hydrogen, halogen or $C_{1-6}$ alkyl, more preferably hydrogen, fluorine, chlorine, bromine or $C_{1-3}$ alkyl, further preferably hydrogen, fluorine, chlorine, bromine or methyl.

In a further preferred embodiment of the present application,

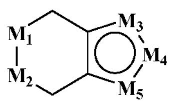

in general formula (I) is selected from the following groups:

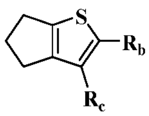, 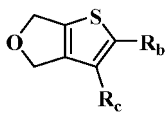, 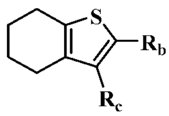, 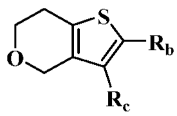, 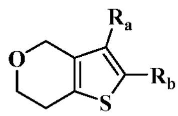,

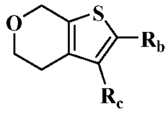, 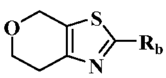 or 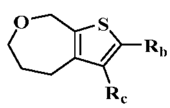,

preferably

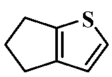, 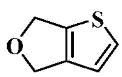,

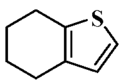, 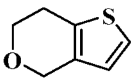, 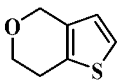, 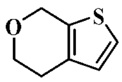, 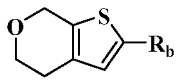, 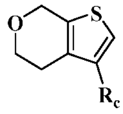, 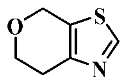

or

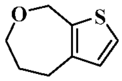;

wherein $R_a$, $R_b$ and $R_c$ are each independently selected from hydrogen, deuterium, halogen, hydroxyl, cyano, amino, $C_{1-6}$ alkyl, $C_{1-6}$ deuterated alkyl, $C_{1-6}$ haloalkyl, $C_{1-6}$ alkoxy or $C_{1-6}$ alkylamine, preferably hydrogen, halogen or $C_{1-6}$ alkyl, more preferably hydrogen, fluorine, chlorine, bromine or $C_{1-3}$ alkyl, further preferably hydrogen, fluorine, chlorine or methyl.

In a further preferred embodiment of the present application, general formula (I) is further represented by general formula (II-A):

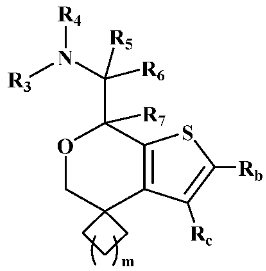

( II-A )

wherein $R_3$ and $R_4$ are each independently selected from hydrogen, $C_{1-6}$ alkyl, $C_{1-6}$ deuterated alkyl or $C_{1-6}$ haloalkyl, preferably hydrogen, $C_{1-3}$ alkyl, $C_{1-3}$ deuterated alkyl or $C_{1-3}$ haloalkyl, more preferably hydrogen, methyl, ethyl, isopropyl, $-CD_3$ or $-CH_2CF_3$;

alternatively, $R_3$ and $R_4$ together with the nitrogen atom to which they are connected are linked to form a 4-6 membered nitrogen-containing heterocyclyl, preferably azetidinyl;

$R_5$ and $R_6$ are each independently selected from hydrogen or $C_{1-6}$ alkyl, preferably hydrogen or $C_{1-3}$ alkyl, more preferably hydrogen or methyl;

alternatively, $R_5$ and $R_6$ together with the carbon atom to which they are connected are linked to form a $C_{3-5}$ cycloalkyl or a 3-5 membered heterocyclyl, preferably a cyclopropyl;

alternatively, $R_5$ or $R_6$ is linked with $R_3$ or $R_4$ to form a 4-6 membered nitrogen-containing heterocyclyl, preferably tetrahydropyrrolyl;

$R_7$ is selected from hydrogen or $C_{1-6}$ alkyl, preferably hydrogen or $C_{1-3}$ alkyl, more preferably hydrogen or methyl;

alternatively, $R_7$ is linked with $R_3$ or $R_4$ to form a 5-6 membered nitrogen-containing heterocyclyl, preferably tetrahydropyrrolyl;

alternatively, $R_7$ is linked with $R_5$ or $R_6$ to form a $C_{4-6}$ cycloalkyl or a 4-6 membered heterocyclyl, preferably cyclobutyl;

$R_b$ and $R_c$ are each independently selected from hydrogen, halogen or $C_{1-6}$ alkyl, preferably hydrogen, fluorine, chlorine, bromine or $C_{1-3}$ alkyl, more preferably hydrogen, fluorine, chlorine, bromine or methyl; and

m is 0 or 1.

In a further preferred embodiment of the present application, general formula (I) can be:

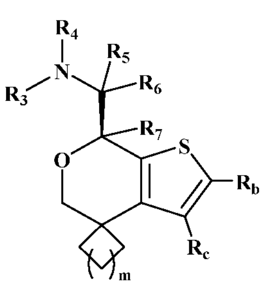

( II-Aa ) ,

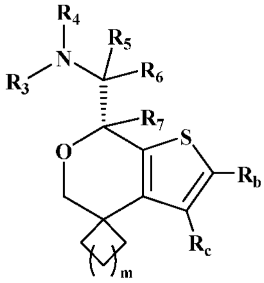

( II-Ab )

or a mixture thereof, preferably (II-Aa).

In a further preferred embodiment of the present application, general formula (I) is further represented by general formula (II):

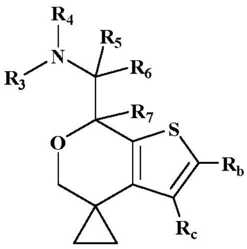

( II ) ,

wherein $R_b$, $R_c$, $R_3$, $R_4$, $R_5$, $R_6$ and $R_7$ are as described above.

In a further preferred embodiment of the present application, general formula (I) can be:

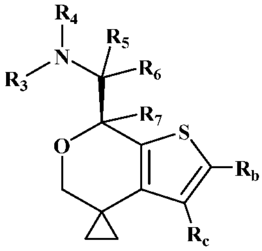

( II a ) ,

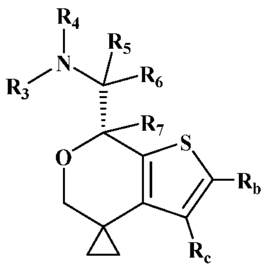

( II b )

or a mixture thereof, preferably (IIa).

In a further preferred embodiment of the present application, general formula (I) is further represented by general formula (III):

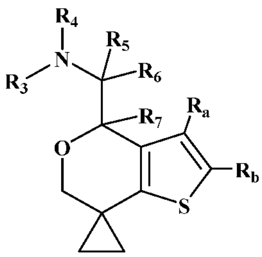

( III )

wherein $R_a$, $R_b$, $R_3$, $R_4$, $R_5$, $R_6$ and $R_7$ are as described above;

for example, $R_3$ and $R_4$ are each independently selected from hydrogen, $C_{1-6}$ alkyl, $C_{1-6}$ deuterated alkyl or $C_{1-6}$

haloalkyl, preferably hydrogen, $C_{1-3}$ alkyl, $C_{1-3}$ deuterated alkyl or $C_{1-3}$ haloalkyl, more preferably hydrogen, methyl, ethyl, isopropyl, $-CD_3$ or $-CH_2CF_3$;

$R_5$, $R_6$ and $R_7$ are each independently selected from hydrogen or $C_{1-6}$ alkyl, preferably hydrogen or $C_{1-3}$ alkyl, more preferably hydrogen;

$R_a$ and $R_b$ are each independently selected from hydrogen, halogen or $C_{1-6}$ alkyl, preferably hydrogen or $C_{1-3}$ alkyl, more preferably hydrogen.

In a further preferred embodiment of the present application, general formula (I) can be:

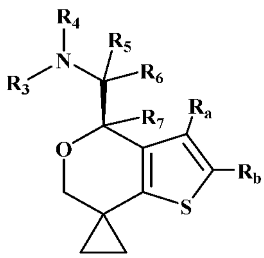

( III a ) ,

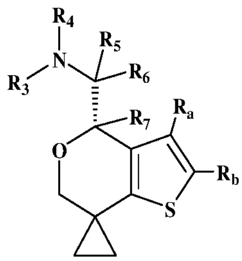

( III b)

or a mixture thereof, preferably (III a).

In a further preferred embodiment of the present application, $R_3$ and $R_4$ in the general formula (I) are not hydrogen at the same time.

In a further preferred embodiment of the present application, in the general formula (I), $R_3$is hydrogen; and $R_4$ is selected from $C_{1-3}$ alkyl, $C_{1-3}$ deuterated alkyl or $C_{1-3}$ haloalkyl.

In a further preferred embodiment of the present application, in the general formula (I), $R_3$ is hydrogen; and $R_4$ is selected from methyl, ethyl, isopropyl, $-CD_3$ or $-CH_2CF_3$.

In a further preferred embodiment of the present application, in the general formula (I), $R_3$ is hydrogen; and $R_4$ is methyl or $-CD_3$.

In a further preferred embodiment of the present application, the compound represented by the general formula (I), a stereoisomer or a pharmaceutically acceptable salt thereof is selected from the group consisting of:

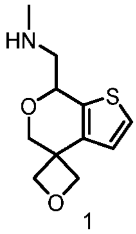

1 ,

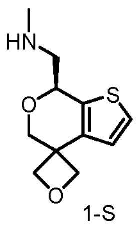

1-S ,

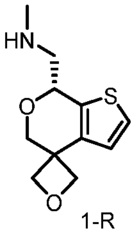

1-R ,

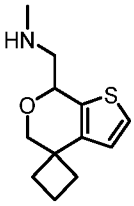

2 ,

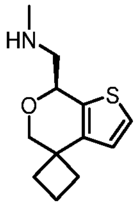

2-S ,

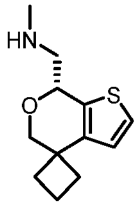

2-R ,

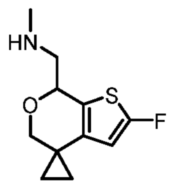

3 ,

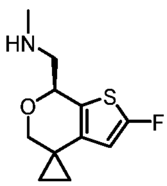

3-S ,

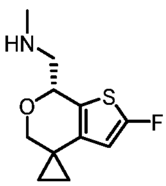

3-R ,

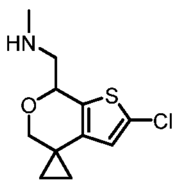

4 ,

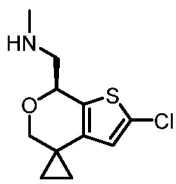

4-S ,

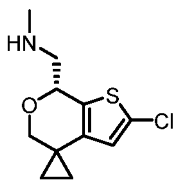

4-R ,

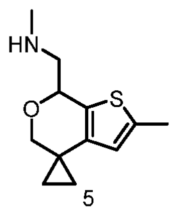

5 ,

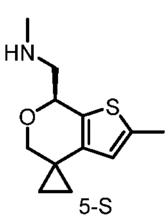 5-S , 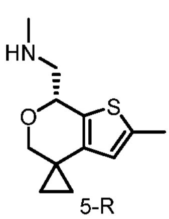 5-R , 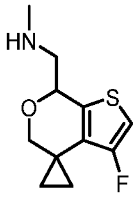 6 , 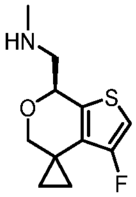 6-S , 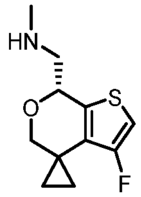 6-R , 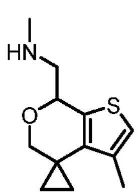 7 , 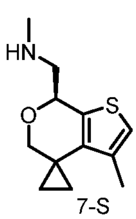 7-S ,

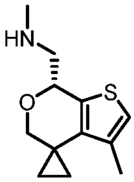 7-R , 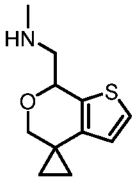 8 , 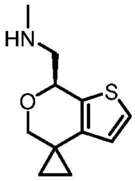 8-S , 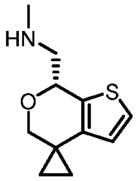 8-R , 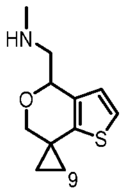 9 , 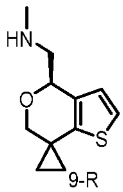 9-R , 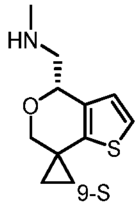 9-S ,

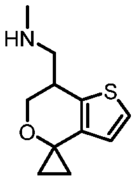 10 , 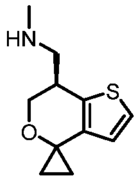 10-R , 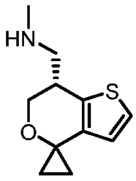 10-S , 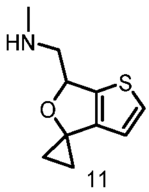 11 , 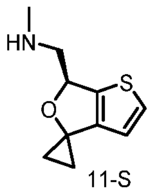 11-S , 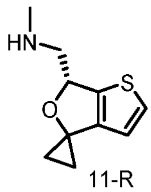 11-R , 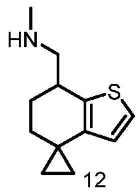 12 ,

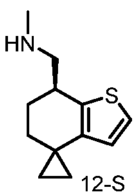 12-S , 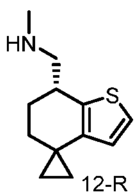 12-R , 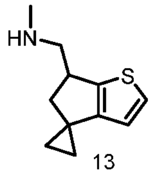 13 , 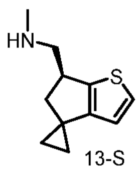 13-S , 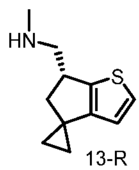 13-R , 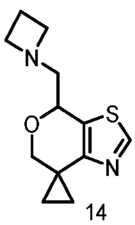 14 , 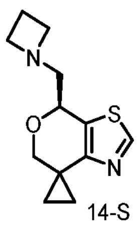 14-S ,

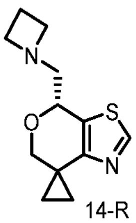 14-R , 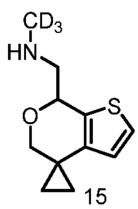 15 , 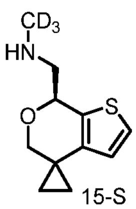 15-S , 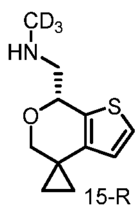 15-R , 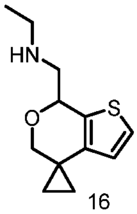 16 , 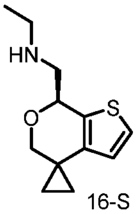 16-S , 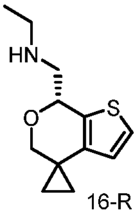 16-R ,

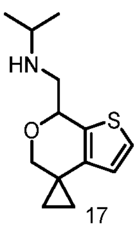 17 , 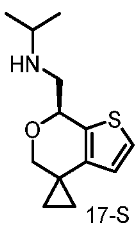 17-S , 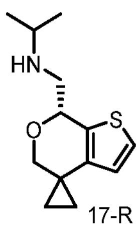 17-R , 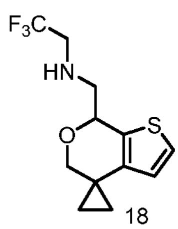 18 , 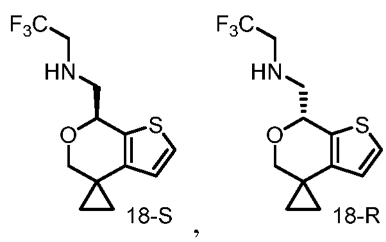 18-S , 18-R ,

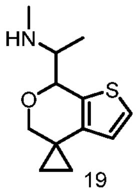 19 , 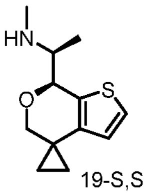 19-S,S , 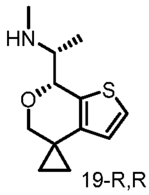 19-R,R , 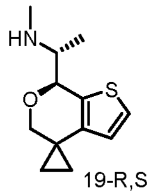 19-R,S , 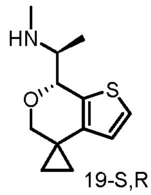 19-S,R , 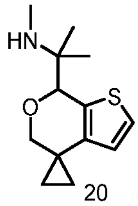 20 , 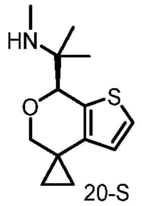 20-S ,

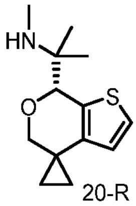 20-R , 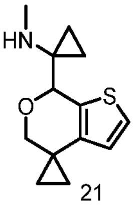 21 , 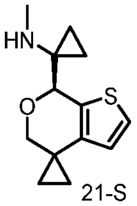 21-S , 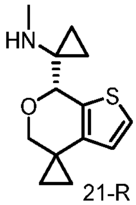 21-R , 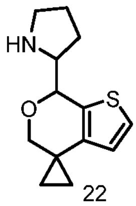 22 , 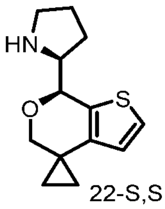 22-S,S , 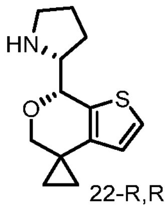 22-R,R ,

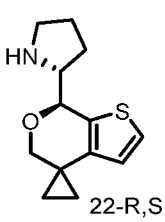 22-R,S , 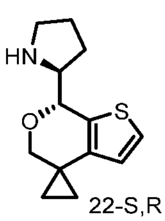 22-S,R , 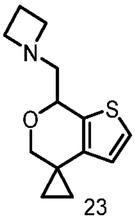 23 , 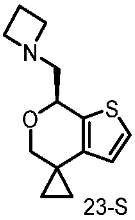 23-S , 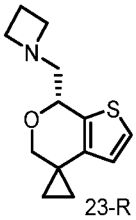 23-R , 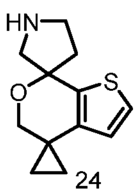 24 , 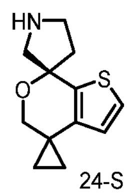 24-S ,

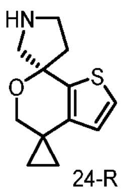 24-R , 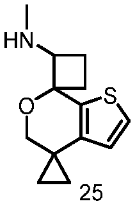 25 , 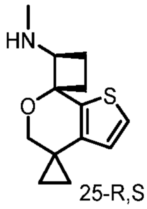 25-R,S , 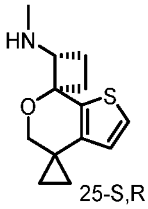 25-S,R , 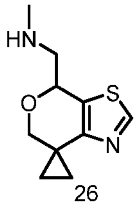 26 , 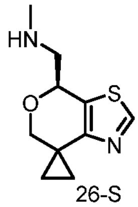 26-S , 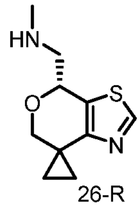 26-R ,

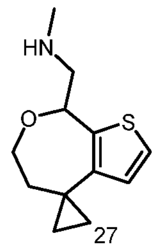 27 , 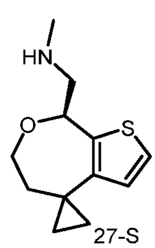 27-S , 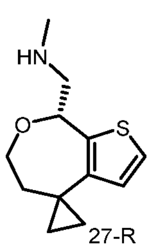 27-R , 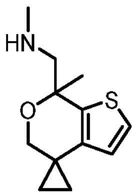 28 , 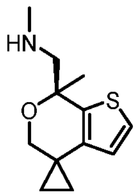 28-S , 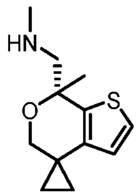 28-R , 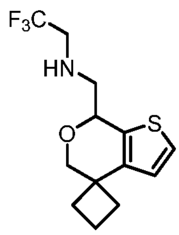 29 ,

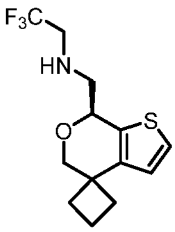 29-S , 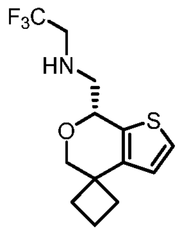 29-R , 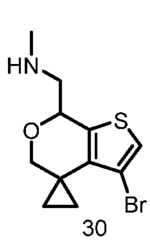 30 , 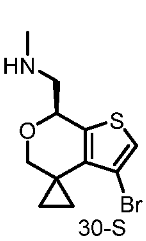 30-S , 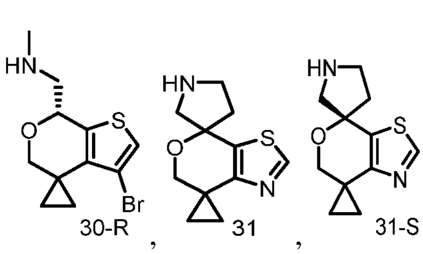 30-R , 31 , 31-S ,

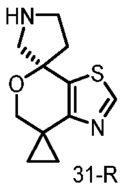 31-R , 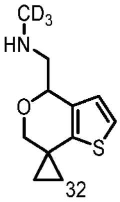 32 , 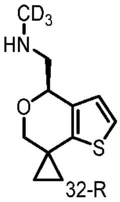 32-R , 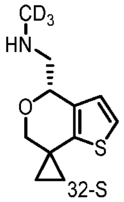 32-S , 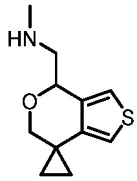 33 , 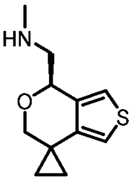 33-R , 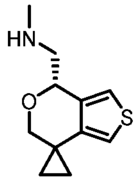 33-S , 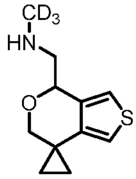 34 ,

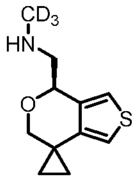 34-R , 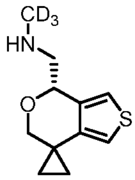 34-S , 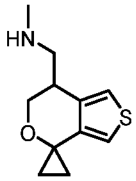 35 , 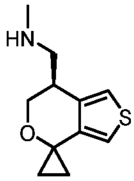 35-R , 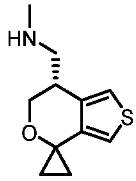 35-S , 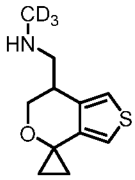 36 , 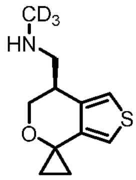 36-R

and

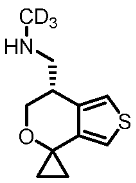

36-S .

The present application further provides a method for preparing the compound represented by the general formula (II-A), a stereoisomer or a pharmaceutically acceptable salt thereof, comprising:

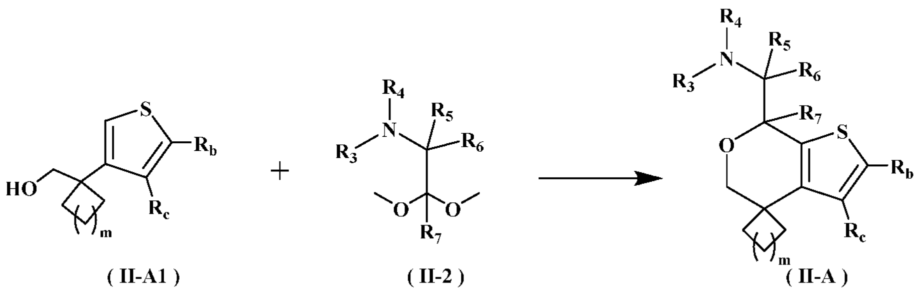

( II-A1 ) ( II-2 ) ( II-A )

reacting a compound of the general formula (II-A1) with a compound of the general formula (II-2) to prepare the compound represented by the general formula (II-A), a stereoisomer or a pharmaceutically acceptable salt thereof; wherein $R_b$, $R_c$, m, $R_3$, $R_4$, $R_5$, $R_6$ and $R_7$ are as described above.

The present application further provides a method for preparing the compound represented by the general formula (II), a stereoisomer or a pharmaceutically acceptable salt thereof, comprising:

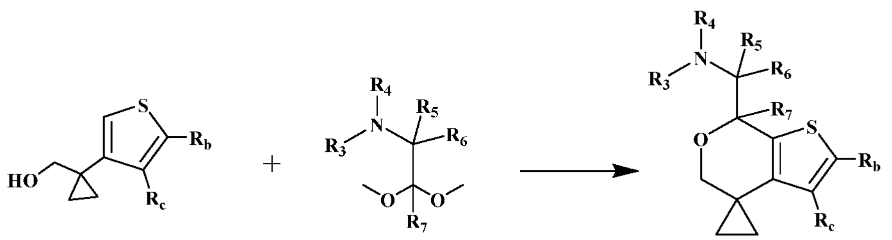

( II-1 ) ( II-2 ) ( II )

reacting a compound of the general formula (II-1) with a compound of the general formula (II-2) to prepare the compound represented by the general formula (II), a stereoisomer or a pharmaceutically acceptable salt thereof; wherein $R_b$, $R_c$, $R_3$, $R_4$, $R_5$, $R_6$ and $R_7$ are as described above.

The present application further provides a method for preparing the compound represented by the general formula (III), a stereoisomer or a pharmaceutically acceptable salt thereof, comprising:

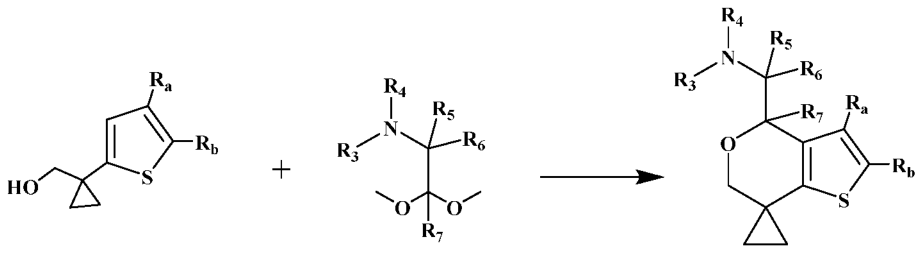

( III-1 ) ( II-2 ) ( III )

reacting a compound of the general formula (III-1) is reacted with a compound of the general formula (II-2) to obtain the compound represented by the general formula (III), a stereoisomer or a pharmaceutically acceptable salt thereof; wherein $R_a$, $R_b$, $R_3$, $R_4$, $R_5$, $R_6$ and $R_7$ are as described above.

The present application further relates to a pharmaceutical composition comprising a therapeutically effective amount of any of the compounds shown, a stereoisomers or a pharmaceutically acceptable salt thereof and one or more pharmaceutically acceptable carriers (or excipients).

In a further preferred embodiment of the present application, the pharmaceutical composition can be formulated in a conventional manner using one or more pharmaceutically acceptable carriers. The active compound of the present application can thus be formulated into a dosage form for oral, buccal, intranasal, parenteral (e.g. intravenous, intramuscular or subcutaneous) or rectal administration, or a dosage form suitable for inhalation or insufflationadministration. The compound of the present application, or a pharmaceutically acceptable salt thereof, can also be formulated into a sustained release dosage form.

In a further preferred embodiment of the present application, for oral administration, the active compound of the present application, for example, can be formulated into tablets or capsules by conventional means with a pharmaceutically acceptable excipient, such as an adhesive, filler, lubricant, disintegrant or wetting agent. Tablets can be coated by methods well known in the art. Liquid preparations for oral administration can be, for example, solutions, syrups or suspensions, or dry products obtained by volatilization, which are regenerated with water or other suitable carrier before use. Such liquid preparations can be prepared by conventional means using pharmaceutically acceptable additives such as suspending agents, emulsifying agents, non-aqueous carriers and preservatives.

In a further preferred embodiment of the present application, when the active compound of the present application is used for parenteral administration, the compound provided by the present application can be combined with sterile water or organic medium to form an injectable solution or suspension.

In a further preferred embodiment of the application, the active compound according to the present application can be formulated into rectal compositions such as suppositories or retention enemas, e.g. comprising conventional suppository matrix such as cocoa butter or other glycerides.

The present application further relates to a use of any one of the shown compounds, a stereoisomer or a pharmaceutically acceptable salt thereof, or a pharmaceutical composition comprising the same in the preparation of medicaments, which can be medicaments for preventing and/or treating a neuropsychiatric disease in mammals; the neuropsychiatric disease is preferably central nervous system disease related to 5-hydroxytryptamine receptors and/or trace amine-associated receptors, and/or dopamine receptors.

The present application further relates to a method for preparing medicaments for preventing and/or treating a central nervous system disease related to5-hydroxytryptamine receptors and/or trace amine-related receptors and/or dopamine receptors in mammals using any of the compounds shown, a stereoisomer or a pharmaceutically acceptable salt thereof, or a pharmaceutical composition comprising the same.

The present application also relates to a method for preventing and/or treating a central nervous system disease related to 5-hydroxytryptamine receptors and/or trace amine-related receptors and/or dopamine receptors in mammals, which comprises administering to said mammals a therapeutically effective amount of any one of the shown compounds, a stereoisomer thereof, or a pharmaceutically acceptable salt, ester, prodrug, solvate, hydrate or derivative thereof, or a pharmaceutical composition comprising the same.

The 5-hydroxytryptamine receptor involved in the present application is preferably a 5-$HT_{1A}$ receptor.

The trace amine-associated receptor involved in the present application is preferably a TAAR1 receptor.

In some embodiments, the neuropsychiatric disease involved in the present application is one or more diseases selected from the group consisting of: schizophrenia, schizophrenia spectrum disease, acute schizophrenia, chronic schizophrenia, NOS schizophrenia, schizoid personality disorder, schizotypal personality disorder, delusional disorder, psychosis, psychotic disorder, brief psychotic disorder, shared psychotic disorder, psychotic disorder due to a physical disease, drug-induced psychosis, psychoaffective disorder, aggression, delirium, Parkinson's psychosis, excitative psychosis, Tourette's syndrome, organic or NOS psychosis, seizure, agitation, post-traumatic stress disorder, behavior disorder, neurodegenerative disease, Alzheimer's disease, Parkinson's disease, dyskinesias, Huntington's disease, dementia, mood disorder, anxiety, affective disorder, depression, major depressive disorder, dysthymia, bipolar disorder, manic disorder, seasonal affective disorder, attention deficit disorder, attention deficit hyperactivity disorder, obsessive-compulsive disorder, vertigo, epilepsy, pain, neuropathic pain, sensitization accompanying neuropathic pain, inflammatory pain, fibromyalgia, migraine, cognitive impairment, movement disorder, restless leg syndrome, multiple sclerosis, sleep disorder, sleep apnea, narcolepsy, excessive daytime sleepiness, jet lag, drowsy side effect of medications, insomnia, substance abuse or dependence, addiction, eating disorder, sexual dysfunction, hypertension, emesis, Lesche-Nyhane disease, Wilson's disease, autism, Huntington's chorea, and premenstrual dysphoria.

## DETAILED DESCRIPTION OF THE APPLICATION

Unless otherwise defined, all technical and scientific terms used herein have the same meaning as commonly understood by one of ordinary skilled in the art to which the present application belongs. In case of conflict, the definitions provided in the present application shall prevail. When a trade name appears herein, it is intended to refer to the corresponding product or its active ingredient. All patents, published patent applications and publications cited herein are incorporated herein by reference.

The term "hydrocarbon chain" refers to a chain group composed of C and H. The hydrocarbon chain may be saturated or unsaturated, and in preferred embodiments, the hydrocarbon chain is saturated. The hydrocarbon chain may be linear or branched, and in preferred embodiments the hydrocarbon chain is linear. The hydrocarbon chain may optionally comprise one or more heteroatoms such as N, O and S. In the case of comprising a heteroatom, the heteroatom may be located on the backbone. In a preferred embodiment, the hydrocarbon chain may be linear or branched, and the hydrocarbon chain is saturated, the hydrocarbon chain optionally comprises one or more heteroatoms such as N, O and S in the backbone. When describing a hydrocarbon chain, whether it comprises heteroatoms or not, it can be described by the number of C atoms without counting the number of heteroatoms. For example, $C_2$-$C_8$ or $C_2$-$C_6$ refers to a hydrocarbon chain comprising 2-8 or 2-6 carbon atoms, which may optionally comprise additional heteroatoms.

The term "alkyl" refers to a linear or branched saturated aliphatic hydrocarbon group consisting of carbon atoms and hydrogen atoms, which is connected to the rest of the molecule by a single bond. "Alkyl" can have 1-8 carbon atoms, that is, "$C_1$-$C_8$ alkyl", such as $C_{1-4}$ alkyl, $C_{1-3}$ alkyl, $C_{1-2}$ alkyl, $C_3$ alkyl, $C_4$ alkyl, $C_{1-6}$ alkyl, $C_{3-6}$ alkyl. Non-limiting examples of alkyl include, but are not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof. The alkyl may be optionally substituted or unsubstituted, and when substituted, a substituent may be substituted at any available connection point, the substituent is preferably one or more groups independently selected from the following groups consisting of: alkyl, alkenyl, alkynyl, alkoxy, alkylthio, halogen, mercapto, hydroxy, nitro, amino, cyano, carboxyl, oxo, cycloalkyl, heterocyclyl, aryl and heteroaryl.

The term "subunit" refers to a group with two connecting sites connected to other parts of the molecule, which is obtained by removing a hydrogen atom from the carbon atom containing free valence electron. For example "alkylene" or "alkyl subunit" refers to a saturated linear or branched divalent hydrocarbon group.

The term "alkylene", when used herein alone or in combination with other groups, refers to a linear or branched saturated divalent hydrocarbon group. For example, the term "$C_{1-8}$ alkylene" refers to an alkylene having 1-8 carbon atoms, such as methylene, ethylene, propylene, butylene, pentylene, hexylene, 1-methylethylidene, 2-methylethylidene, methyl propylidene or ethyl propylidene. The alkylene may be optionally substituted or unsubstituted, and when substituted, a substituent may be substituted at any available connection point, the substituent is preferably one or more groups independently selected from the following groups consisting of: alkyl, alkenyl, alkynyl, alkoxy, alkylthio, halogen, mercapto, hydroxy, nitro, amino, cyano, carboxyl, oxo, cycloalkyl, heterocyclyl, aryl and heteroaryl.

The term "cycloalkyl" refers to a saturated or partially unsaturated monocyclic or polycyclic cyclic hydrocarbon substituent, the cycloalkyl comprises 3 to 20 carbon atoms, that is, "$C_3$-$C_{20}$ cycloalkyl", such as $C_{3-18}$cycloalkyl, $C_{3-16}$ cycloalkyl, $C_{3-12}$ cycloalkyl, $C_{3-8}$ cycloalkyl, $C_{3-6}$ cycloalkyl, $C_{3-5}$ cycloalkyl, $C_{3-4}$ cycloalkyl, $C_{4-8}$ cycloalkyl, $C_{4-6}$ cycloalkyl, $C_{5-6}$ cycloalkyl, preferably $C_{3-8}$ cycloalkyl, $C_{3-6}$ cycloalkyl, $C_{3-5}$ cycloalkyl, $C_{3-4}$ cycloalkyl. Non-limiting examples of

monocyclic cycloalkyls include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; polycyclic cycloalkyl includes cycloalkyl of spiro rings, fused rings and bridged rings.

The term "spirocycloalkyl" refers to a 5 to 20 membered polycyclic group in which one carbon atom (called a spiro atom) is shared between the monocyclic rings, which may comprise one or more double bonds, but none of the rings has a fully conjugated $\pi$-electron system. Preferably it is 6 to 14 membered, more preferably 7 to 10 membered. According to the number of spiro atoms shared between the rings, spirocycloalkyls are divided into monospirocycloalkyl, bispirocycloalkyl or polyspirocycloalkyl, preferably monospirocycloalkyl or bispirocycloalkyl, more preferably 3 membered/7 membered, 3 membered/6 membered, 3 membered/5 membered, 4 membered/4 membered, 4 membered/5 membered, 4 membered/6 membered, 5 membered/5 membered or 5 membered/6 membered monospirocycloalkyl. Also included is spiroheterocycloalkyl in which monospirocycloalkyl shares a spiro atom with heterocycloalkyl.

The term "fused cycloalkyl" refers to a 5 to 20 membered all-carbon polycyclic group in which each ring of the system shares one adjacent pair of carbon atoms with other rings in the system, wherein one or more rings may contain one or more double bonds, but none of the rings have a fully conjugated $\pi$-electron system. Preferably it is 6 to 14 membered, more preferably 7 to 10 membered. According to the number of constituent rings, they are divided into bicyclic, tricyclic, tetracyclic or polycyclic fused cycloalkyl, preferably bicyclic or tricyclic fused cycloalkyl, more preferably 5-membered/5-membered or 5-membered/6-membered bicycloalkyl.

The term "bridged cycloalkyl" refers to a 5 to 20 membered all-carbon polycyclic group in which any two rings sharing two carbon atoms that are not directly connected, and it comprises one or more double bonds, but none of the rings has a fully conjugated $\pi$-electron system. Preferably it is 6 to 14 membered, more preferably 7 to 10 membered. According to the number of constituent rings, they are divided into bicyclic, tricyclic, tetracyclic or polycyclic bridged cycloalkyl, preferably bicyclic, tricyclic or tetracyclic bridged cycloalkyl, more preferably bicyclic or tricyclic bridged cycloalkyl.

The cycloalkyl described above can all be fused to an aryl, heteroaryl or heterocycloalkyl ring, wherein the ring connected to the parent structure is a cycloalkyl. The cycloalkyl may be optionally substituted or unsubstituted, and when substituted, a substituent may be substituted at any available connection point, the substituent is preferably one or more groups independently selected from the following groups consisting of: alkyl, alkenyl, alkynyl, alkoxy, alkylthio, halogen, mercapto, hydroxy, nitro, amino, cyano, carboxyl, oxo, cycloalkyl, heterocyclyl, aryl and heteroaryl.

The term "heterocyclyl" refers to a saturated or unsaturated monocyclic or polycyclic cyclic hydrocarbon substituent comprising 3 to 20 ring atoms, one or more of which are heteroatoms selected from the group consisting of: nitrogen, oxygen and $S(O)_m$ (wherein m is an integer from 0 to 2), but excluding the ring part of -O-O-, -O-S- or -S-S-, and the remaining ring atoms are carbon. That is, "3-20 membered heterocyclyl", such as 3-18 membered heterocyclyl, 3-16 membered heterocyclyl, 3-12membered heterocyclyl, 3-8 membered heterocyclyl, 3-6 membered heterocyclyl, 3-5 membered heterocyclyl, 3-4 membered heterocyclyl, 4-8 membered heterocyclyl, 4-6 membered heterocyclyl, 5-6 membered heterocyclyl, preferably 3-8 membered heterocyclyl, 3-6 membered heterocyclyl, 3-5 membered heterocyclyl, 3-4 membered heterocyclyl, 4-8 membered heterocyclyl, 4-6 membered heterocyclyl, 5-6 membered heterocyclyl, which optionally comprises 1-4 heterocycles, 1-3 heterocycles or 1-2 heterocycles, wherein the heteroatoms are optionally N, O or S atoms.

Non-limiting examples of monocyclic heterocyclyl include oxetanyl, thietanyl, pyrrolidinyl, imidazolidinyl, tetrahydrofuranyl, tetrahydrothiophenyl, tetrahydropyranyl, dihydroimidazole, dihydrofuranyl, dihydropyrazolyl, piperidinyl, piperazinyl, morpholinyl, 1,3-dioxolan, 2,2-difluoro-1,3-dioxolan or azepinyl et al. Non-limiting examples of polycyclic heterocyclyl include spiro, fused and bridged heterocyclyl, wherein the spiro, fused and bridged heterocyclyl are optionally connected to other groups through a single bond, or further ring-connected with other cycloalkyl, heterocyclyl, aryl and heteroaryl through any two or more atoms on the ring.

The term "spiroheterocyclyl" refers to a 5 to 20 membered polycyclic heterocyclyl in which one atom (called spiro atom) is shared between the monocyclic rings, wherein one or more ring atoms are heteroatoms selected from the group consisting of: nitrogen, oxygen and $S(O)_m$ (wherein m is an integer from 0 to 2), and the remaining ring atoms are carbon. It may comprise one or more double bonds, but none of the rings has a fully conjugated $\pi$-electron system. Preferably it is 6 to 14 membered, more preferably 7 to 10 membered. According to the number of spiro atoms shared between the rings, spiroheterocyclyls are divided into monospiroheterocyclyl, bispiroheterocyclyl or polyspiroheterocyclyl, preferably monospiroheterocyclyl or bispiroheterocyclyl, more preferably 3 membered/6 membered, 3 membered/5 membered, 4 membered/4 membered, 4 membered/5 membered, 4 membered/6 membered, 5 membered/5 membered or 5 membered/6 membered monospiroheterocyclyl.

The term "fused heterocyclyl" refers to a 5 to 20 membered polycyclic heterocyclyl in which each ring of the system shares one adjacent pair of atoms with other rings in the system, wherein one or more ring atoms are heteroatoms selected from the group consisting of: nitrogen, oxygen and $S(O)_m$ (where m is an integer from 0 to 2), the remaining ring atoms are carbon, and one or more rings may comprise one or more double bonds, but none of the rings has a fully conjugated $\pi$-electron system. Preferably it is 6 to 14 membered, more preferably 7 to 10 membered. According to the

number of constituent rings, fused heterocyclyls can be divided into bicyclic, tricyclic, tetracyclic or polycyclic fused heterocyclyl, preferably bicyclic or tricyclic fused heterocyclyl, more preferably 5-membered/5-membered or 5-membered/6-membered bicyclic fused heterocyclyl.

The term "bridged heterocyclyl" refers to a 5 to 20 membered polycyclic heterocyclyl in which any two rings share two atoms not directly connected, wherein one or more ring atoms are heteroatoms selected from the group consisting of: nitrogen, oxygen and $S(O)_m$ (where m is an integer from 0 to 2), the remaining ring atoms are carbon. It contains one or more double bonds, but none of the rings has a fully conjugated $\pi$-electron system. Preferably it is 6 to 14 membered, more preferably 7 to 10 membered. According to the number of constituent rings, bridged heterocyclyls can be divided into bicyclic, tricyclic, tetracyclic or polycyclic bridged heterocyclyl, preferably bicyclic, tricyclic or tetracyclic bridged heterocyclyl, more preferably bicyclic or tricyclic bridged heterocyclyl.

The heterocyclyls described above can all be fused to an aryl, heteroaryl or cycloalkyl ring, wherein the ring connected to the parent structure is a heterocyclyl. The heterocyclyl may be optionally substituted or unsubstituted, and when substituted, a substituent may be substituted at any available connection point, the substituent is preferably one or more groups independently selected from the following groups consisting of: alkyl, alkenyl, alkynyl, alkoxy, alkylthio, halogen, mercapto, hydroxy, nitro, amino, cyano, carboxyl, oxo, cycloalkyl, heterocyclyl, aryl and heteroaryl.

The term "aryl" refers to a 6 to 14 membered all-carbon monocyclic or fused polycyclic group with a conjugated $\pi$-electron system, preferably 6 to 12 membered, such as phenyl or naphthyl, more preferably phenyl. The aryl can be fused to a heteroaryl, heterocyclic or cycloalkyl ring, wherein the ring connected to the parent structure is an aryl ring, including benzo 5-10 membered heteroaryl, benzo 3-8 membered cycloalkyl and benzo 3-8 membered heterocyclyl, preferably benzo 5-6 membered heteroaryl, benzo 3-6 membered cycloalkyl and benzo 3-6 membered heterocyclyl. The aryl may be optionally substituted or unsubstituted, and when substituted, a substituent may be substituted at any available connection point, the substituent is preferably one or more groups independently selected from the following groups consisting of: alkyl, alkenyl, alkynyl, alkoxy, alkylthio, halogen, mercapto, hydroxy, nitro, amino, cyano, carboxyl, oxo, cycloalkyl, heterocyclyl, aryl and heteroaryl.

The term "heteroaryl" refers to a heteroaromatic system comprising 1 to 4 heteroatoms and 5 to 14 ring atoms, wherein one or more heteroatoms are selected from the group consisting of: oxygen, sulfur, nitrogen, etc. Heteroaryl is preferably 5 to 12 membered, more preferably 5 to 6 membered, such as pyrrolyl, imidazolyl, furyl, pyranyl, thienyl, thiazolyl, thiadiazolyl, pyrazolyl, oxazolyl, oxadiazolyl, triazolyl, tetrazolyl, pyridyl, pyrimidyl, pyridazinyl, pyrazinyl. The heteroaryl may be fused to an aryl, cycloalkyl or heterocyclyl ring, wherein the ring connected to the parent structure is a heteroaryl ring. The heteroaryl may be optionally substituted or unsubstituted, and when substituted, a substituent may be substituted at any available connection point, the substituent is preferably one or more groups independently selected from the following groups consisting of: alkyl, alkenyl, alkynyl, alkoxy, alkylthio, halogen, mercapto, hydroxy, nitro, amino, cyano, carboxyl, oxo, cycloalkyl, heterocyclyl, aryl and heteroaryl.

The term "alkoxy" refers to -O-(alkyl) or -O-(unsubstituted cycloalkyl), wherein the definitions of alkyl and cycloalkyl are as described above. Non-limiting examples of alkoxy include: methoxy, ethoxy, propoxy, butoxy, cyclopropoxy, cyclobutoxy, cyclopentyloxy, cyclohexyloxy, etc. The alkoxy may be optionally substituted or unsubstituted, and when substituted, a substituent may be substituted at any available connection point, the substituent is preferably one or more groups independently selected from the following groups consisting of: alkyl, alkenyl, alkynyl, alkoxy, alkylthio, halogen, mercapto, hydroxy, nitro, amino, cyano, carboxyl, oxo, cycloalkyl, heterocyclyl, aryl and heteroaryl.

The term "alkylthio" refers to -S-(alkyl) or -S-(unsubstituted cycloalkyl), wherein the definitions of alkyl and cycloalkyl are as described above. Non-limiting examples of alkylthio include: methylthio, ethylthio, propylthio, butylthio, cyclopropylthio, cyclobutylthio, cyclopentylthio, cyclohexylthio, etc. The alkylthio may be optionally substituted or unsubstituted, and when substituted, a substituent may be substituted at any available connection point, the substituent is preferably one or more groups independently selected from the following groups consisting of: alkyl, alkenyl, alkynyl, alkoxy, alkylthio, halogen, mercapto, hydroxy, nitro, amino, cyano, carboxyl, oxo, cycloalkyl, heterocyclyl, aryl and heteroaryl.

The term "alkylamino" refers to -NH-(alkyl or unsubstituted cycloalkyl), or -N-(alkane or unsubstituted cycloalkyl) (alkyl or unsubstituted cycloalkyl), wherein The definitions of alkyl and cycloalkyl are as described above. Non-limiting examples of alkylamino groups include: methylamino, ethylamino, propylamino, butylamino, cyclopropylamino, cyclobutylamino, cyclopentylamino, cyclohexylamino, etc. The alkylamino may be optionally substituted or unsubstituted, and when substituted, a substituent may be substituted at any available connection point, the substituent is preferably one or more groups independently selected from the following groups consisting of: alkyl, alkenyl, alkynyl, alkoxy, alkylthio, halogen, mercapto, hydroxy, nitro, amino, cyano, carboxyl, oxo, cycloalkyl, heterocyclyl, aryl and heteroaryl.

The term "halogen" or "halogen element" or "halogenated" should be understood to mean fluorine (F), chlorine (Cl), bromine (Br) or iodine (I) atom, preferably a fluorine, chlorine, or bromine atom.

The term "deuterated hydrocarbon chain" refers to a hydrocarbon chain substituted with one or more deuteriums, wherein the hydrocarbon chain is as defined above.

The term "deuterated alkyl" refers to an alkyl substituted with one or more deuteriums, wherein the alkyl is as

defined above.

The term "halogenated hydrocarbon chain" refers to a hydrocarbon chain substituted with one or more halogens, wherein the hydrocarbon chain is as defined above.

The term "haloalkyl" refers to an alkyl substituted with one or more halogens, wherein the alkyl is as defined above.

The term "haloalkoxy" refers to an alkoxy substituted with one or more halogens, wherein alkoxy is as defined above.

The term "alkenyl" refers to achain alkenyl, also known as alkylene, wherein the alkylene can be further substituted by other related groups.

The term "alkynyl" refers to (CH=C-), wherein the alkynyl may be further substituted by other related groups.

"Hydroxy" refers to -OH.
"Amino" refers to $-NH_2$.
"Cyano" refers to -CN.
"Nitro" refers to $-NO_2$.
"Mercapto" refers to -SH.
"Carbonyl" refers to -C(O)-.
"Carboxy" refers to -C(O)OH.
"Oxo" means =O.

The terms "comprising", "including", "having", "containing" or "involving" and other variations thereof herein are inclusive or open-ended, and do not exclude other unlisted elements or method steps. Those skilled in the art should understand that the above mentioned terms such as "comprising" cover the meaning of "consisting of...".

The term "one or more" or the similar expression "at least one" can represent, for example, 1, 2, 3, 4, 5, 6, 7, 8, 9, 10 or more.

When the lower and upper limits of a numerical range are disclosed, any values and any included ranges falling within this range are specifically disclosed. Particularly, each range of values disclosed herein is to be understood to mean each value and range encompassed within the broader range.

Herein, "Z" and "-Z-" both represent the same specific group, which can be used interchangeably.

The expression m-n as used herein refers to the range from m to n, as well as each point value and the sub-range composed of each point value. For example, the expression "$C_2$-$C_8$" or "$C_{2-8}$" covers the range of 2-8 carbon atoms, and should be understood to also cover any sub-range therein as well as each point value, such as $C_2$-$C_5$, $C_3$-$C_4$, $C_2$-$C_6$, $C_3$-$C_6$, $C_4$-$C_6$, $C_4$-$C_7$, $C_4$-$C_8$, $C_2$-$C_5$, and $C_2$, $C_3$, $C_4$, $C_5$, $C_6$, $C_7$, $C_8$. For example, the expression "$C_3$-$C_{10}$" or "$C_{3-10}$" should also be understood in a similar manner, for example to cover any sub-range and point value contained therein, such as $C_3$-$C_9$, $C_6$-$C_9$, $C_6$-$C_8$, $C_6$-$C_7$, $C_7$-$C_{10}$, $C_7$-$C_9$, $C_7$-$C_8$, $C_8$-$C_9$, and $C_3$, $C_4$, $C_5$, $C_6$, $C_7$, $C_8$, $C_9$, $C_{10}$. As another example, the expression "$C_1$-$C_6$" or "$C_{1-6}$" covers the range of 1-6 carbon atoms, and should be understood as also covering any sub-range and each point value, such as $C_2$-$C_5$, $C_3$-$C_4$, $C_1$-$C_2$, $C_1$-$C_3$, $C_1$-$C_4$, $C_1$-$C_5$, $C_1$-$C_6$, and $C_1$, $C_2$, $C_3$, $C_4$, $C_5$, $C_6$. As another example, the expression "three membered to ten membered" should be understood as covering any sub-range and each point value therein, such as three membered to five membered, three membered to six membered, three membered to seven membered, three membered to eight membered, four membered to five membered, four membered to six membered, four membered to seven membered, four membered to eight membered, five membered to seven membered, five membered to eight membered, six membered to seven membered, six membered to eight membered, nine membered to ten membered, and three, four, five, six, seven, eight, nine, ten membered. Other similar expressions herein should also be understood in a similar manner.

As used herein, the expression "X is selected from A, B or C", "X is selected from A, B and C", "X is A, B or C", "X is A, B and C", etc., express the same meaning, that is X can be any one or several of A, B, and C.

The term "optional" or "optionally" means that the subsequently described event or situation may or may not occur, and the expression includes the occurrence of the event or situation and the nonoccurrence of the event or situation. For example, "cycloalkyl optionally substituted with alkyl" means that alkyl may but does not have to exist, including the situation where cycloalkyl is substituted by alkyl and the situation where cycloalkyl is not substituted by alkyl.

The terms "substitute" and "substituted" mean that one or more (e.g., one, two, three or four) hydrogens on the indicated atom being replaced by a selection from the indicated group, provided that the normal atomic valence of the specified atom in the current situation is not exceeded and the substitution a stable compound is formed. Combinations of substituents and/or variables are permissible only if such combinations form stable compounds. When describing the absence of a substituent, it should be understood that the substituent may be one or more hydrogen atoms, provided that the structure can bring the compound to a stable state. When describing how each carbon atom in a group can be optionally replaced by a heteroatom, the condition is that the normal atomic valence of all atoms in the group in the current situation is not exceeded and a stable compound is formed.

If a substituent is described as "optionally...substituted", the substituent may be unsubstituted or may be substituted. If an atom or group is described as being optionally substituted with one or more of the list of substituents, the atom or one or more hydrogens on the group may be substituted by independently selected, optional substituents. When a substituent is oxo (i.e., =O), it means that two hydrogen atoms are replaced. As used herein, unless otherwise indicated, the connection point of a substituent may be from any suitable position of the substituent.

When the bond of a substituent appears as a bond connecting two atoms through a ring, such substituent can be bonded to any ring-forming atom in the substitutable ring.

When any variable (such as R), as well as labeled variables (such as $R_1$, $R_2$, $R_3$, $R_4$, $R_5$, $R_6$, $R_7$) occur more than once in the composition or structure of a compound, its definition is independent in each case at each occurrence. For example, if a group is substituted with 0, 1, 2, 3 or 4 R substituents, the group may optionally be substituted with up to four R substituents, and in each case, the options for each R substituent are independent of each other.

The term "substituted" means that one or more hydrogen atoms in a compound or group are replaced by other atoms or groups, provided that a stable valence state or compound is formed. The expression "non-substituted" can be understood as "unsubstituted". It should be understood that when a substituent is hydrogen, this can also mean that the corresponding group is "non-substituted" or "unsubstituted".

The compounds of the present application may exist in particular geometric or stereoisomeric forms. The present application envisages all such compounds, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present application. Additional asymmetric carbon atoms may be present in substituents such as alkyl. All these isomers, as well as mixtures thereof, are included within the scope of the present application. In certain embodiments, preferred compounds are those isomeric compounds that exhibit superior biological activity. Purified or partially purified isomers and stereoisomers, or racemic mixtures or diastereomer mixtures of the compounds of the present application are also included within the scope of the present application. Purification and isolation of such substances can be achieved by standard techniques known in the art.

The hydrogen atom described in the present application can be substituted by its isotope deuterium, and the content of the deuterium isotope is at least greater than that of the natural deuterium isotope, and any hydrogen atom in the compounds of the examples involved in the present application can also be substituted by a deuterium atom.

The term "pharmaceutically acceptable" substance means a substance which, within the scope of normal medical judgment, is suitable for use in contact with the tissues of a patient without undue toxicity, irritation, allergic reaction, etc., with a reasonable ratio of benefit to harm, and can be effectively used for its intended purpose.

The term "pharmaceutically acceptable salt" refers to a salt of the compound of the present application, which is safe and effective when used in mammals, and has due biological activity.

The term "pharmaceutical composition" refers to a mixture containing one or more compounds of the present application or their physiologically/pharmaceutically acceptable salts or prodrugs and other chemical components, as well as other components such as physiologically/pharmaceutically acceptable carriers or excipients. The purpose of pharmaceutical compositions is to promote the administration of drugs to organisms, facilitate the absorption of active ingredients and thus exert biological activity.

The term "pharmaceutically acceptable carrier" refers to those substances that have no obvious irritating effect on the organism and will not impair the biological activity and performance of the active compound. "Pharmaceutically acceptable carrier" includes, but is not limited to, glidants, sweeteners, diluents, preservatives, dyes/colorants, flavoring agents, surfactants, wetting agents, dispersants, disintegrants, stabilizers, solvents or emulsifiers.

The terms "administering" or "administration" and the like refer to methods that enable the delivery of a compound or composition to the desired site of biological action. These methods include, but are not limited to, oral or parenteral (including intraventricular, intravenous, subcutaneous, intraperitoneal, intramuscular, intravascular injection or infusion), topical, rectal administration, etc.; particularly by injection or by oral administration.

As used herein, the term "treating" includes alleviating, relieving or improving a disease or condition, preventing other conditions, improving or preventing underlying metabolic factors of a condition, inhibiting a disease or condition, e.g. preventing the development of a disease or condition, relieving a disease or condition, promoting the alleviation of a disease or condition, or stopping the symptoms of a disease or condition, and extending to include prevention. "Treatment" also includes achieving therapeutic benefit and/or preventive benefit. Therapeutic benefit refers to eradication or improvement of the treated condition. In addition, therapeutic benefit is achieved by eradicating or improving one or more physiological symptoms associated with an underlying disease, and although patients may still suffer from the underlying disease, the improvements of the patient's disease can be observed. Preventive benefit means that a patient uses a composition to prevent the risk of a certain disease, or the patient takes it when one or more physiological symptoms of the disease occur, although the disease has not yet been diagnosed.

The terms "active ingredient", "therapeutic agent", "active substance" or "active agent" refer to a chemical entity that is effective in the treatment or prevention of a target disorder, disease or condition. The term "neuropsychiatric

disease" refers to the general term for neurological diseases and psychiatric diseases, including neurological diseases and/or psychiatric diseases.

For a drug, a drug unit or an active ingredient, the term "effective amount", "therapeutically effective amount" or "preventively effective amount" refers to a sufficient amount of a drug or agent that has acceptable side effects but can achieve the desired effect. The determination of the effective amount varies from person to person, depending on the individual's age and general condition, as well as the specific active substance. The appropriate effective amount in each case can be determined by those skilled in the art according to routine experiments.

"Individual" as used herein includes a human or non-human animal. Exemplary human individuals include human individuals suffering from a disease (e.g., a disease described herein) (referred to as a patient) or normal individuals. "Non-human animals" in the present application include all vertebrates, such as non-mammals (e.g., birds, amphibians, reptiles) and mammals, such as non-human primates, livestocks and/or domesticated animals (e.g., sheep, dogs, cats, cows, pigs).

The following detailed description of the application is intended to illustrate non-limiting embodiments, enabling other skilled in the art to have a more comprehensive understanding of the technical solutions of the present application, the principles and practical applications thereof, so that other skilled in the art can modify and implement the application in many forms to best adapt to the requirements of specific uses.

## BENEFICIAL EFFECT

The compound of the present application has good agonistic effects on TAAR1 receptor and $5\text{-}HT_{1A}$ receptor, and/or has good *in-vivo* efficacy, and has anti-neuropsychiatric disease activity, that is, it has therapeutic or preventive effects on neuropsychiatric diseases.

## DETAILED EMBODIMENT

The present application is further described below in combination with specific examples. It should be understood that these examples are only used to illustrate the present application and are not intended to limit the scope of the present application. In addition, it should be understood that after reading the content of the present application, those skilled in the art may make various changes or modifications to the present application, and these equivalent forms also fall within the scope defined by the appended claims of the present application.

## EXAMPLE

The embodiments of the present invention will be described in detail below in combination with examples. However, those skilled in the art will understand that the following examples are only used to illustrate the present application and should not be considered as limiting the scope of the present application. If specific conditions are not specified in the example, the conventional conditions or conditions suggested by the manufacturer shall be followed. The reagents or instruments used without specifying the manufacturer are all commercially available conventional products. Unless otherwise specified, the proportions or percentages used herein are calculated by weight.

The structures of the compounds of the present application were determined by nuclear magnetic resonance (NMR) and/or liquid chromatography-mass chromatography (LC-MS).

NMR chemical shifts (δ) were given in unit of parts per million (ppm). NMR was determined by AVANCE III600 nuclear magnetic instrument, and the determination solvent was deuterated dimethyl sulfoxide (DMSO-$d_6$), deuterated methanol ($CD_3OD$) and deuterated chloroform ($CDCl_3$), and the internal standard was tetramethylsilane (TMS).

Liquid chromatography-mass chromatography (LC-MS) was determined by Shimadzu LCMS2020 mass spectrometer.

HPLC was determined by Shimadzu LC20A liquid chromatograph.

The thin-layer chromatography silica gel plates used were Yantai Jiangyou silica gel plates, the specification of TLC used was 0.2mm±0.03mm, and the specification of thin-layer chromatography separation and purification products was 0.4mm-0.5mm.

## EXAMPLE 1:1-(5'H,7'H-spiro[oxetane-3,4'-thieno[2,3-c]pyran]-7'-yl)-N-methylmethylamine

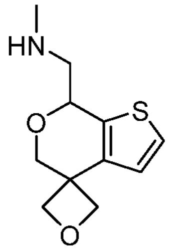

Synthesis scheme 1:

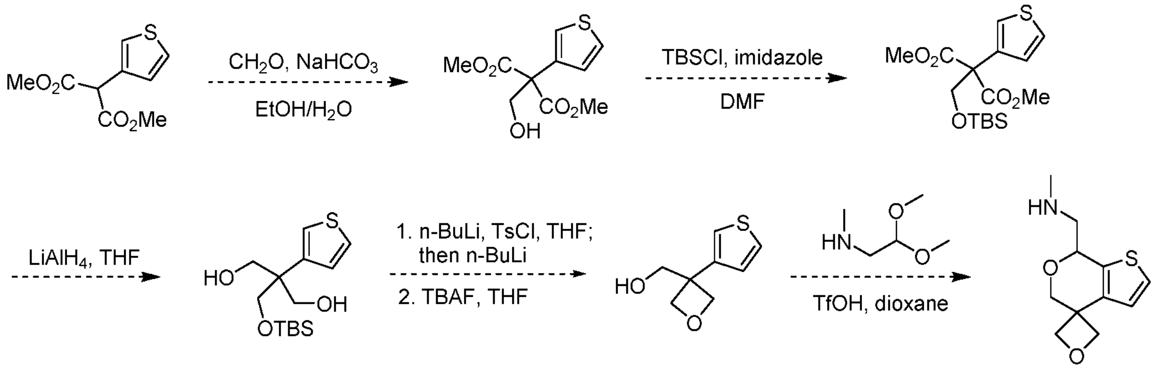


Synthesis scheme 2:

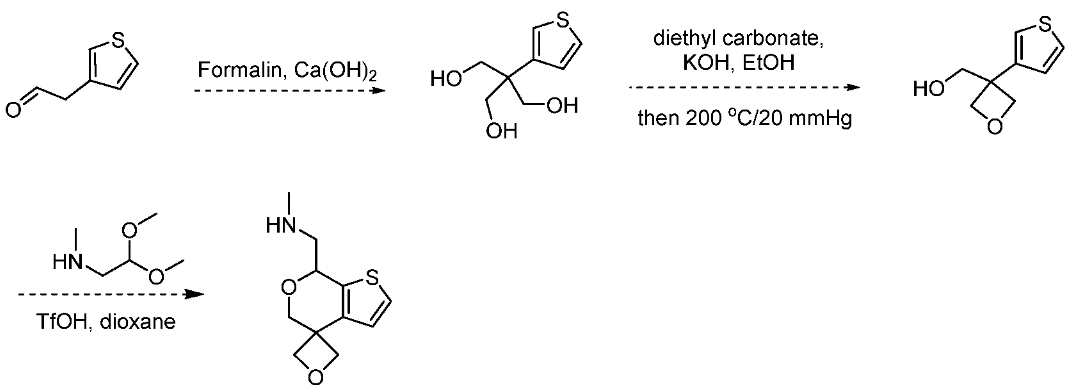


LC-MS $[M+H]^+$: 226.1.

**EXAMPLE 2:1-(5'H,7'H-spiro[cyclobutane-1,4'-thieno[2,3-c]pyran]-7'-yl)-N -methylmethylamine**

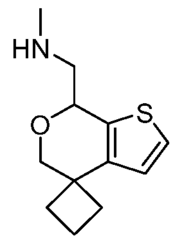

**Synthesis route 1:**

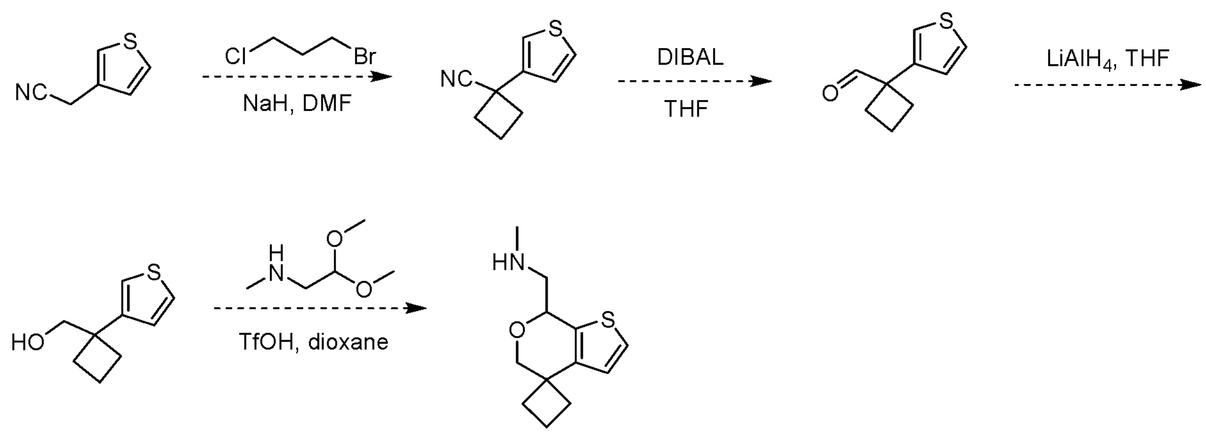


**Synthesis route 2:**

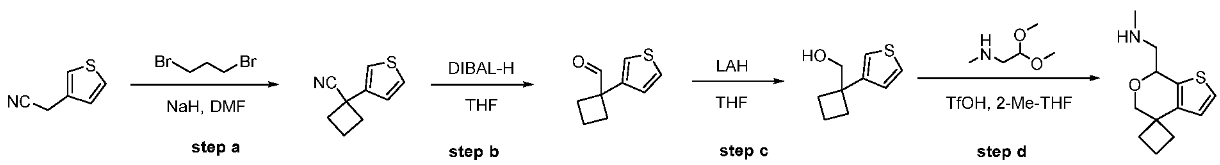


**Step a: synthesis of 1-(thiophen-3-yl)cyclobutyronitrile**

Sodium hydride (60%, 1.6g, 40.59mmol) was added to a solution of 2-(thiophene-3-yl) acetonitrile (2.0 g, 16.24 mmol) in N, N-dimethylformamide (30 mL) in an ice bath under nitrogen protection; after reacting for 1 hour, 1,3-dibromopropane (4.0 g, 19.8 mmol) was slowly added, and the reaction solution was slowly heated to room temperature and stirred overnight. After the reaction was completed, the reaction solution was poured into ice water (300 mL), extracting with ethyl acetate (300 mL × 3); the extract phase was washed with water (200 mL × 3) and saturated salt solution (200 mL × 3), drying with anhydrous sodium sulfate, filtering and concentrating, then the crude product was separated and purified by column chromatography (petroleum ether/ethyl acetate) to obtain the target product (1.5 g, 56.6% yield).

$^1H$ NMR (300MHz, $CDCl_3$) δ7.39-7.33(m, 1H), 7.30-7.23(m, 1H), 7.15(dd, J=5.1, 1.5Hz, 1H), 2.89-2.75(m, 2H), 2.68-2.49 (m, 2H), 2.44-2.26 (m, 1H), 2.19-2.00 (m, 1H).

**Step b: synthesis of 1-(thiophen-3-yl)cyclobutyraldehyde**

An n-hexane solution of diisobutyl aluminum hydride (1 M in hexane, 19.0 mL, 19.0 mmol) was slowly added to a tetrahydrofuran (100 mL) solution of 1-(thiophene-3-yl)cyclobutyronitrile (1.4 g, 8.58 mmol) in an ice bath under nitrogen protection, stirring at room temperature for 3 hours; after the reaction was completed, water (300 mL) was added to quench the reaction in ice bath, extracting with ethyl acetate (300 mL × 3); the extract phase was washed with water (300 mL × 3) and saturated salt solution (300 mL × 3), drying with anhydrous sodium sulfate, filtering and

concentrating, then the crude product was separated and purified by column chromatography (petroleum ether/ethyl acetate) to obtain the target product (632.0 mg, 44.3% yield).

$^1H$ NMR (400MHz, $CDCl_3$) δ9.57 (s, 1H), 7.35(dd, J=4.8, 2.8Hz, 1H), 7.10-7.07(m, 1H), 6.93(dd, J=5.2, 1.2Hz, 1H), 2.76-2.65 (m, 2H), 2.39-2.28 (m, 2H), 2.04-1.92 (m, 2H).

**Step c: synthesis of (1-(thiophen-3-yl)cyclobutyl)methanol**

A tetrahydrofuran solution of lithium aluminum hydride (2.5 M in THF, 3.3 mL, 8.30 mmol) was slowly added to a tetrahydrofuran (20 mL) solution of 1-(thiophene-3-yl)cyclobutyraldehyde (632.0 mg, 3.8 mmol) in an ice bath under nitrogen protection, then slowly heating to room temperature and stirring for 2 hours; after the reaction was completed, water (200 mL) was added to quench the reaction inice bath, extracting with ethyl acetate (300 mL × 3); the extract phase was washed with water (300 mL × 3) and saturated salt solution (300 mL × 3), drying with anhydrous sodium sulfate, filtering and concentrating, then the crude product was separated and purified by column chromatography (petroleum ether/ethyl acetate) to obtain the target product (570.0 mg, 89.02% yield).

$^1H$ NMR (300 MHz, $CDCl_3$) δ 7.34 - 7.28 (m, 1 H), 7.04 - 7.00 (m, 1 H), 6.95 (d, J= 4.8 Hz, 1 H), 3.75 (s, 2 H), 2.40 - 1.83 (m, 6 H).

**Step d: synthesis of 1-(5'H, 7'H-spiro[cyclobutane-1,4'-thieno[2,3-c]pyran] -7'-yl)-N-methylmethylamine**

2,2-dimethoxy-N-methylethylamine (170.0 mg, 1.43 mmol) and trifluoromethanesulfonic acid (0.4 mL) were added to a 2-methyltetrahydrofuran (4 mL) solution of (1-(thiophen-3-yl)cyclobutyl)methanol (200.0 mg, 1.19 mmol), stirring at 80°C for 20 minutes; after the reaction was completed, the pH of the reaction solution was adjusted to 13 with 15% sodium hydroxide aqueous solution at room temperature, then the reaction solution was poured into water (20 mL), extracting with ethyl acetate (20 mL × 3), combining the extract phases and concentrating to obtain the crude product, and then the crude product was separated and purified by column chromatography (petroleum ether/ethyl acetate) to obtain the target end-product (37.3 mg, 11.7% yield).

$^1H$ NMR (400 MHz, $CDCl_3$) δ 8.49 (s, 1 H), 7.22 (d, *J* = 4.8 Hz, 1 H), 7.15 (d, *J* = 5.2 Hz, 1 H), 5.12 (dd, *J* = 9.6, 2.8 Hz, 1 H), 4.11 (d, *J* = 11.2 Hz, 1 H), 3.68 (d, *J* = 11.6 Hz, 1 H), 3.22 (dd, *J* = 12.8, 3.2 Hz, 1 H), 3.05 (dd, *J* = 12.8, 9.6 Hz, 1 H), 2.68 (s, 3 H), 2.39 - 2.28 (m, 1 H), 2.23 - 2.14 (m, 2 H), 2.05 - 1.88 (m, 3 H).

LC-MS $[M+H]^+$: 224.2.

**Example 3: 1-(2'-fluoro-5'H,7'H-spiro[cyclopropane-1,4'-thieno[2,3-c]pyran] -7'-yl)-N-methylmethylamine**

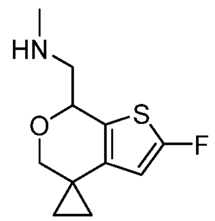

**Synthesis route 1:**

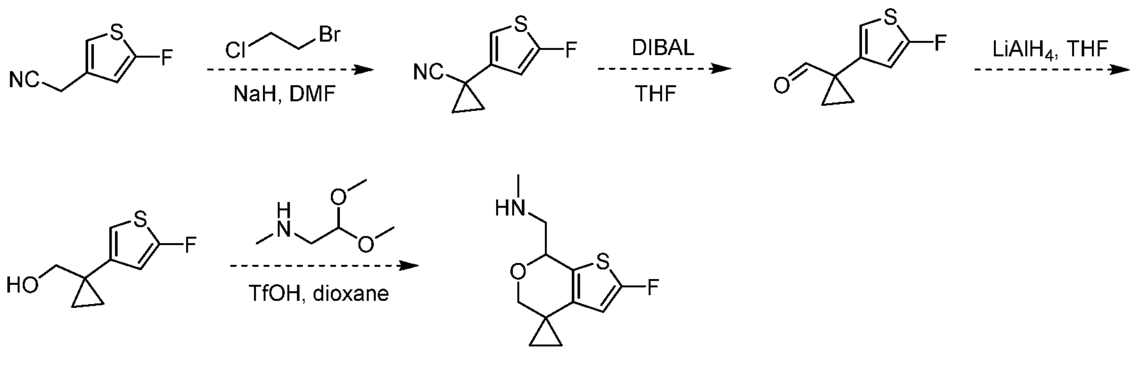


**Synthesis route 2:**

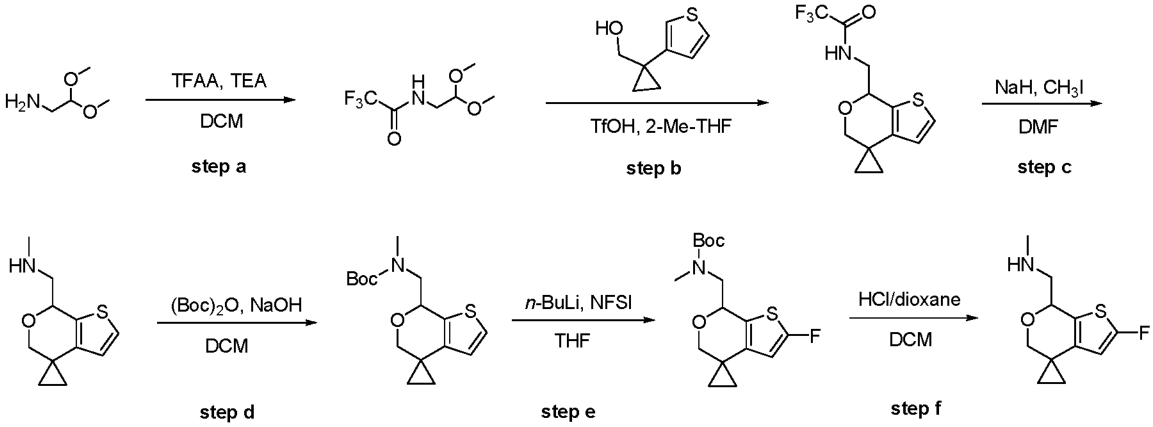


**Step a: synthesis of N-(2,2-dimethoxyethyl)-2,2,2-trifluoroacetamide**

Triethylamine (1.8 g, 17.8 mmol) was added to a dichloromethane (10 mL) solution of 2,2-dimethoxyethylamine (500.0 mg, 4.75 mmol) in an ice bath under nitrogen protection, stirring for 30 minutes, then trifluoroacetic anhydride (1.19 g, 5.7 mmol) was slowly added, and stirring was continued for 2 hours at room temperature; after the reaction was completed, the reaction solution was neutralized with saturated sodium bicarbonate aqueous solution (20 mL) in ice bath, then extracting with dichloromethane (20 mL × 3); the extract phase was washed with water (20 mL × 3) and saturated salt solution (20 mL × 3), drying with anhydrous sodium sulfate, filtering and concentrating to obtain the target crude product (800 mg), which could be put into the next reaction without purification.

**Step b:synthesis of N-((5'H,7'H-spiro[cyclopropane-1,4'-thieno[2,3-c]pyran] -7'-yl)methyl)-2,2,2-trifluoroacetamide**

N-(2,2-dimethoxyethyl)-2,2,2-trifluoroacetamide (800 mg of crude product) and trifluoromethanesulfonic acid (389.6 mg, 2.6 mmol) were added to a 2-methyltetrahydrofuran (15 mL) solution of (1-(thiophen-3-yl)cyclopropyl)methanol (500.0 mg, 3.24 mmol), stirring at room temperature for 16 hours; after the reaction was completed, the pH of the reaction solution was adjusted to 13 with 15% sodium hydroxide aqueous solution, diluting with water (20 mL), extracting the obtained mixture with ethyl acetate (20 mL × 3); after concentrating the extract phase, the crude product obtained extract phase was separated and purified by column chromatography (petroleum ether/ethyl acetate) to obtain the target product (800.0 mg, 84.7% yield).

LC-MS $[M+H]^+$: 292.0.

**Step c: synthesis of 1-(5'H,7'H-spiro[cyclopropane-1,4'-thieno[2,3-c]pyran] -7'-yl)-N-methylmethylamine**

Sodium hydride (60%, 219.9 mg, 5.5 mmol) and iodomethane (429.1 mg, 3.02 mmol) were added to a N,N-dimethylformamide (10 mL) solution of N-(5'H,7'H-spiro[cyclopropane-1,4'-thieno[2,3-c]pyran]-7'-yl)methyl)-2,2,2-trifluoroa cetamide (800.0 mg, 2.75 mmol), stirring at room temperature for 16 hours; after the reaction was completed, water (10 mL) was added to the reaction solution, then extracting with ethyl acetate (10 mL × 3), after concentrating the extract phase, the crude product obtained was separated and purified by column chromatography to obtain the target product (320.0 mg, 55.7% yield).

LC-MS $[M+H]^+$: 210.0.

**Step d: synthesis of tert-butyl((5'H,7'H-spiro[cyclopropane-1,4'-thieno[2,3 -c]pyran]-7'-yl)methyl)(methyl)carbamate**

Di-tert-butyl dicarbonate (526.0 mg, 2.40 mmol) was added to the dichloromethane (10 mL) solution comprising 1-(5'H,7'H-spiro [cyclopropane-1,4'-thieno[2,3-c]pyran]-7'-yl)-N-methylmethylamine (252.0 mg, 1.20 mmol) and sodium hydroxide (241.0 mg, 6.00 mmol), then stirring overnight; after the reaction was completed, water (10 mL) was added to the reaction solution for dilution, extracting with ethyl acetate (10 mL × 3); after concentrating the extract phase, the crude product obtained was separated and purified by column chromatography (ethyl acetate: petroleum ether=1:10) to obtain the target product (211.0 mg, 56.6% yield).

LC-MS $[M+H-100]^+$: 210.1.

**Step e: synthesis of tert-butyl((2'-fluoro-5'H,7'H-spiro[cyclopropane-1,4'-thieno[2,3-c]pyran]-7'-yl)methyl)(methyl)carbamate**

N-butyl lithium (2.5 M in hexane, 0.9 mL, 2.25 mmol) was added to the tetrahydrofuran (4 mL) solution of tert-butyl ((5'H, 7'H-spiro[cyclopropane-1,4'-thieno[2,3-c]pyran]-7'-yl)methyl)(methyl)carbamate (337.0 mg, 1.09 mmol) under nitrogen protection at -70°C, and heat-preservation stirring was carried out for 30 minutes after adding; after that, a tetrahydrofuran (2 mL)solution of N-fluorobisbenzenesulfonamide (412.0 mg, 1.30 mmol) was added, and stirring was continued at -70°C for 2 hours, then adding saturated aqueous ammonium chloride (10 mL) to quench the reaction, extracting with ethyl acetate (100 mL); the extract phase was washed with water (100 mL), concentrating, then the obtained crude product was purified by column chromatography to obtain the target product (250.0 mg, 70% yield).

LC-MS $[M+H-100]^+$: 228.1.

**Step f: synthesis of 1-(2'-fluoro-5'H,7'H-spiro[cyclopropane-1,4'-thieno[2,3 -c]pyran]-7'-yl)-N-methylmethylamine**

Dioxane hydrochloride (1 mL) was added to the dichloromethane (3 mL) solution of tert-butyl((2'-fluoro-5'H,7'H-spiro[cyclopropane-1,4'-thieno[2,3-c]pyran]-7'-yl)methyl )(methyl)carbamate (250.0 mg, 0.76 mmol), stirring for 3 hours after adding; after the reaction was completed, the reaction solution was concentrated, and the obtained crude product was separated and purified by preparative liquid chromatography (water/formic acid/acetonitrile) to obtain the target product (36.2 mg, 17.3% yield).

$^1H$ NMR (400 MHz, $CD_3OD$) δ 8.53 (s, 1 H), 6.17 (d, *J* = 2.0 Hz, 1 H), 5.13 - 5.05 (m, 1 H), 4.02 (d, *J* = 11.6 Hz, 1 H), 3.63 (d, *J* = 11.6 Hz, 1 H), 3.39 (dd, *J* = 12.8, 3.2 Hz, 1 H), 3.33 - 3.24 (m, 1 H), 2.75 (s, 3 H), 1.06 - 0.76 (m, 4 H).

LC-MS $[M+H]^+$: 228.1.

**Example 4: 1-(2'-chloro-5'H,7'H-spiro[cyclopropane-1,4'-thieno[2,3-c]pyran]-7'-yl)-N-methylmethylamine**

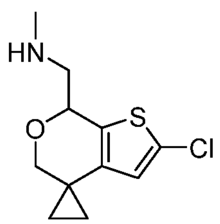

**Synthesis route 1:**

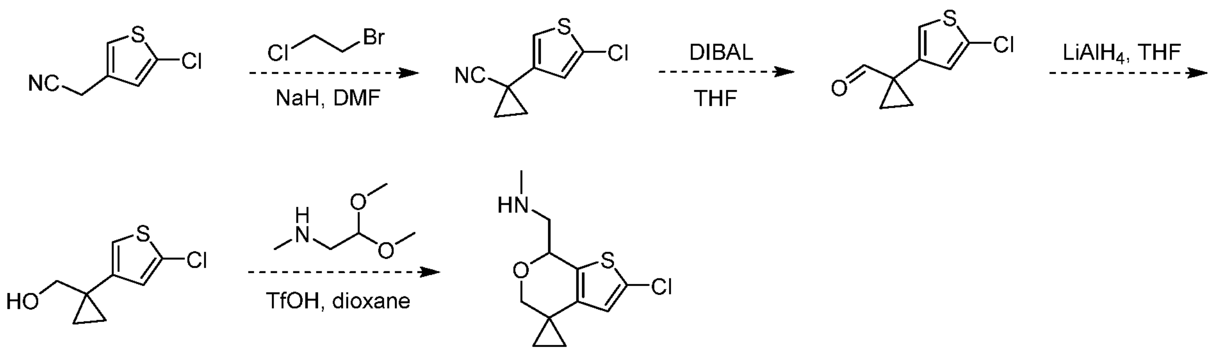


**Synthesis route 2:**

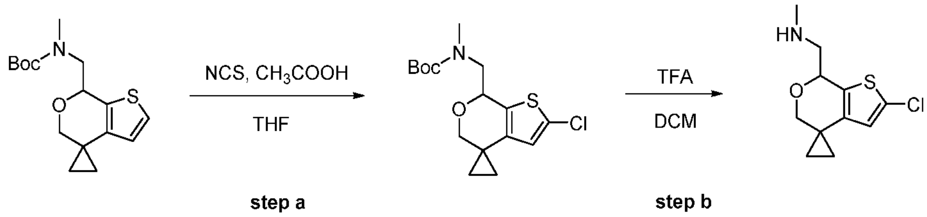


**Step a: synthesis of tert-butyl((2'-chloro-5'H,7'H-spiro[cyclopropane-1,4'-thieno[2,3-c]pyran]-7'-yl)methyl)(methyl)carbamate**

N-chlorosuccinimide (137.0 mg, 1.02 mmol) was added to the mixed solvent of acetic acid and tetrahydrofuran (2 mL: 8 mL) comprising tert-butyl((5'H, 7'H-spiro[cyclopropane-1,4'-thieno[2,3-c]pyran]-7'-yl)methyl)(methyl)carbamate (211.0 mg, 0.68 mmol) at 50°C, and heat-preservation stirring was carried out for 3 hours; after the reaction was completed, the reaction solution was cooled to room temperature and quenched with water (30 mL), then extracting with ethyl acetate (15 mL × 3); after the extract phase was concentrated, the crude product obtained was separated and purified by column chromatography (ethyl acetate: petroleum ethet=1:10) to obtain the target product (203.0 mg, 86.8% yield).
LC-MS $[M+H]^+$: 344.1.

**Step b: synthesis of 1-(2'-chloro-5'H,7'H-spiro[cyclopropane-1,4'-thieno[2, 3-c]pyran]-7'-yl)-N-methylmethylamine**

Trifluoroacetic acid (2 mL) was added to the dichloromethane (5 mL) solution of tert-butyl((2'-chloro-5'H,7'H-spiro[cyclopropane-1,4'-thiene[2,3-c]pyran]-7'-yl)methyl )(methyl)carbamate (203.0 mg, 0.59 mmol) at room temperature, stirring for 30 minutes; after the reaction was completed, the reaction solution was concentrated, and the crude product was separated and purified by preparative liquid chromatography (water/formic acid/acetonitrile) to obtain the target product (10.6 mg, 6.2% yield).
$^1$H NMR (400 MHz, $CD_3OD$) δ 8.55 (s, 1 H), 6.58 (s, 1 H), 5.13 (dd, *J* = 8.4, 2.8 Hz, 1 H), 4.00 (dd, *J* = 11.6, 1.2 Hz, 1 H), 3.62 (d, *J* = 11.6 Hz, 1 H), 3.41 (dd, *J* = 12.8, 3.2 Hz, 1 H), 3.33 - 3.25 (m, 1 H), 2.74 (s, 3 H), 1.10 - 0.82 (m, 4 H).
LC-MS $[M+H]^+$: 244.1.

**Example 5:1-(2'-methyl-5'H,7'H-spiro[cyclopropane-1,4'-thieno[2,3-c]pyr-a n]-7'-yl)-N-methylmethylamine**

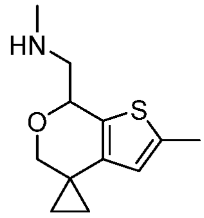

**Synthesis route 1:**

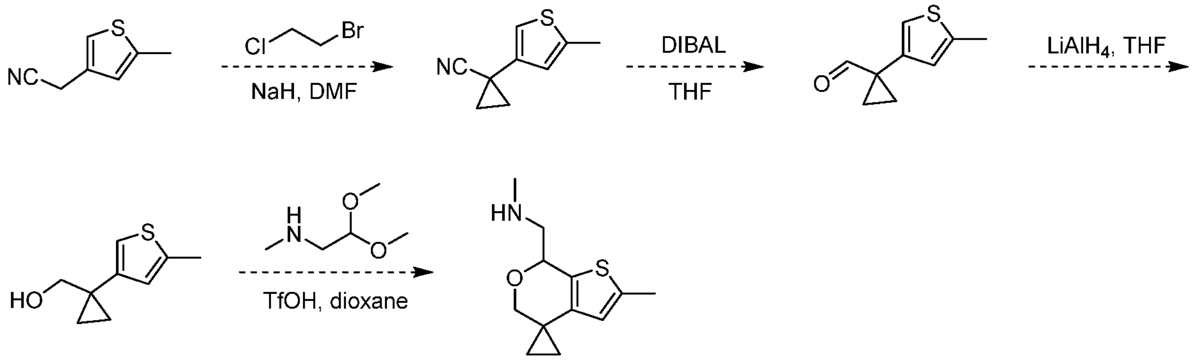


**Synthesis route 2:**

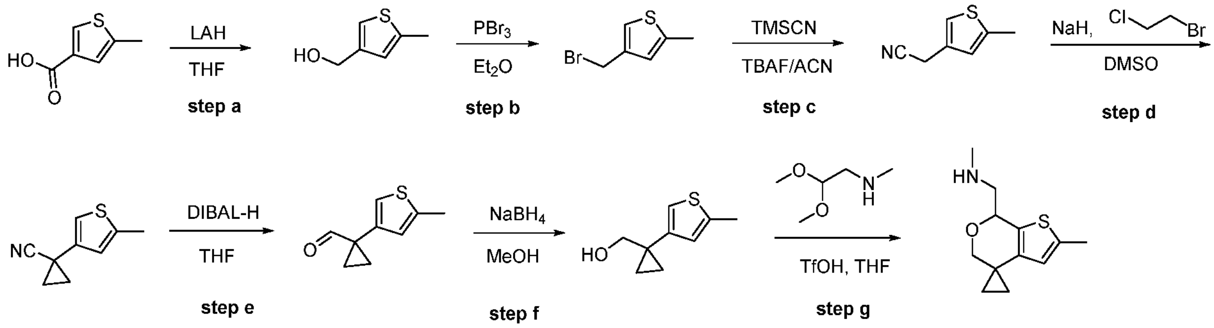


**Step a: synthesis of (5-methylthiophen-3-yl)methanol**

5-methylthiophene-3-carboxylic acid (7.4g, 52.1mmol) was dissolved in tetrahydrofuran (200mL), under nitrogen protection and in an ice bath, a tetrahydrofuran solution of lithium aluminum hydride (2.5 M in THF, 31.0 mL, 78.0 mmol) was slowly added dropwise to the above solution, then stirring at room temperature for 12 hours; after the reaction was completed, ethyl acetate (200 mL) and dilute hydrochloric acid (1.0 M, 20 mL) were successively added in ice bath to quench the reaction and stirring was continued for 1 hour. The above mixture was dried with anhydrous sodium sulfate, filtering and concentrating, and then the crude product was separated and purified by column chromatography to obtain the target product (6.7 g, 99% yield).

$^1H$ NMR (400 MHz, $CDCl_3$): $\delta$ 6.96 (s, 1 H), 6.75 (s, 1 H), 4.59 (s, 2 H), 2.47 (s, 3 H) 1.75 (brs, 1H).

**Step b: synthesis of 4-(bromomethyl)-2-methylthiophene**

Phosphorus tribromide (4.2 g, 15.6 mmol) was added to an ether(100 mL) solution of (5-methylthiophen-3-yl)methanol (4.0 g, 31.3 mmol) under nitrogen protection and in an ice bath, diluting with ethyl acetate (200mL) after stirring for 30 minutes; the mixture was washed with water (100mL), the organic phase was separated , drying with anhydrous sodium sulfate, filtering and concentrating, then the crude product was separated and purified by column

chromatography to obtain the target product (4.2g, 70.2% yield).

$^1H$ NMR (400 MHz, $CDCl_3$): δ 7.10 (s, 1H), 6.80 (s, 1H), 4.12 (s, 2H), 2.45 (s, 3H).

**Step c: synthesis of 2-(5-methylthiophen-3-yl)acetonitrile**

Trimethylsilyl nitrile (6.2 g, 63 mmol) and tetrabutylammonium fluoride (1 M in THF, 63 mL, 63 mmol) were slowly added to the acetonitrile (70 mL) solution of 4-(bromomethyl)-2-methylthiophene (4.0 g, 21 mmol) under nitrogen protection, stirring at room temperature for 14 hours, then the reaction solution was diluted with ethyl acetate (400 mL), washing with dilute hydrochloric acid (0.5 M, 200 mL × 2), water (200 mL), and saturated salt solution (200 mL) successively; the organic phase was dried with anhydrous sodium sulfate, filtering and concentrating, then the crude product was purified by column chromatography to obtain the target product (2.6 g, 90.2% yield).

$^1H$ NMR (400 MHz, $CDCl_3$): δ 6.97 (s, 1 H), 6.69 (s, 1 H), 3.64 (s, 2 H), 2.47 (s, 3 H).

**Step d: synthesis of 1-(5-methylthiophen-3-yl)cyclopropanenitrile**

(5-methylthiophene-3-yl)acetonitrile (2.6 g, 19.0 mmol) and 1-bromo-2-chloroethane (6.89 g, 47.0 mmol) were added to a dimethyl sulfoxide (20 mL) solution of sodium hydride (60%, 1.89 g, 47.0 mmol), stirring at room temperature for 14 hours, then the reaction solution was poured into water (100 mL) for quenching; the mixture was extracted with ethyl acetate (200 mL), the extract liquors was washed with water (300 mL × 2) and saturated salt solution (50 mL), drying with anhydrous sodium sulfate, filtering and concentrating, then the crude product was separated and purified by column chromatography (petroleum ether: ethyl acetate=20:1-10:1) to obtain the target product (2.9 g, 93.5% yield).

$^1H$ NMR (400 MHz, $CDCl_3$): δ 6.91 (d, *J* = 1.6 Hz, 1 H), 6.57 (s, 1 H), 2.45 (s, 3 H), 1.68 - 1.58 (m, 2 H), 1.36 - 1.26 (m, 2 H).

**Step e: synthesis of 1-(5-methylthiophen-3-yl)cyclopropane aldehyde**

Diisobutyl aluminum hydride (1M in hexane, 7.5 mL, 7.5 mmol) was slowly added dropwise to a tetrahydrofuran (20 mL) solution of 1-(5-methylthiophene-3-yl) cyclopropanenitrile (815 mg, 5.0 mmol) in an ice bath and under nitrogen protection, stirring at room temperature for 3 hours; after the reaction was completed, water (0.5 mL) was added to quench the reaction, and ethyl acetate (100 mL) was added to dilute, then washing with water (100 mL × 3) and saturated salt solution (50 mL); the organic phase was separated and dried with anhydrous sodium sulfate, filtering and concentrating, then the crude product was separated and purified by column chromatography to obtain the target product (800.0 mg, 96.2% yield).

$^1H$ NMR (400 MHz, $CDCl_3$): δ 9.22 (s, 1 H), 6.94 (d, *J* = 1.6 Hz, 1 H), 6.71 (s, 1 H), 2.47 (d, *J* = 1.2 Hz, 3 H), 1.56 - 1.48 (m, 2 H), 1.42 - 1.32 (m, 2 H).

**Step f: synthesis of (1-(5-methylthiophen-3-yl)cyclopropyl)methanol**

Sodium borohydride (201.0 mg, 5.3 mmol) was slowly added to a methanol (20mL)solution of 1-(5-methylthiophen-3-yl)cyclopropane aldehyde (800.0 mg, 4.8 mmol) in an ice bath, stirring at room temperature for 1 hour; after the reaction was completed, the reaction was quenched with dilute hydrochloric acid (1.0M, 2mL), then ethyl acetate (50mL) was added for dilution, washing with water (50mL × 2) and saturated salt solution (30mL); the organic phase was separated, drying with anhydrous sodium sulfate, filtering and concentrating, then the crude product was separated and purified by column chromatography (petroleum ether: ethyl acetate = 1:1) to obtain the target product (697.0 mg, 86.3% yield).

$^1H$ NMR (400 MHz, $CDCl_3$): δ 6.84 (d, *J* = 1.2 Hz, 1 H), 6.65 (s, 1 H), 3.67 (s, 2 H), 2.45 (s, 3 H), 0.90 - 0.75 (m,4 H).

**Step g: synthesis of 1-(2'-methyl-5'H,7'H-spiro[cyclopropane-1,4'-thieno[2, 3-c]pyran]-7'-yl)-N-methylmethylamine**

A 2-methyltetrahydrofuran (3 mL) solution of trifluoromethanesulfonic acid (900.0 mg, 6.0 mmol) was slowly added dropwise to a tetrahydrofuran (3 mL) solution comprising (1-(5-methylthiophen-3-yl)cyclopropyl)methanol (168.0 mg, 1.0 mmol) and methylaminoacetaldehyde dimethyl acetal (120.0 mg, 1.0 mmol), stirring overnight at room temperature; after the reaction was completed, diluting with dichloromethane (50mL), washing with 5% sodium hydroxide aqueous solution (100 mL), water (100 mL), and saturated salt solution (50 mL) successively; the organic phase was separated, drying with anhydrous sodium sulfate, filtering and concentrating, then the crude product was purified by column chromatography (petroleum ether: ethyl acetate = 10:1) and preparative liquid phase chromatography to obtain the target product (11.6 mg, 5.2% yield).

$^{1}H$ NMR (400 MHz, $CD_3OD$): δ 6.23 (s, 1 H), 4.97 - 4.88 (m, 1 H), 3.90 (d, *J* = 15.6 Hz, 1 H), 3.52 (d, *J* = 15.2 Hz, 1 H), 2.92 - 2.84 (m, 2 H), 2.46 (s, 3 H), 2.40 (s, 3 H), 1.05 - 0.67 (m, 4 H).

LC-MS $[M+H]^+$: 224.1.

**Example 6:1-(3'-fluoro-5'H,7'H-spiro[cyclopropane-1,4'-thieno[2,3-c]pyran] -7'-yl)-N-methylmethylamine**

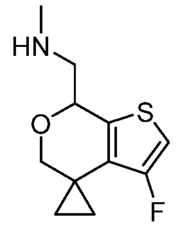

**Synthesis route:**

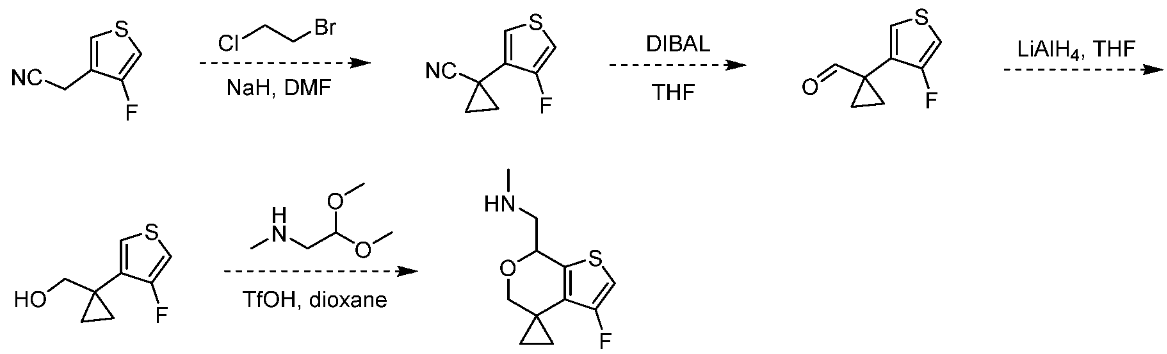


LC-MS $[M+H]^+$: 228.1.

**Example 7:1-(3'-methyl-5'H,7'H-spiro[cyclopropane-1,4'-thieno[2,3-c]pyr-a n]-7'-yl)-N-methylmethylamine**

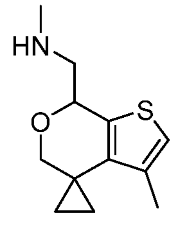

**Synthesis route:**

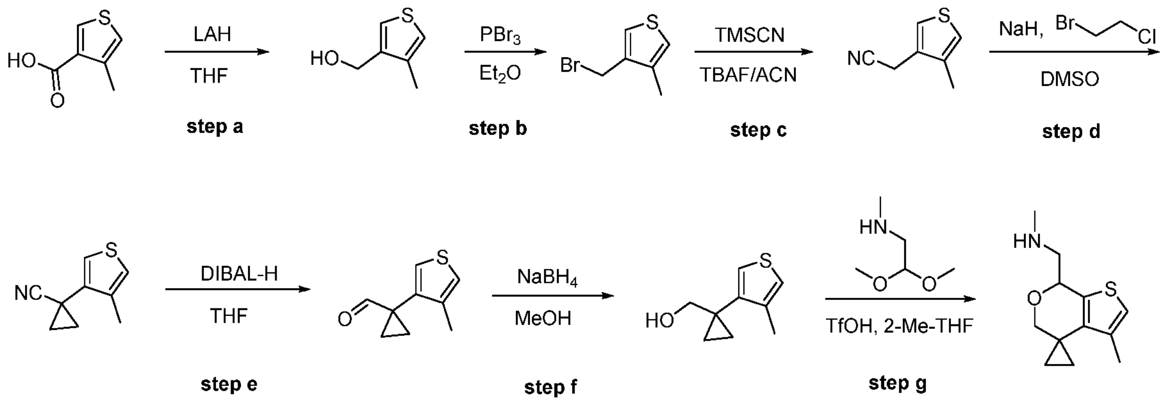


**Step a: synthesis of (4-methylthiophen-3-yl)methanol**

Lithium aluminum hydride (2.5 M in THF, 60 mL, 150 mmol) was slowly added to tetrahydrofuran (100 mL) solution of 4-methylthiophen-3-carboxylic acid (10.0 g, 70.4 mmol) in an ice bath and under nitrogen protection, stirring at room temperature for 12 hours; after the reaction was completed, dilute hydrochloric acid (1.0 M, 15 mL) was added to the reaction solution in ice bath to quench the reaction, then extracting with ethyl acetate (400mL × 3), combining the extract liquors and washing with water (400 mL × 3) and saturated salt solution (400 mL × 3), and then drying with anhydrous sodium sulfate, filtering and concentrating, finally the crude product was purified by column chromatography to obtain the target product (4.0g, 44.3% yield).

$^1$H NMR (400MHz, $CDCl_3$): δ7.20(s, 1H), 6.94(s, 1H), 4.63(s, 2H), 2.26(s, 3H).

**Step b: synthesis of 3-(bromomethyl)-4-methylthiophene**

Phosphorus tribromide (2.85 g, 10.5 mmol) was slowly added to a ether (30 mL) solution of (4-methylthiophen-3-yl)methanol (2.7 g, 21.1 mmol) in an ice bath and under nitrogen protection, after reacting for 30 minutes, dilute hydrochloric acid (2.5 M, 20 mL) was added to quench the reaction, extracting with ethyl acetate (400mL × 3), combining the extract liquors and washing with water (400mL × 3) and saturated salt solution (400mL × 3), and then drying with anhydrous sodium sulfate, filtering and concentrating, finally the crude product was separated and purified by column chromatography to obtain the target product (1.5 g, 37.5% yield).

$^1$H NMR (400MHz, $CDCl_3$): δ7.29(s, 1H), 6.95(s, 1H), 4.48(s, 2H), 2.29(s, 3H).

**Step c: synthesis of 2-(4-methylthiophen-3-yl)acetonitrile**

Trimethylsilylnitrile (2.3g, 23.7 mmol) and tetrabutylammonium fluoride (5.8 g, 23.7 mmol) were added to acetonitrile (30 mL) solution of 3-(bromomethyl) -4-methylthiophene (1.5 g, 7.9 mmol) under nitrogen protection, stirring at room temperature for 14 hours; after the reaction was completed, water (200 mL) was added in ice bath, extracting with ethyl acetate (300 mL × 3), combining the extract liquors and washing with water (300 mL × 3) and saturated salt solution (300 mL × 3), then drying with anhydrous sodium sulfate, filtering and concentrating, finally the crude product was purified by column chromatography (petroleum ether: ethyl acetate = 10:1) to obtain the target product (1.0g, 92.6% yield).

$^1$H NMR (400MHz, $CDCl_3$): δ7.25(s, 1H), 6.99(s, 1H), 3.59(s, 2H), 2.22(s, 3H).

**Step d: synthesis of 1-(4-methylthiophen-3-yl)cyclopropanenitrile**

2-(4-methylthiophen-3-yl)acetonitrile (1.0 g, 7.3mmol) and 1-bromo-2-chloroethane (2.6 g, 18.3 mmol) were slowly added to dimethyl sulfone (20 mL) solution of sodium hydride (60%, 0.74 g, 18.3 mmol), stirring at room temperature for 14 hours, then the reaction was quenched in ice bath with water (300 mL), extracting with ethyl acetate (300 mL × 3). The extract liquor was washed with water (200 mL × 3) and saturated salt solution (200 mL × 3), then drying with anhydrous sodium sulfate, filtering and concentrating, finally the crude product was separated and purified by column chromatography to obtain the target product (1.0g, 83.9% yield).

$^1$H NMR (400MHz, $CDCl_3$): δ7.09 (d, J=3.2Hz, 1H), 6.96-6.92 (m, 1H), 2.40(s, 3H), 1.65-1.57(m, 2H), 1.29-1.20 (m, 2H).

**Step e: synthesis of 1-(4-methylthiophen-3-yl)cyclopropane aldehyde**

Diisobutylaluminum hydride (1M in hexane, 12.2mL, 12.2mmol) was slowly added to atetrahydrofuran (15 mL) solution of 1-(4-methylthiophen-3-yl)cyclopropanenitrile (1.0 g, 6.1 mmol) in an ice bath and under nitrogenprotection, stirring at room temperature for 16 hours; after that,water (300mL) was added in ice bath to quench the reaction, extracting with ethyl acetate (300mL × 3); the extract liquor was washed with water (300mL × 3) and saturated salt solution (300mL × 3), drying with anhydrous sodium sulfate and filtering and concentrating, then the crude product was purified by column chromatography (petroleum ether: ethyl acetate = 10:1) to obtain the target product (450.0 mg, 44.5% yield).

$^1$H NMR (400MHz, $CDCl_3$): δ9.10(s, 1H), 7.06(d, J=3.2Hz, 1H), 6.98-6.93(m, 1H), 2.18(s, 3H), 1.66-1.50(m, 2H), 1.40-1.28 (m, 2H).

**Step f: synthesis of (1-(4-methylthiophen-3-yl)cyclopropyl)methanol**

Sodium borohydride (90.0 mg, 2.3 mmol) was slowly added to a methanol (7 mL) solution of 1-(4-methylthiophen-3-yl)cyclopropane aldehyde (350 mg, 2.1 mmol) in an ice bath, stirring at room temperature for 2 hours; after the reaction was completed, the reaction solution was poured into water (50mL), extracting with ethyl acetate (50mL × 3); the extract liquor was washed with water (50mL × 3) and saturated salt solution (50mL × 3) successively, drying with anhydrous sodium sulfate, filtering and concentrating, then the crude product was purified by column chromatography to obtain the target product (280.0 mg, 79.2% yield).

$^1$H NMR (400MHz, $CDCl_3$): δ7.10 (d, J=4.4Hz, 1H), 6.93-6.87(m, 1H), 3.54(s, 2H), 2.29(s, 3H), 0.89-0.76(m, 4H).

**Step g: synthesis of 1-(3'-methyl-5'H,7'H-spiro[cyclopropane-1,4'-thieno[2, 3-c]pyran]-7'-yl)-N-methylmethylamine**

Trifluoromethanesulfonic acid (900.0 mg, 5.5 mmol) was slowly added dropwise to a 2-methyl-tetrahydrofuran solution of (1-(4-methylthiene-3-yl)cyclopropyl)methanol (170.0 mg, 1.0 mmol) and methylaminoacetaldehyde dimethyl acetal(120.0 mg, 1 mmol) in an ice bath, stirring at room temperature for 19 hours; after that, methylene chloride (50 mL) was added for dilution in an ice bath, and the mixture was washed with 5% sodium hydroxide aqueous solution (50 mL × 3) and saturated salt solution (50 mL × 3), and the organic phase was separated, drying with anhydrous sodium sulfate, filtering and concentrating, then the crude product was purified by column chromatography (petroleum ether: ethyl acetate = 10:1) to obtain the target product (58.9 mg, 26.1% yield).

$^1$H NMR (400 MHz, $CD_3OD$): δ 6.83 (s, 1 H), 5.02 - 4.92 (m, 1 H), 3.88 (dd, *J* = 15.6, 2.0 Hz, 1 H), 3.39 (d, *J* = 15.6 Hz, 1 H), 2.99 - 2.82 (m,2 H), 2.46 (s, 3 H), 2.10 (s, 3 H), 1.61 - 1.47 (m,1 H), 1.21-1.09 (m,1 H), 0.89 - 0.77 (m,1 H), 0.68 - 0.55 (m,1 H).

LC-MS $[M+H]^+$: 224.2.

**Example 8: 1-(5'H,7'H-spiro[cyclopropane-1,4'-thieno[2,3-c]pyran]-7'-yl)-N -methylmethylamine**

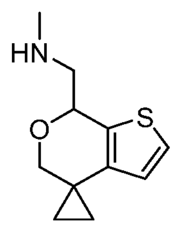

**Synthesis scheme:**

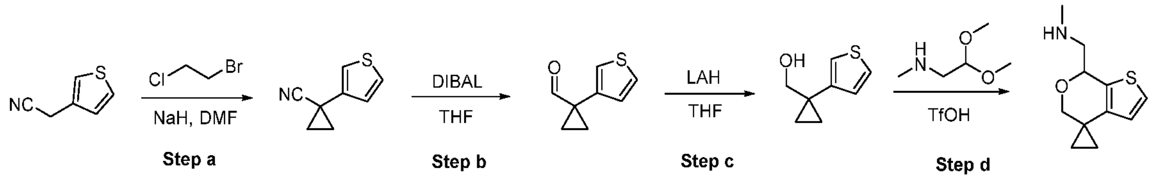


**Step a: synthesis of 1-(thiophen-3-yl)cyclopropane acetonitrile**

2-(thiophene-3-yl)acetonitrile (5g, 40.59mmol) was dissolved in N, N-dimethylformamide (130mL), then adding NaH (60%, 4.06 g, 101.48mmol) in batches at 0°C, stirring for 0.5 hours, and then a N, N-dimethylformamide (20 mL) solution of 1-bromo-2-chloroethane (11.64 g, 81.39 mmol) was slowly added dropwise to the reaction solution, then reacting at room temperature for 15 hours; after that, the reaction solution was poured into 400 mL of water, extracting twice with ethyl acetate (300 mL $\times$ 2), combining the organic phase and washing with saturated saline solution (300 mL), drying with anhydrous sodium sulfate, filtering and concentrating to obtain the crude product; after separation and purification (petroleum ether: ethyl acetate=10:1) by column chromatography, the target compound was obtained (4.2 g, yield 69.3%).

LC-MS $[M+H]^+$: 150.1.

$^1H$ NMR ($CDCl_3$, 600 MHz): $\delta$ 7.33 - 7.29 (m, 1 H), 7.20 - 7.17 (m, 1 H), 6.91 (dd, *J* = 5.4, 1.2 Hz, 1 H), 1.71 - 1.67 (m, 2 H), 1.38 - 1.34 (m, 2 H).

**Step b: synthesis of 1-(thiophen-3-yl)cyclopropane formaldehyde**

1-(thiophen-3-yl)cyclopropane acetonitrile (4.2g, 28.15mmol)was dissolved in 100mL of tetrahydrofuran, then diisobutylaluminum hydride (1M in hexane, 56.30mL, 56.30mmol ) solution was slowly added dropwise in an ice bath. After the dropwise addition was completed, the mixture was reacted at room temperature for 2 hours; then the reaction solution was slowly poured into 300mL of ammonium chloride aqueous solution, adding 300mL of ethyl acetate for extraction, layering to obtain the organic phase, then extracting the aqueous phase with ethyl acetate (200mL), combining the organic phases, drying with anhydrous sodium sulfate, filtering and concentrating to obtain the crude product, which was separated and purified by column chromatography (petroleum ether: ethyl acetate = 10:1) to obtain the target compound (3.0g, yield 70.1%).

LC-MS $[M+H]^+$: 153.1.

$^1H$ NMR ($CDCl_3$, 600 MHz): $\delta$ 9.23 (s, 1 H), 7.34 - 7.31 (m, 1 H), 7.25 - 7.22 (m, 1 H), 7.05 (dd, *J* = 4.8, 1.2 Hz, 1 H), 1.60 - 1.55 (m, 2 H), 1.43 - 1.39 (m, 2 H).

**Step c: synthesis of 1-(thiophen-3-yl)cyclopropanemethanol**

1-(thiophen-3-yl)cyclopropane formaldehyde (1.0g, 6.57mmol) was dissolved in tetrahydrofuran (20mL), and lithium aluminum hydride (0.25g, 6.57mmol) was added in an ice bath and the reaction was continued for 10 minutes; then ethyl acetate was slowly added dropwise to the reaction solution to quench the reaction, and the solvent was spin-dried to obtain the crude product, which was separated and purified by column chromatography (petroleum ether: ethyl acetate=4:1) to obtain the target compound (0.9g, yield 89 %).

LC-MS $[M+H]^+$: 155.1.

$^1H$ NMR ($CDCl_3$, 600 MHz): $\delta$ 7.27 - 7.24 (m, 1 H), 7.12 - 7.09 (m, 1 H), 6.97 (dd, *J* = 4.8, 1.2 Hz, 1 H), 3.69 (d, *J* = 4.8 Hz, 2 H), 0.91 - 0.81 (m, 4 H).

**Step d: synthesis of 1-(5'H,7'H-spiro[cyclopropane-1,4'-thieno[2,3-c]pyran] -7'-yl)-N-methylmethylamine**

1-(thiophen-3-yl)cyclopropanemethanol (450.0mg, 2.92mmol) and 2,2-dimethoxy-N-methylethylamine (417.5mg, 3.50mmol) were dissolved in 9mL of dioxane, then adding trifluoromethanesulfonic acid (1314.7mg, 8.76mmol) dropwise in an ice bath, stirring at room temperature for 15 hours; then the reaction solution was poured into 30 mL of water, alkalizing with saturated sodium bicarbonate aqueous solution, extracting with ethyl acetate (30 mLx2), combining the organic phases and drying with anhydrous sodium sulfate, filtering and concentrating to obtain the crude product, which was separated and purified by column chromatography (dichloromethane: methanol=10:1) to obtain the target product (90.0 mg, yield 14.7%).

LC-MS $[M+H]^+$: 210.1.

$^1H$ NMR ($CD_3OD$, 600 MHz): $\delta$ 7.29 (d, *J* = 4.8 Hz, 1 H), 6.58 (d, *J* = 5.4 Hz, 1 H), 5.21 (dd, *J* = 9.0, 3.0 Hz, 1 H), 3.93 (dd, *J* = 12.0, 1.2 Hz, 1 H), 3.59 (d, *J* = 11.4 Hz, 1 H), 3.49 (dd, *J* = 13.2, 3.0 Hz, 1 H), 3.30 -3.24 (m, 1 H), 2.73

(s, 3 H), 1.07-0.76 (m, 4 H).

**Example 8-R and Example 8-S:**

**(R)-1-(5'H,7'H-spiro[cyclopropane-1,4'-thieno[2,3-c]pyran]-7'-yl)-N-methyl-methylamine and (S)-1-(5'H,7'H-spiro[cyclopropane-1,4'-thieno[2,3-c]pyran]-7' -yl)-N-methylmethylamine**

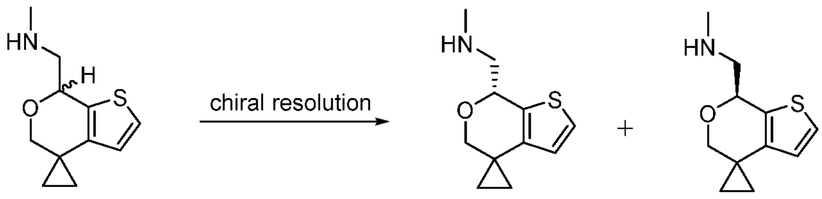


**8** **8-R** **8-S**

Example 8 was resolved by chiral chromatographic column to obtain optical enantiomer 1 (Example 8-R) and optical enantiomer 2 (Example 8-S). Liquid chromatography method: chromatographic column: DAICEL CHIRALPAK IG column; mobile phase A: supercritical $CO_2$, mobile phase B: methanol (containing 0.1% dimethylamine); detection wavelength: 214nm; flow rate: 1.5mL/min; column temperature: 35°C; background column pressure: 1800psi. Mobile phase gradient:

| Time (min) | A(%V/V) | B(%V/V) |
|---|---|---|
| 0.00 | 95 | 5 |
| 0.50 | 95 | 5 |
| 5.50 | 60 | 40 |
| 8.00 | 60 | 40 |

Optical Enantiomer 1 (Example 8-R):

RT: 3.29 min; $[\alpha]D^{29}$ = 81.9 (c 0.177, MeOH); LC-MS $[M+H]^+$: 210.1.

$^1H$ NMR ($CDCl_3$, 600 MHz): $\delta$ 7.12 (dd, *J* = 4.8, 0.6 Hz, 1 H), 6.48 (d, *J*= 5.4 Hz, 1 H), 4.99 (dd, *J* = 7.8, 4.8 Hz, 1 H), 3.99 (dd, *J* = 11.4, 1.2 Hz, 1 H), 3.50 (d, *J* = 11.4 Hz, 1 H), 3.01-2.94 (m, 2 H), 2.52 (s, 3 H), 1.04 - 0.75 (m, 4 H).

Optical antipode 2 (Example 8-S):

RT: 3.67 min; $[\alpha]D^{29}$ = -109.8 (c 0.186, MeOH); LC-MS $[M+H]^+$: 210.1.

$^1H$ NMR ($CDCl_3$, 600 MHz): $\delta$ 7.12 (dd, *J* = 5.4, 1.2 Hz, 1 H), 6.48 (d, *J*= 4.8 Hz, 1 H), 4.99 (dd, *J* = 7.8, 4.2 Hz, 1 H), 3.99 (dd, *J* = 11.4, 1.2 Hz, 1 H), 3.50 (d, *J* = 11.4 Hz, 1 H), 3.02 - 2.93 (m, 2 H), 2.52 (s, 3 H), 1.05 - 0.74 (m, 4 H).

**Example 9:1-(4'H,6'H-spiro[cyclopropane-1,7'-thieno[3,2-c]pyran]-4'-yl)-N-methylmethylamine**

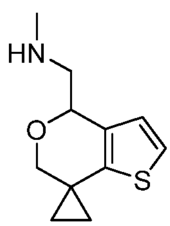

**Synthesis route 1:**

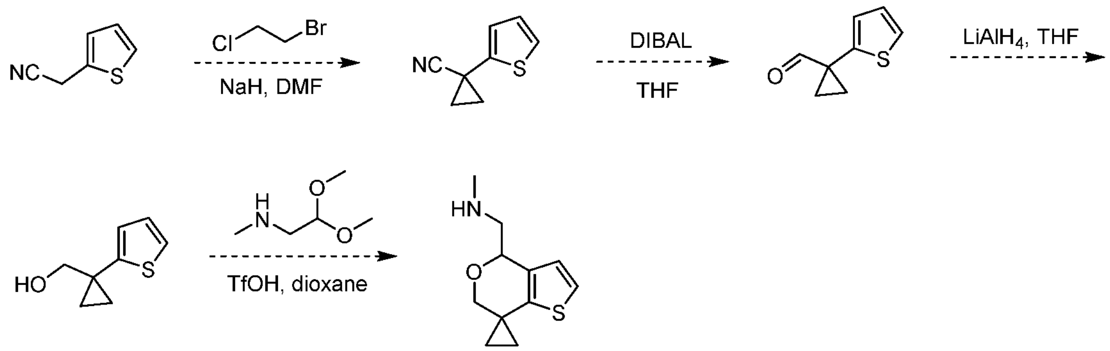


**Synthesis route 2:**

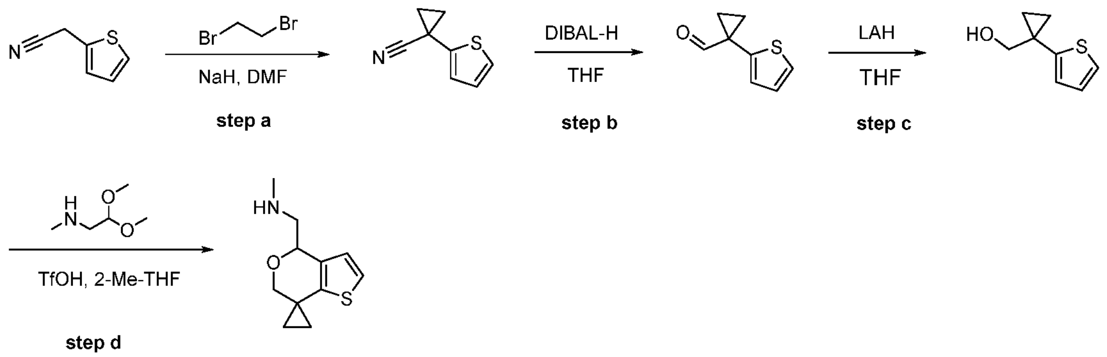


**Step a: synthesis of 1-(thiophen-2-yl)cyclopropanenitrile**

Sodium hydride (60%, 1.6g, 40.59mmol) was added to a N, N-dimethylformamide (30mL) solution of 2-(thiophen-2-yl) acetonitrile (2.0g, 16.24mmol)under nitrogen protection and in an ice bath, stirring for 1 hour; then 1,2-dibromoethane (4.0g, 21.3mmol) was slowly added, slowly heating the reaction solution to room temperature and stirring overnight. After the reaction was completed, water (300 mL) was added to quench the reaction in ice bath, extracting with ethyl acetate (300 mL × 3); the extract phase was washed with water (200 mL × 3) and saturated salt solution (200 mL × 3), drying with anhydrous sodium sulfate, filtering and concentrating, then the crude product was separated and purified by column chromatography (petroleum ether/ethyl acetate) to obtain the target product (1.5g, 61.9% yield).

$^1H$ NMR (300 MHz, $CDCl_3$) $\delta$ 7.17 (dd, $J$ = 5.1, 0.6 Hz, 1 H), 7.07 - 7.02 (m, 1 H), 6.95 - 6.89 (m, 1 H), 1.78 - 1.67 (m, 2 H), 1.47 - 1.37 (m, 2 H).

**Step b: synthesis of 1-(thiophen-2-yl)cyclopropane aldehyde**

Diisobutylaluminum hydride (1M in hexane, 18.8mL, 18.8mmol) was slowly added dropwise to a tetrahydrofuran (100mL) solution of 1-(thiophen-2-yl)cyclopropanenitrile (1.4g, 9.38mmol) in an ice bath and under nitrogen protection, and stirring was continued for 3 hours at room temperature; after the reaction was completed, water (300mL) was added to quench the reaction in ice bath, extracting with ethyl acetate (300 mL × 3); the extract phase was washed with water (300mL × 3) and saturated salt solution (300mL × 3), drying with anhydrous sodium sulfate, filtering and concentrating, then the crude product was separated and purified by column chromatography (petroleum ether/ethyl acetate) to obtain the target product (632.0mg, 44.3% yield).

$^1H$ NMR (300 MHz, $CDCl_3$) $\delta$ 9.34 (s, 1 H), 7.27 - 7.20 (m, 1 H), 7.03 - 6.96 (m, 2 H), 1.73 - 1.64 (m, 2 H), 1.55 - 1.47 (m, 2 H).

**Step c: synthesis of (1-(thiophen-2-yl)cyclopropyl)methanol**

A tetrahydrofuran solution of lithium aluminum hydride (2.5M in THF, 3.3mL, 8.30mmol) was slowly added dropwise to a tetrahydrofuran (20mL) solution of 1-(thiophen-2-yl)cyclopropane aldehyde (632.0mg, 4.15mmol) in an ice bath and under nitrogen protection, stirring at room temperature for 2 hours; after the reaction was completed, water (200mL) was added to quench the reaction in ice bath, extracting with ethyl acetate (300mL × 3); the extract phase was washed with water (300mL × 3) and saturated salt solution (300mL × 3), drying with anhydrous sodium sulfate, filtering and concentrating, then the crude product was separated and purified by column chromatography to obtain the target product (570.0mg, 89.0% yield).

$^1H$ NMR (300 MHz, $CDCl_3$) δ 7.17- 7.11 (m, 1 H), 6.97 - 6.90 (m, 2 H), 3.68 (s, 2 H), 1.96 (s, 1 H), 1.06 - 0.89 (m, 4 H).

**Step d: synthesis of 1-(4'H,6'H-spiro[cyclopropane-1,7'-thieno[3,2-c]pyran] -4'-yl)-N-methylmethylamine**

2,2-dimethoxy-N-methylethylamine (386.0mg, 3.24mmol) and trifluoromethanesulfonic acid (0.8mL) were added to a 2-methyltetrahydrofuran (5mL) solution of (1-(thiophen-2-yl)cyclopropyl)methanol (250mg, 1.62mmol), stirring at 80°C for 20 minutes; after the reaction was completed, the pH of reaction solution was adjusted tol3 with 15% sodium hydroxide aqueous solution in ice bath, then diluting with water (20mL), extracting with ethyl acetate (20mL × 3); after concentrating the extract phase, the crude product was separated and purified by column chromatography (petroleum ether/ethyl acetate) to obtain the target product (7.3mg, 2.2% yield).

$^1H$ NMR (400 MHz, $CD_3OD$) δ 7.21 (d, *J* = 5.2 Hz, 1 H), 6.89 (d, *J* = 5.2 Hz, 1 H), 5.11 (d, *J* = 8.8 Hz, 1 H), 3.99 (d, *J* = 11.6 Hz, 1 H), 3.68 (d, *J* = 11.2 Hz, 1 H), 3.60 (dd, *J* = 12.8, 2.4 Hz, 1 H), 3.32-3.25 (m, 1 H), 2.77 (s, 3 H), 1.16 - 0.85 (m, 4 H).

LC-MS $[M+H]^+$: 210.1.

**Example 9-S and Example 9-R:**

**(*S*)-1-(4'H,6'H-spiro[cyclopropane-1,7'-thieno[3,2-c]pyran]-4'-yl)-N-methylm -ethylamine and (*R*)-1-(4'H,6'H-spiro[cyclopropane-1,7'-thieno[3,2-c]pyran]-4'-yl)-N-methylmethylamine**

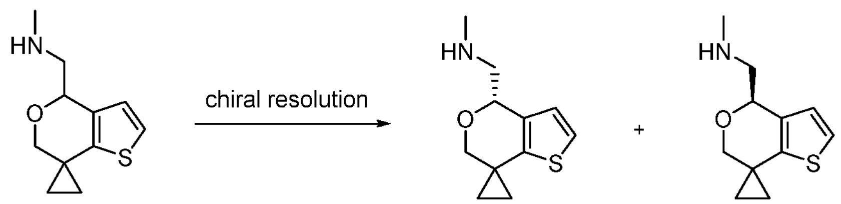


9 9-S 9-R

Example 9 was resolved by chiral chromatographic column to obtain optical enantiomer 1 (Example 9-S) and optical enantiomer 2 (Example 9-R). Liquid chromatography method: chromatographic column: DAICEL CHIRALPAK IG column; mobile phase A: supercritical $CO_2$, mobile phase B: methanol (containing 0.1% dimethylamine); detection wavelength: 214nm; flow rate: 1.5mL/min; column temperature: 35°C; background column pressure: 1800psi. Mobile phase gradient:

| time(min) | A(%V/V) | B(%V/V) |
|---|---|---|
| 0.00 | 95 | 5 |
| 0.50 | 95 | 5 |
| 5.50 | 60 | 40 |
| 10.00 | 60 | 40 |

Optical Enantiomer 1 (Example 9-S):

RT: 3.23 min; $[\alpha]D^{29}$ = 65.1 (c 0.175, MeOH); LC-MS $[M+H]^+$: 210.1.

$^1H$ NMR ($CDCl_3$, 600 MHz): $\delta$ 7.00 (d, *J* = 5.4 Hz, 1 H), 6.75 (d, *J* = 4.8 Hz, 1 H), 4.98 (dd, *J* = 9.0, 2.4 Hz, 1 H), 3.92 (d, *J* = 11.4 Hz, 1 H), 3.57 (d, *J* = 11.4 Hz, 1 H), 3.07 (dd, *J* = 12.6, 2.4 Hz, 1 H), 2.95 (dd, *J* = 12.0, 9.0 Hz, 1H), 2.54 (s, 3 H), 1.08-0.90 (m, 4 H).

Optical Enantiomer 2 (Example 9-R):

RT: 3.62 min; $[\alpha]D^{29}$ = -59.1 (c 0.163, MeOH); LC-MS $[M+H]^+$: 210.1.

$^1H$ NMR ($CDCl_3$, 600 MHz): $\delta$ 7.00 (d, *J* = 5.4 Hz, 1 H), 6.75 (d, *J* = 4.8 Hz, 1 H), 4.93 (dd, *J* = 9.0, 3.0 Hz, 1 H), 3.92 (dd, *J* = 11.4, 1.2 Hz, 1 H), 3.56 (d, *J* = 11.4 Hz, 1 H), 3.02 (dd, *J* = 12.6, 3.0 Hz, 1 H), 2.92 (dd, *J* = 12.6, 9 Hz, 1 H), 2.51 (s, 3 H), 1.05 - 0.90 (m, 4 H).

**Example 10:1-(6',7'-dihydrospiro[cyclopropane-1,4'-thieno[3,2-c]pyran]-7'-yl)-N-methylmethylamine**

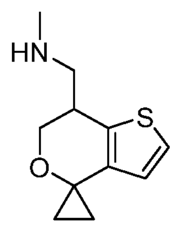

Synthesis route:

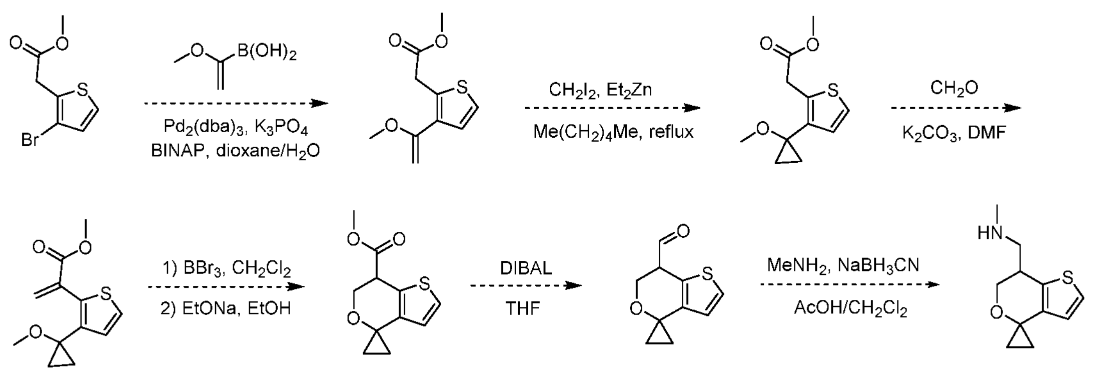


LC-MS $[M+H]^+$: 210.1.

**Example 11:1-(6'H-spiro[cyclopropane-1,4'-thieno[2,3-c]furan]-6'-yl)-N-m-e thylmethylamine**

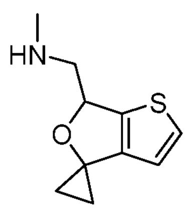

Synthesis route:

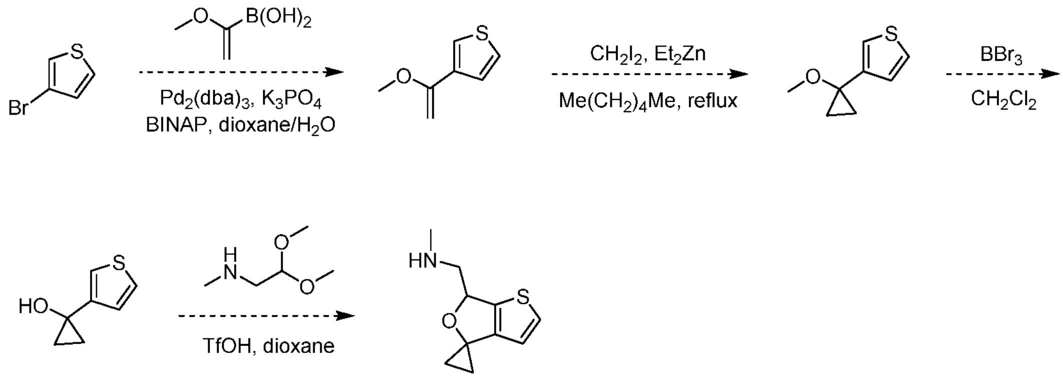


LC-MS $[M+H]^+$: 196.1.

**Example 12: 1-(6,7-dihydro-5H-spiro[benzo[b]thiophene-4,1'-cyclopropane] -7-yl)-N-methylmethylamine**

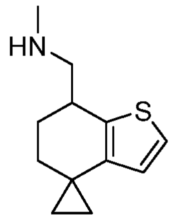

Synthesis route:

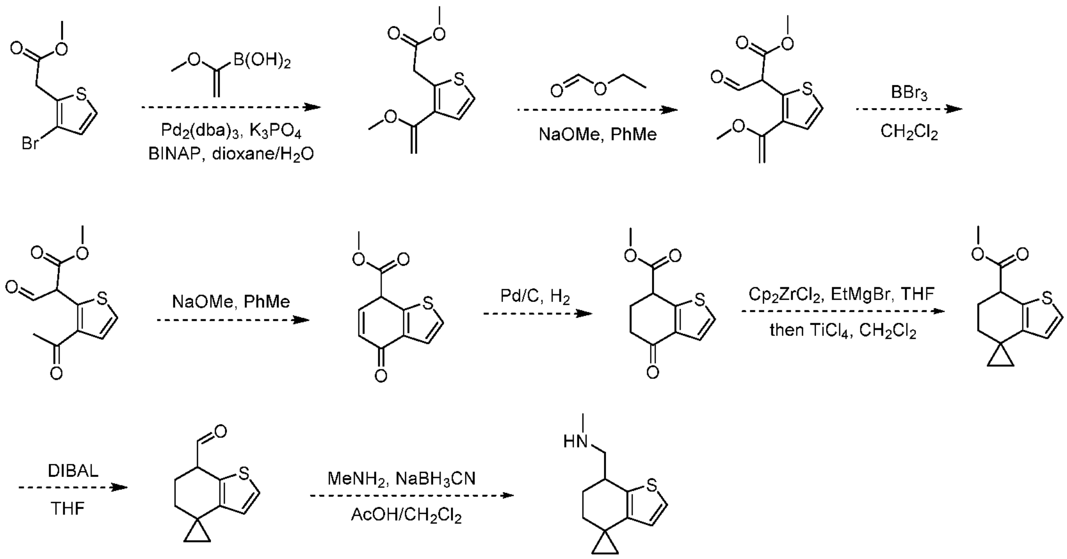


LC-MS $[M+H]^+$: 208.1.

**Example 13: 1-(5,6-dihydrospiro[cyclopentadieno[b]thiophene-4,1'-cyclopropane]-6-yl)-N-methylmethylamine**

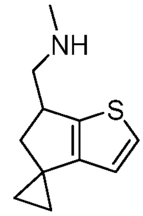

Synthesis route:

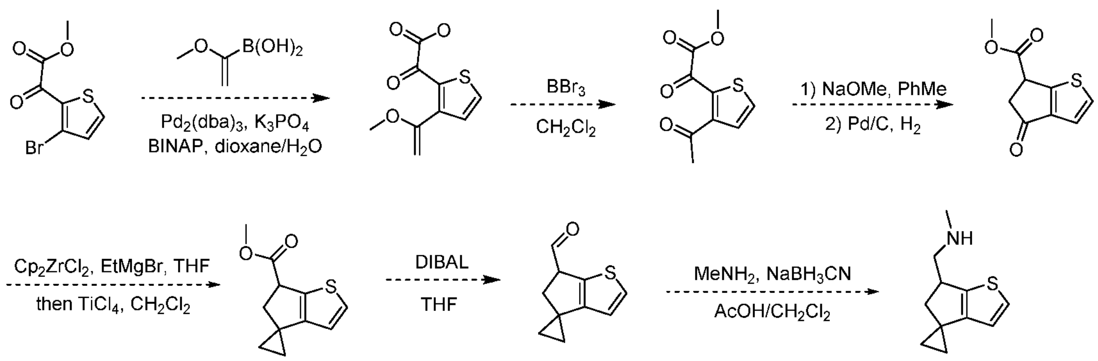


LC-MS $[M+H]^+$: 194.1.

**Example 15: N-((5'H,7'H-spiro[cyclopropane-1,4'-thieno[2,3-c]pyran]-7'-yl) methyl)methane-*d*$_3$-amine**

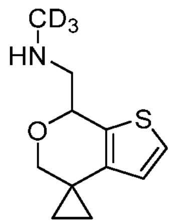

Synthesis route 1:

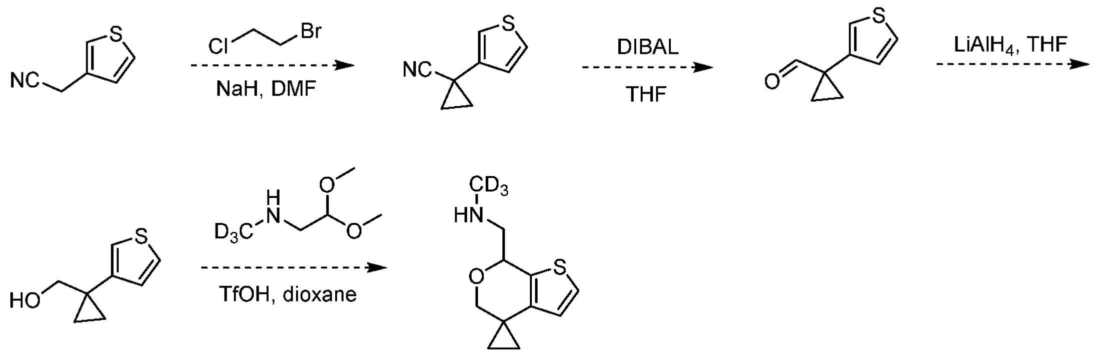


Synthesis route 2:

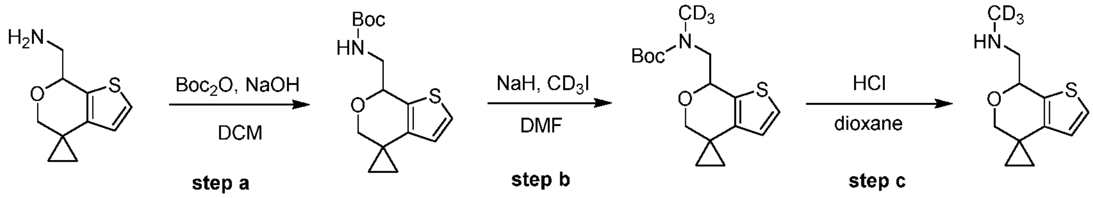


**Step a: synthesis of tert-butyl((5'H,7'H-spiro[cyclopropane-1,4'-thieno[2,3-c]pyran]-7'-yl)methyl)carbamate**

Sodium hydroxide (512.04mg, 12.80mmol) and di-tert-butyl dicarbonate (1.12g, 5.12mmol) were added to a dichloromethane (5mL) solution of (5'H,7'H-spiro[cyclopropane-1,4'-thieno[2,3-c]pyran]-7'-yl)methylamine (500.0mg, 2.56mmol), stirring at room temperature for 16 hours; after the reaction was completed, water (10 mL) was added to the reaction solution to quench the reaction, extracting with ethyl acetate (10mL × 3), then the extract phase was concentrated to obtain the crude product, which was then separated and purified by column chromatography to obtain the target product (430.0mg, 56.9% yield).

LC-MS $[M+H-56]^+$: 240.1.

**Step b: synthesis of tert-butyl((5'H,7'H-spiro[cyclopropane-1,4'-thieno[2,3 -c]pyran]-7'-yl)methyl)(methyl-*d*$_3$)carbamate**

Sodium hydride (60%, 48.8mg, 1.22mmol) and deuterated iodomethane (92.3mg, 0.64mmol) were added to N,N-dimethylformamide (6 mL) solution of tert-butyl(5'H,7'H-spiro[cyclopropane-1,4'-thieno[2,3-c]pyran]-7'-yl)methyl)carbamat e (180.0 mg, 0.61 mmol), stirring at room temperature for 16 hours; after the reaction was completed, water (10mL) was added to the reaction solution for dilution, extracting with ethyl acetate (10mL × 3), then the extract phase was concentrated to obtain the target crude product (600mg).

LC-MS $[M+H-100]^+$: 213.0.

**Step c: synthesis of N-((5'H,7'H-spiro[cyclopropane-1,4'-thieno[2,3-c]pyra-n]-7'-yl)methyl)methane-*d*$_3$-amine**

Dioxane hydrochloride (4M, 2mL) was added to a dioxane (6mL) solution of tert-butyl((5'H,7'H-spiro[cyclopropane-1,4'-thieno[2,3-c]pyran]-7'-yl)methyl)(methyl-*d*$_3$)carbamate (600mg of crude), stirring at room temperature for 2 hours; after the reaction was completed, the reaction solution was concentrated, and the crude product obtained was separated and purified by the preparative liquid chromatography to obtain the target product (21.2mg, 4.3% yield).

$^1H$ NMR (600 MHz, $CDCl_3$): δ 7.12 (d, *J* = 5.4 Hz, 1 H), 6.48 (d, *J* = 5.4 Hz, 1 H), 5.01 - 4.97 (m, 1 H), 3.98 (d, *J* = 11.4 Hz, 1 H), 3.49 (d, *J* = 11.4 Hz, 1 H), 3.01 - 2.93 (m, 2H), 1.05 - 0.75 (m, 4 H).

LC-MS $[M+H]^+$: 213.1.

**Example 16: N-((5'H,7'H-spiro[cyclopropane-1,4'-thieno[2,3-c]pyran]-7'-yl) methyl)ethylamine**

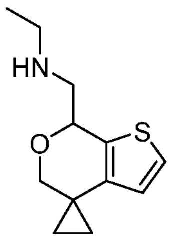

Synthesis route 1:

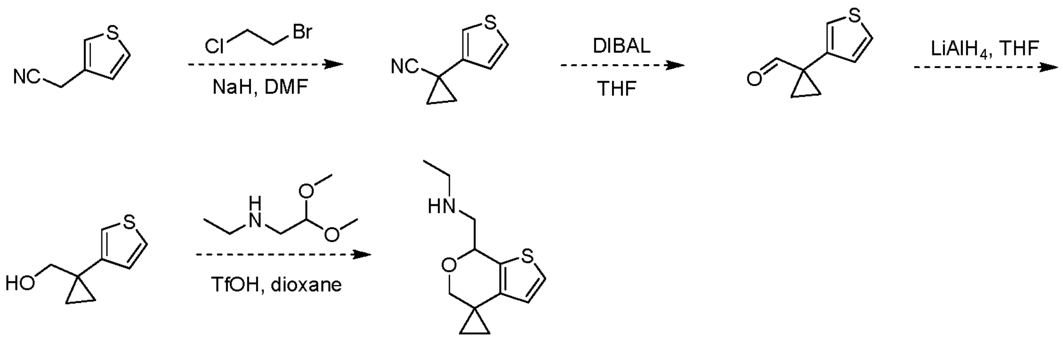


Synthesis route 2:

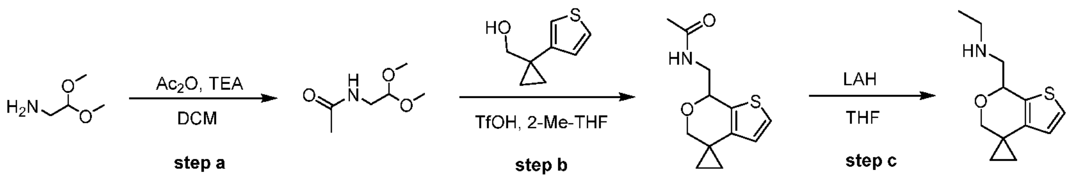


**Step a: synthesis of N-(2,2-dimethoxyethyl)acetamide**

Triethylamine (288.6mg, 2.85mmol) was added to a dichloromethane (2mL) solution of 2,2-dimethoxyethylamine(200.0mg, 1.90mmol) in an ice bath and under nitrogen protection, after stirring for 30 minutes, acetic anhydride (233.0mg, 2.28 mmol) was slowly added, stirring at room temperature for 2 hours; after the reaction was completed, saturated sodium bicarbonate aqueous solution (10mL) was added in ice bath, extracting with dichloromethane (10mL × 3); the extract liquor was washed with water (10mL × 3) and saturated salt solution (10mL × 3), drying with anhydrous sodium sulfate, filtering and concentrating, then the crude product was purified by column chromatography to obtain the target product (500mg of crude product), which was directly put into the next reaction without purification.

**Step b: synthesis of N-((5'H,7'H-spiro[cyclopropane-1,4'-thieno[2,3-c]pyra -n]-7'-yl)methyl)acetamide**

N-(2,2-dimethoxyethyl)acetamide (500mg of crude) and trifluoromethanesulfonic acid (389.6mg, 2.6mmol) were added to a 2-methyltetrahydrofuran (9 mL) solution of (1-(thiophen-3-yl)cyclopropyl)methanol (200mg, 1.3mmol), stirring at room temperature for 16 hours; after the reaction was completed, the pH of the reaction solution was adjusted to 13 with a 15% sodium hydroxide aqueous solution, diluting with 10 mL of water, then extracting with ethyl acetate (10mL × 3), and then the extract solution was purified by column chromatography to obtain the target product (250.0 mg, 81.0% yield).

LC-MS $[M+H]^+$: 238.1.

**Step c: synthesis of N-((5'H,7'H-spiro[cyclopropane-1,4'-thieno[2,3-c]pyra-n]-7'-yl)methyl)ethylamine**

Lithium aluminum hydride (2.5M in THF, 0.84mL, 2.1mmol) was slowly added to a tetrahydrofuran (2.5mL) solution of N-(5'H,7'H-spiro[cyclopropane-1,4'-thieno[2,3-c]pyran]-7'-yl)methyl)acetamide (250mg, 1.05mmol) in an ice bath and under nitrogen protection, reacting at 70°C for 2 hours; after the reaction was completed, water (10 mL) was added to quench the reaction in ice bath, extracting with ethyl acetate (10mL × 3); the extract phase was washed with water (10mL × 3) and saturated salt solution (10mL × 3), drying with anhydrous sodium sulfate, filtering and concentrating, then the crude product was separated and purified by preparative liquid chromatography (water/formic acid/acetonitrile) to obtain the target product (87.0 mg, 19.9% yield).

$^1$H NMR (400 MHz, $CD_3OD$): δ 8.49 (s, 1 H), 7.31 (dd, *J* = 5.2, 0.8 Hz, 1 H), 6.61 (d, *J* = 5.2 Hz, 1 H), 5.22 (dd, *J* = 9.2, 2.8 Hz, 1 H), 3.96 (dd, *J* = 11.6, 1.2 Hz, 1 H), 3.62 (d, *J* = 11.6 Hz, 1 H), 3.51-3.44 (m, 1 H), 3.30 - 3.23 (m, 1 H), 3.17 - 3.09 (m, 2 H), 1.32 (t, *J* = 7.6 Hz, 3 H), 1.10 - 1.02 (m, 1 H), 1.00 - 0.78 (m, 3 H).

LC-MS $[M+H]^+$: 224.1.

**Example 17: N-((5'H,7'H-spiro[cyclopropane-1,4'-thieno[2,3-c]pyran]-7'-yl) methyl)propane-2-amine**

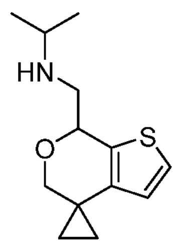

Synthesis route 1:

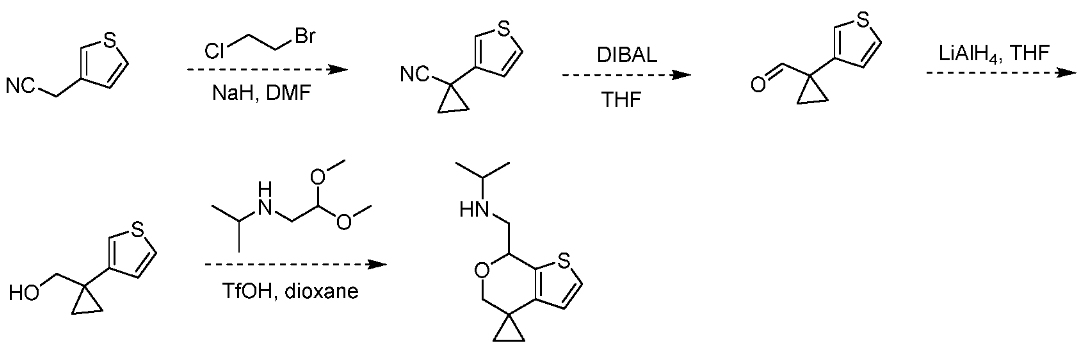


Synthesis route 2:

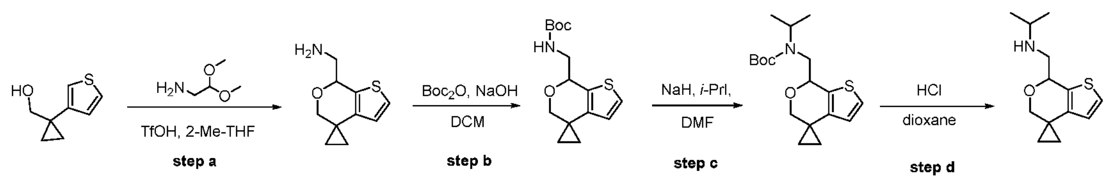


**Step a: synthesis of (5'H,7'H-spiro[cyclopropane-1,4'-thieno[2,3-c]pyran]-7'-yl)methanamine**

2,2-dimethoxy-ethylamine (1.5g, 14.26mmol) and trifluoromethanesulfonic acid (3.89g, 25.94mmol) were added to a 2-methyltetrahydrofuran (40mL) solution of (1-(thiophen-3-yl)cyclopropyl)methanol (2.0g, 12.97mmol), stirring at

room temperature for 16 hours; after that, the pH of the reaction solution was adjusted to 13 with a 15% sodium hydroxide aqueous solution, diluting with water (20mL), then the mixture was extracted with ethyl acetate (20mL × 3), drying the extract phase with anhydrous sodium sulfate, filtering and concentrating, and then the crude product was separated and purified by column chromatography to obtain the target product (500.0 mg, 19.7% yield).

LC-MS $[M+H]^+$: 196.1.

**Step b: synthesis of tert-butyl((5'H,7'H-spiro[cyclopropane-1,4'-thieno[2,3 -c]pyran]-7'-yl)methyl)carbamate**

Sodium hydroxide (512.04g, 12.80mmol) and di-tert-butyl dicarbonate (1.12g, 5.12mmol) were added to a dichloromethane (5mL) solution of (5'H,7'H-spiro[cyclopropane-l,4'-thieno[2,3-c]pyran]-7'-yl)methylamine (500.0mg, 2.56mmol), stirring at room temperature for 16 hours; after that, water (10mL) was added to the reaction solution for dilution, extracting with ethyl acetate (10 mL × 3); the extract phase was dried with anhydrous sodium sulfate, filtering and concentrating, then the crude product was separated and purified by column chromatography to obtain the target product (430 mg, 56.9% yield).

LC-MS $[M+H-56]^+$: 240.1.

**Step c: synthesis of tert-butyl((5'H,7'H-spiro[cyclopropane-1,4'-thieno[2,3-c]pyran]-7'-yl)methyl)(isopropyl)carbamate**

Sodium hydride (60%, 67.8mg, 1.69mmol) and 2-iodopropane (1.12g, 1.69mmol) were added to a N,N-dimethylformamide (2.5mL) solution of tert-butyl((5'H, 7'H-spiro[cyclopropane-1,4'-thieno[2,3-c]pyran]-7'-yl)methyl)carbamate (250.0mg, 0.85mmol), stirring at room temperature for 3 days; after that, water (10mL) was added to quench the reaction, extracting with ethyl acetate (10mL × 3); after the extracted phase was concentrated, the crude product obtained was purified by column chromatography to obtain the target product (120.0 mg, 41.8% yield).

LC-MS $[M+H-100]^+$: 238.1.

**Step d: synthesis of N-((5'H,7'H-spiro[cyclopropane-1,4'-thieno[2,3-c]pyra n]-7'-yl)methyl)propane-2-amine**

Dioxane hydrochloride (4M, 1mL) was added to the dioxane (2mL) solution of tert-butyl((5',7'-dihydrospiro[cyclopropane-1,4'-thieno[2,3-c]pyran]-7'-yl)methyl)(isopropyl)carbamate (120.0mg, 0.36mmol), concentrating directly after stirring at room temperature for 2 hours, and the crude product obtained was separated and purified by the preparative liquid chromatography to obtain the target product (6.1mg, 7.2% yield).

$^1H$ NMR (400 MHz, $CD_3OD$): δ 7.25 (d, *J* = 5.2 Hz, 1 H), 6.59 (d, *J* = 5.2 Hz, 1 H), 5.02 (dd, *J* = 9.2, 3.2 Hz, 1 H), 3.96 (d, *J* = 11.6 Hz, 1 H), 3.58 (d, *J* = 11.6 Hz, 1 H), 3.06 (dd, *J* = 12.4, 3.2 Hz, 1 H), 3.01 - 2.90 (m, 2 H), 1.16 (d, *J* = 6.4 Hz, 6 H), 1.09 - 0.77 (m, 4 H).

LC-MS $[M+H]^+$: 238.2.

**Example 18: N-((5'H,7'H-spiro[cyclopropane-1,4'-thieno[2,3-c]pyran]-7'-yl) methyl)-2,2,2-trifluoroethane-1-amine**

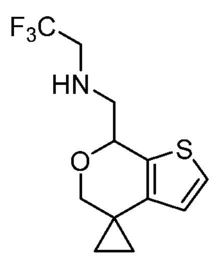

**Synthesis route 1:**

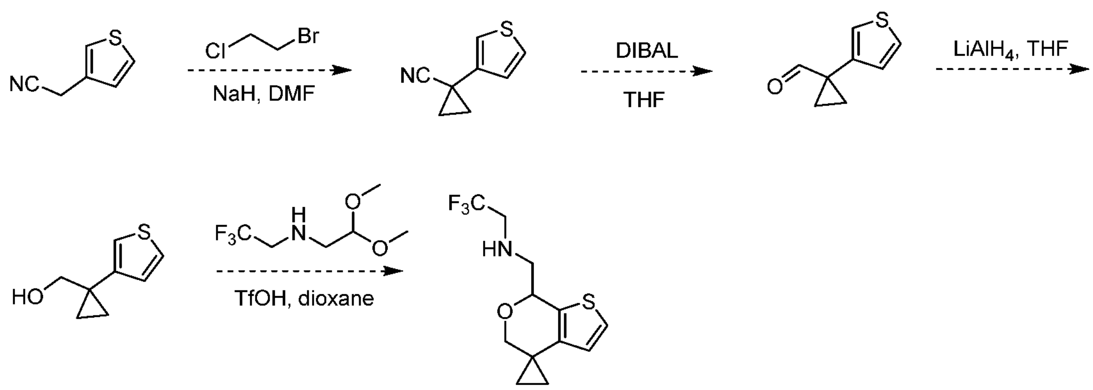


**Synthesis route 2:**

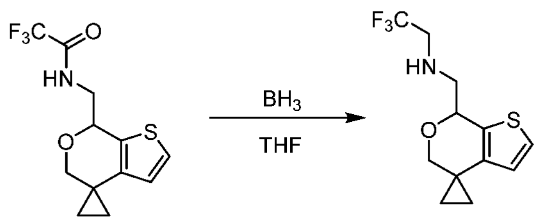


Tetrahydrofuran solutionofborane (1 M in THF, 3.09mL, 3.09mmol)was added to a tetrahydrofuran (10mL) solution of N-((5'H,7'H-spiro[cyclopropane-1,4'-thieno[2,3-c]pyran]-7'-yl)methyl)-2,2,2-trifluoro acetamide (300.0mg, 1.03mmol) in an ice bath and under nitrogen protection, stirring at 70°C for 2 hours after adding; after the reaction was completed, methanol (10mL) and hydrochloric acid (2M, 10mL) were added in ice bath to quench the reaction; the mixture was extracted with ethyl acetate (10mL $\times$ 3), and the extract phase was washed with water (10mL $\times$ 3) and saturated salt solution (10mL $\times$ 3), drying with anhydrous sodium sulfate, filtering and concentrating, then the crude product obtained was separated and purified by the preparative liquid chromatography to obtain the target product (76.6 mg, 26.8% yield).

$^1$H NMR (400 MHz, $CD_3OD$): $\delta$ 7.23 (d, *J* = 5.2 Hz, 1 H), 6.58 (d, *J* = 5.2 Hz, 1 H), 4.97 (t, *J* = 5.6 Hz, 1 H), 3.96 (d, *J* = 11.2 Hz, 1 H), 3.56 (d, *J* = 11.6 Hz, 1 H), 3.44 - 3.26 (m, 2 H), 3.12 - 3.02 (m, 2 H), 1.10 - 0.77 (m, 4 H).

LC-MS $[M+H]^+$: 278.1

**Example 19: N-methyl-1-(5'H,7'H-spiro[cyclopropane-1,4'-thieno[2,3-c]pyr-an]-7'-yl)ethane-1-amine**

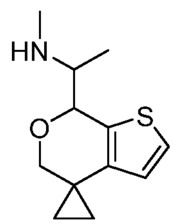

Synthesis route 1:

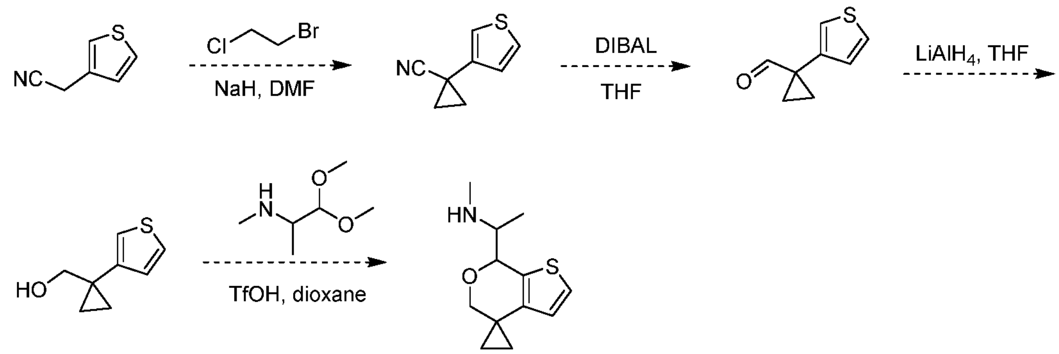


Synthesis route 2:

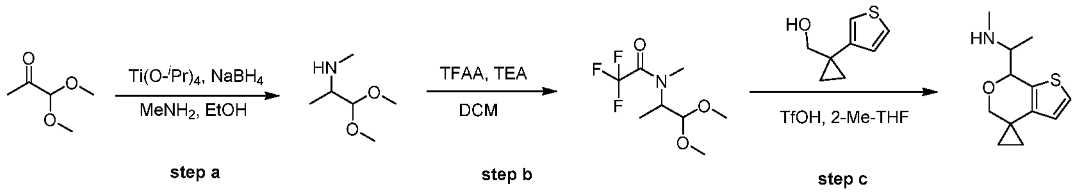


**Step a: synthesis of 1,1-dimethoxy-N-methylpropane-2-amine**

Titanium tetra-isopropoxide (9.62g, 33.86mmol), methylamine (526.0mg, 33.86mmol), and triethylamine (3.42g, 33.86mmol) were added to an ethanol (20mL) solution of 1,1-dimethoxypropane-2-one (2.0g, 16.93mmol) under nitrogen protection at 10°C, stirring at room temperature for 16 hours; after that, sodium borohydride (1.28g, 33.86mmol) was added, stirring at room temperature for 6 hours; after the reaction was completed, ammonia water (2M, 60mL) was added to quench the reaction in ice bath; the reaction solution was filtered, and the filtrate was extracted with dichloromethane (30 mL × 3), and the extract phase was washed with water (15 mL × 3) and saturated salt solution (15 mL × 3), drying with anhydrous sodium sulfate, filtrating and concentrating to obtain the target crude product (1.8 g).

**Step b: synthesis of N-(1,1-dimethoxypropane-2-yl)-2,2,2-trifluoro-N-methylacetamide**

Triethylamine (341.7mg, 3.37mmol) was added to a dichloromethane (10mL) solution of 1,1-dimethoxy-N-methylpropane-2-amine (300.0mg, 2.25mmol) in an ice bath and under nitrogen protection, stirring for 30 minutes, then trifluoroacetic anhydride (568.4mg, 2.7mmol) was added, and stirring was continued for 2 hours at room temperature; after the reaction was completed, saturated sodium bicarbonate aqueous solution (10mL) was added to quench the reaction in ice bath, then extracting with dichloromethane (10mL × 3); the extract phase was washed with water (10 mL × 3) and saturated salt solution (10 mL × 3), drying with anhydrous sodium sulfate, filtering and concentrating to obtain the target crude product (600mg).

**Step c: synthesis of N-methyl-1-(5'H,7'H-spiro[cyclopropane-1,4'-thieno[2, 3-c]pyran]-7'-yl)ethane-1-amine**

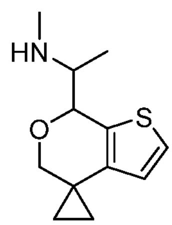

N-(1,1-dimethoxypropane-2-yl)-2,2,2-trifluoro-N-methylacetamide (600 mg of crude) and trifluoromethanesulfonic acid (584.4mg, 3.9mmol) were added to a2-methyltetrahydrofuran (9mL) solution of (1-(thiophen-3-yl)cyclopropyl)methanol (200.0 mg, 1.3 mmol), stirring at room temperature for 16 hours; after the reaction was completed, the pH of the reaction solution was adjusted to 13 with 15% sodium hydroxide aqueous solution, then diluting with water (10 mL), extracting with ethyl acetate (10 mL × 3); after the extract phase was concentrated, the crude product was separated and purified by preparative liquid chromatography (water/formic acid/acetonitrile) to obtain the target product (5.3mg, 1.5% yield).

$^1$H NMR (400 MHz, $CD_3OD$): δ 8.56 (s, 1 H), 7.38 (d, *J* = 5.2, 0.8 Hz, 1 H), 6.67 (d, *J* = 5.2 Hz, 1 H), 5.05 (d, *J* = 3.6 Hz, 1 H), 4.05 (d, *J* = 11.2, 1.6 Hz, 1 H), 3.60 (d, *J* = 11.6 Hz, 1 H), 3.57 - 3.48 (m, 1 H), 2.62 (s, 3 H), 1.56 (d, *J* = 6.4 Hz, 3 H), 1.18-1.08 (m, 1 H), 1.07 - 0.97 (m, 1 H), 0.96 - 0.82 (m, 2 H).

LC-MS $[M+H]^+$: 224.1.

**Example 20: N-Methyl-2-(5'H,7'H-spiro[cyclopropane-1,4'-thieno[2,3-c]pyr an]-7'-yl)propane-2-amine**

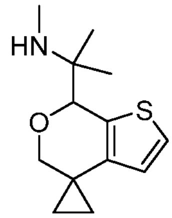

Synthesis route 1:

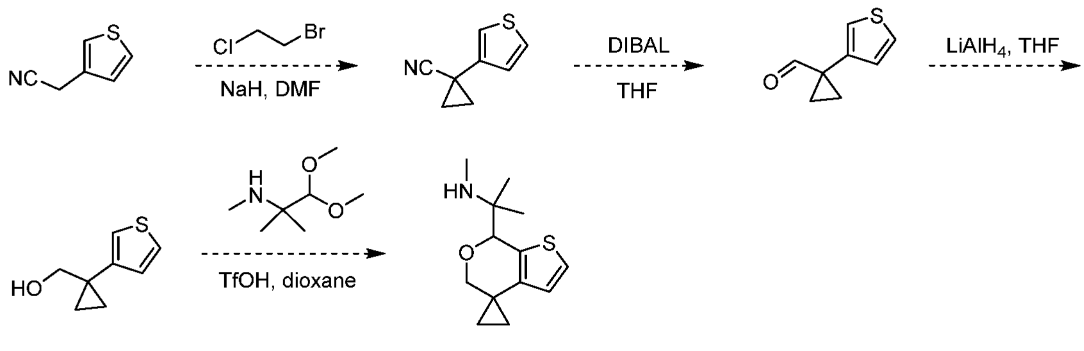


Synthesis route 2:

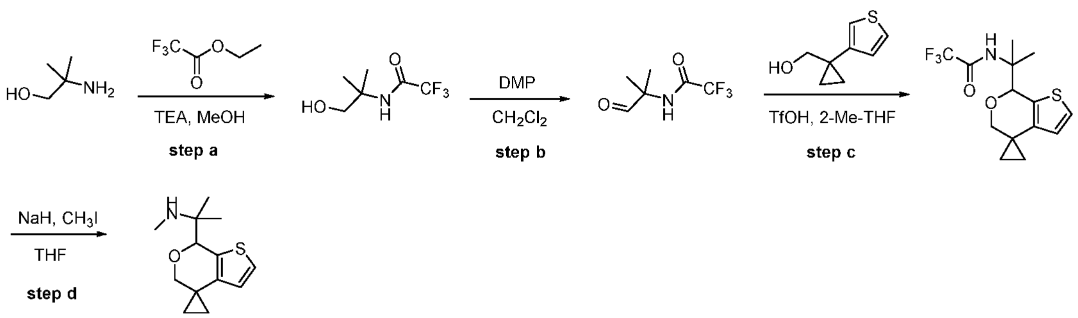

**Step a: synthesis of 2,2,2-trifluoro-N-(1-hydroxy-2-methylpropane-2-yl)acetamide**

Ethyl trifluoroacetate (8.5g, 60.0mmol) and triethylamine (7.6g, 75.0 mmol) were added to a methanol (100 mL) solution of 2-amino-2-methylpropane-1-ol (4.4g, 49.4 mmol) in an ice bath and under nitrogen protection, and the reaction solution was stirred overnight; after that, the reaction solution was directly concentrated to obtain the crude product, which was separated and purified by column chromatography (dichloromethane: methanol=20:1) to obtain the target product (4.2 g, 45.9% yield).

$^1$H NMR (400 MHz, $CDCl_3$): δ 6.47 (brs, 1 H), 3.63 (s, 2 H), 1.39 (s, 6 H).

**Step b: synthesis of 2,2,2-trifluoro-N-(2-methyl-1-oxypropane -2-yl)acetamide**

Deiss-Martin reagent (14.4g, 34.0mmol) was added to a dichloromethane (140mL) solution of 2,2,2-trifluoro-N-(1-hydroxy-2-methylpropane-2-yl)acetamide (4.2g, 22.8 mmol) in an ice bath and under nitrogen protection and heat-preservation stirring was carried out for 12 hours; after that, a saturated sodium thiosulfate aqueous solution (100 mL) was added, extracting with dichloromethane (100 mL); the extract phase was washed with saturated sodium bicarbonate aqueous solution (100 mL × 2), water (100 mL), and saturated salt solution (100 mL), drying with anhydrous sodium sulfate, then filtering and concentrating to obtain the target crude product (3.5 g, 91.0% yield).

**Step c: synthesis of N-(2-(5'H,7'H-spiro[cyclopropane-1,4'-thieno[2,3-c]pyran]-7'-yl)propane-2-yl)-2,2,2-trifluoroacetamide**

A 2-methyltetrahydrofuran (2 mL) solution of trifluoromethanesulfonic acid (2.4g, 16.0mmol) was slowly added to a 2-methyltetrahydrofuran (10 mL) solution of (1-(thiophen-3-yl)cyclopropyl)methanol (600.0mg, 3.89mmol) and 2,2,2-trifluoro-N-(2-methyl-1-oxypropane-2-yl)acetamide (1.4g, 8.0mmol), stirring for 6 hours in ice bath; after the reaction was completed, the pH of the reaction solution was adjust to 13 with 15% sodium hydroxide aqueous solution, then diluting with water (100 mL), extracting with ethyl acetate (100 mL × 2); the extract phase was concentrated to obtain the crude product, which was then separated and purified by column chromatography (petroleum ether: ethyl acetate=20:1-10:1) to obtain the target product (250.0 mg, 20.1% yield).

$^1$H NMR (400 MHz, $CDCl_3$): δ 7.15 (d, *J* = 5.2 Hz, 1 H), 6.67 (brs, 1 H), 6.52 (d, *J* = 5.6 Hz, 1 H), 5.15 (s, 1 H), 4.04 (dd, *J* = 11.2, 1.2 Hz, 1 H), 3.39 (d, *J* = 11.2 Hz, 1 H), 1.54 (d, *J* = 4.4 Hz, 6 H), 1.11-1.03 (m, 1 H), 1.02 - 0.92 (m, 1 H), 0.90 - 0.73 (m, 2 H).

**Step d: synthesis of N-methyl-2-(5'H,7'H-spiro[cyclopropane-1,4'-thieno[2, 3-c]pyran]-7'-yl)propane-2-amine**

Sodium hydride (60%, 123.0mg, 3.1mmol) was added to a tetrahydrofuran (6mL) solution of N-(2-(5'H,7'H-spiro[cyclopropane-1,4'-thieno[2,3-c]pyran]-7'-yl)propane-2-yl)-2,2,2-trifluoroacetamide (245.0mg, 0.77mmol) in an ice bath, stirring for 30 minutes, then adding iodomethane (219.0mg, 1.54mmol), stirring at room temperature for 2 hours; then heating up to 55°C and continued stirring for 3 hours; after the reaction was completed, ethyl acetate (100mL) was added for dilution, washing with saturated ammonium chloride aqueous solution (50mL), water (50mL), and saturated salt solution (50 mL); the organic phase was separated, drying with anhydrous sodium sulfate, filtering and concentrating to obtain the crude product, which was then separated and purified by column chromatography (dichloromethane: methanol=20:1) and preparative liquid chromatography column to obtain the target product (14.6 mg, 6.7% yield).

$^1$H NMR (400 MHz, $CD_3OD$): δ 8.53 (s, 1 H), 7.33 (d, *J* = 5.2 Hz, 1 H), 6.66 (d, *J* = 5.2 Hz, 1 H), 5.10 (s, 1 H), 4.07 (d, *J* = 11.2 Hz, 1 H), 3.49 (d, *J* = 11.6 Hz, 1 H), 2.66 (s, 3 H), 1.62 (s, 3 H), 1.23 (s, 3 H), 1.21-1.10 (m, 1 H), 1.05-0.95 (m, 1 H), 0.88-0.78 (m, 2 H).

LC-MS $[M+H]^+$: 238.1.

**Example 21: N-methyl-1-(5'H,7'H-spiro[cyclopropane-1,4'-thieno[2,3-c]pyr-an]-7'-yl)cyclopropane-1-amine**

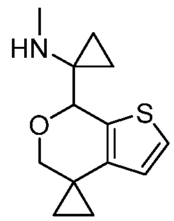

Synthesis route 1:

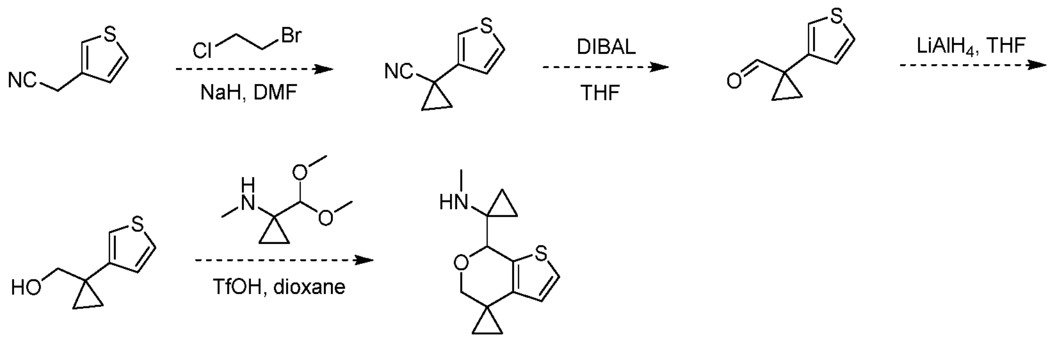


Synthesis route 2:

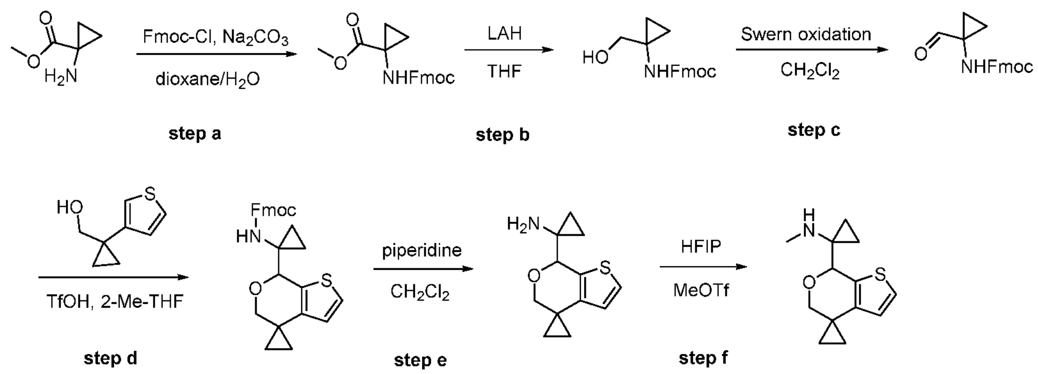


**Step a: synthesis of 1-(((9H-fluoren-9-yl)methoxy)carbonyl)amino)methyl cyclopropane carboxylate**

Sodium carbonate (4.77g, 45.0mmol), fluorenyl methoxycarbonyl chloride (11.6g, 45.0mmol), and triethylamine (7.6g, 75.0mmol) were added to a mixed solution of indioxane and water (50 mL: 50 mL) of 2-amino-2-methylpropane-1-ol (3.45g, 30.0mmol)in an ice bath, and the reaction solution was slowly heated to room temperature and stirred overnight; after that, ethyl acetate (300mL) was added for dilution, washing with water (300 mL × 3) and saturated salt solution (50mL); the organic phase was separated to obtain the crude product, then stirring and washing with a mixed solvent (petroleum ether: ethyl acetate: methanol=10:2:1) to obtain the target product (9.4 g, 92.9% yield).

**Step b: synthesis of (9H-fluoren-9-yl)methyl(1-(hydroxymethyl)cyclopropyl)carbamate**

Lithium aluminium hydride (2.5M in THF, 14.2mL, 35.6mmol) was added to a tetrahydrofuran (100 mL) solution of 1-(((9H-fluorene-9-yl)methoxy)carbonyl)amino)methyl cyclopropane carboxylate (8.0g, 23.7mmol) in an ice bath and under nitrogen protection, and heat-preservation stirring was carried out for 3 hours; after the reaction was completed, water (1 mL), 10% sodium hydroxide aqueous solution (4 mL), and ethyl acetate (100 mL) were successively added to the reaction solution, stirring at room temperature for 1 hour; the organic phase was separated, then drying with anhydrous

sodium sulfate, filtering and concentrating, and then the crude product obtained was purified by column chromatography (petroleum ether: ethyl acetate=4:1) to obtain the target product (3.0 g, 40.9% yield).

**Step c: synthesis of (9H-fluoren-9-yl)methyl(1-formylcyclopropyl)carbamate**

A dichloromethane (5mL) solution of oxalyl chloride (1.9g, 15mmol) was slowly added dropwise to a dichloromethane (50mL) solution of dimethyl sulfoxide (1.17g, 15mmol) at -70°C under nitrogen protection, and heat-preservation stirring was carried out for 30 minutes; then (9H-fluoren-9-yl)methyl(1-(hydroxymethyl)cyclopropyl)carbamate (3.0g, 9.7mmol) was dissolved in the mixed solvent of tetrahydrofuran and dichloromethane (20mL: 20mL), which was slowly dropped into the above reaction solution for heat-preservation reaction for 1 hour, then triethylamine (7mL, 50mmol) was added and the reaction solution was allowed to slowly heat to room temperature; after that, water (100mL) was added to quench the reaction, extracting with dichloromethane (100 mL); the organic phase was separated, drying with anhydrous sodium sulfate, filtrating and concentrating, then the crude product obtained was purified by column chromatography to obtain the target product (900.0 mg, 30.2% yield).

**Step d: synthesis of (9H-fluoren-9-yl)methyl(1-(5'H,7'H-spiro[cyclopropane-1,4'-thieno[2,3-c]pyran]-7'-yl)cyclopropyl)carbamate**

A 2-methyltetrahydrofuran (2mL) solution of trifluoromethanesulfonic acid (978.0mg, 6.52mmol) was added to a 2-methyltetrahydrofuran (10mL) solution of (1-(thiophene-3-yl)cyclopropyl)methanol (251.0mg, 1.63mmol) and (9H-fluoren-9-yl)methyl(1-formylcyclopropyl)carbamate (500.0mg, 1.63mmol), stirring for 30 minutes in an ice bath; after the reaction was completed, the pH of the reaction solution was adjusted to 13 with 15% sodium hydroxide aqueous solution, diluting with water (100mL) and extracting with ethyl acetate (100mL × 2); the extract phase was concentrated to obtain the crude product, which was then purified by column chromatography to obtain the target product (170.0 mg, 23.5% yield).

LC-MS $[M+H]^+$: 444.2.

**Step e: synthesis of 1-(5'H,7'H-spiro[cyclopropane-1,4'-thieno[2,3-c]pyran] -7'-yl)cyclopropaneamine**

In an ice bath and under nitrogen protection, piperidine (161.0mg, 1.9mmol) was added to a dichloromethane (3mL) solution of (9H-fluoren-9-yl)methyl(1-((5'H,7'H-spiro[cyclopropane-1,4'-thieno[2,3-c]pyran]-7'-y l)cyclopropyl)carbamate (170.0mg, 0.38mmol), stirring at room temperature for 5 hours; after that, the reaction solution was poured into water (50mL), extracting with dichloromethane (50mL); the extract phase was washed with saturated sodium bicarbonate aqueous solution (100mL × 2), water (100mL), and saturated salt solution (100mL), drying with anhydrous sodium sulfate, filtering and concentrating, then the crude product was purified by column chromatography to obtain the target product (80 mg, 95.1% yield).

LC-MS $[M+H]^+$: 222.1.

**Step f: synthesis of N-methyl-1-(5'H,7'H-spiro[cyclopropane-1,4'-thieno[2, 3-c]pyran]-7'-yl)cyclopropane-1-amine**

Methyl trifluoromethanesulfonate (50.0mg, 0.3mmol) was added to a hexafluoroisopropanol (6mL) solution of 1-(5',7'-dihydrospiro[cyclopropane-1,4'-thieno[2,3-c]pyran]-7'-yl)cyclopropylamine (44.0mg, 0.2mmol) in an ice bath, stirring at room temperature for 3 hours; after the reaction was completed, ethyl acetate (100 mL) was added for dilution, washing successively with saturated sodium bicarbonate aqueous solution (10mL), water (50mL), and saturated salt solution (50mL); the organic phase was separated, drying with anhydrous sodium sulfate, filtering and concentrating, then the crude product was purified by column chromatography and preparative liquid chromatography (water/formic acid/acetonitrile) to obtain the target product (8.4mg, 15.0% yield).

$^1$H NMR (400 MHz, $CD_3OD$): δ 8.46 (s, 1 H), 7.36 (d, *J* = 5.2 Hz, 1 H), 6.67 (d, *J* = 5.2 Hz, 1 H), 4.72 (s, 1 H), 4.02 (dd, *J* = 11.6, 1.6 Hz, 1 H), 3.60 (d, *J* = 11.6 Hz, 1 H), 2.59 (s, 3 H), 1.32 - 0.80 (m, 8 H).

LC-MS $[M+H]^+$: 236.1.

**Example 22: 2-(5'H,7'H-spiro[cyclopropane-1,4'-thieno[2,3-c]pyran]-7'-yl)-pyrrolidine**

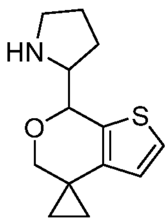

Synthesis route 1:

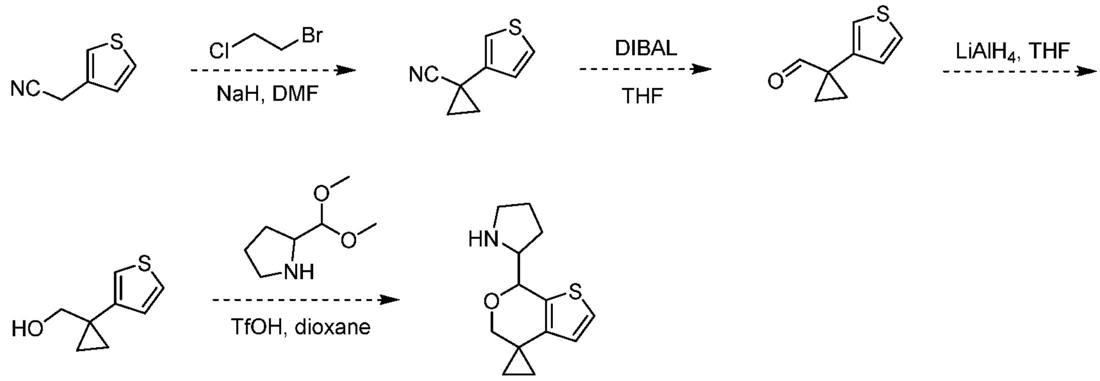


Synthesis route 2:

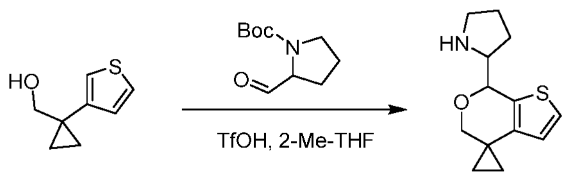


Tert-butyl 2-formylpyrrolidine-1-carboxylate (1.42g, 7.13mmol) and trifluoromethanesulfonic acid (1.94g, 12.96mmol) were added to a 2-methyltetrahydrofuran (40mL) solution of (1-(thiophen-3-yl)cyclopropyl)methanol (1.0g, 6.48mmol), stirring at room temperature for 20 minutes; after the reaction was completed, the pH of the reaction solution was adjusted to 13 with 15% sodium hydroxide aqueous solution, then diluting with water (10mL), extracting with ethyl acetate (10mL $\times$ 3); the extract phase was concentrated to obtain the crude product, which was then separated and purified with column chromatography and supercritical fluid liquid phase (diethylamine/ethanol) to obtain a pair of diastereoisomers, wherein diastereoisomer 1 (20.1 mg, 1.32% yield).

$^1H$ NMR (400 MHz, $CD_3OD$): $\delta$ 7.27 (d, $J$ = 5.2 Hz, 1 H), 6.59 (d, $J$ = 5.2 Hz, 1 H), 4.88 (d, $J$ = 9.6 Hz, 1 H), 3.95 (d, $J$ = 11.6 Hz, 1 H), 3.67 - 3.52 (m, 2 H), 3.20-3.06 (m, 1 H), 3.05 - 2.92 (m, 1 H), 2.30 - 2.14 (m, 1 H), 2.07 - 1.84 (m, 3 H), 1.13-0.75 (m, 4 H).

LC-MS $[M+H]^+$: 236.1.

Diastereoisomer 2 (37.5 mg, 2.46% yield).

$^1H$ NMR (400 MHz, $CD_3OD$): $\delta$ 7.29 (d, $J$ = 5.2 Hz, 1 H), 6.60 (d, $J$ = 5.2 Hz, 1 H), 4.94 (d, $J$ = 6.0 Hz, 1 H), 3.97 (dd, $J$ = 11.6, 1.6 Hz, 1 H), 3.74 - 3.65 (m, 1 H), 3.61 (d, $J$ = 11.6 Hz, 1 H), 3.24 - 3.14 (m, 1 H), 3.13 - 3.03 (m, 1 H), 2.36 - 2.19 (m, 1 H), 2.10 - 1.85 (m, 3 H), 1.11 - 0.76 (m, 4 H).

LC-MS $[M+H]^+$: 236.1.

**Example 23: 1-((5'H,7'H-spiro[cyclopropane-1,4'-thieno[2,3-c]pyran]-7'-yl) methyl)azetidine**

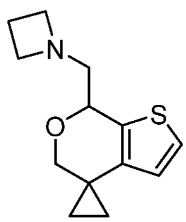

Synthesis route 1:

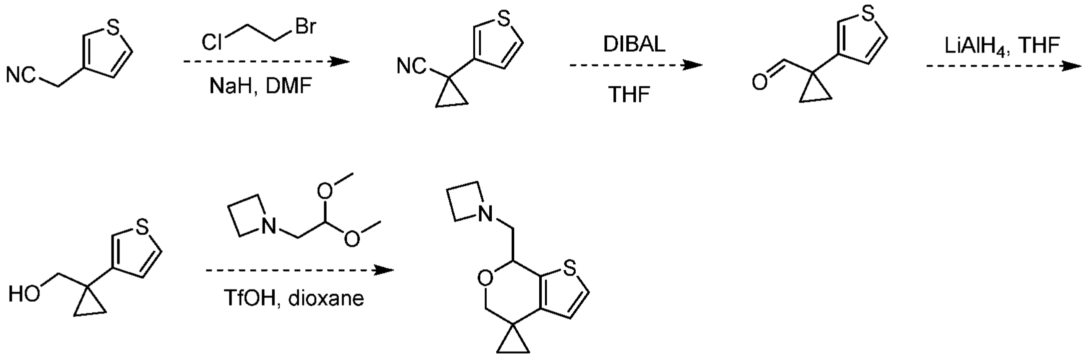


Synthesis route 2:

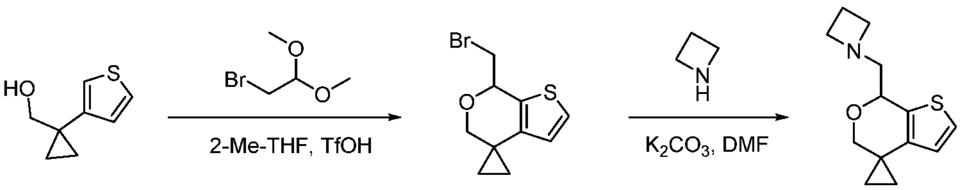


step a

step b

**Step a: synthesis of 7'-(bromomethyl)-5'H,7'H-spiro[cyclopropane-1,4'-thieno[2,3-c]pyran**

Trifluoromethanesulfonic acid (10.0g, 66.6mmol) was added to a 2-methyltetrahydrofuran (25mL) solution of (1-(thiophen-3-yl)cyclopropyl)methanol (2.21 g, 14.3 mmol) and 2-bromo-1,1-dimethoxyethane (2.0g, 11.9mmol) in an ice bath, stirring at room temperature for 5 hours; after the reaction was completed, the pH of the reaction solution was adjusted to 13 with 15% sodium hydroxide aqueous solution, diluting with water (100mL) and extracting with ethyl acetate (100 mL × 3); the extract phase was concentrated to obtain the crude product, which was then purified by column chromatography to obtain the target product (2.6 g, 70.2% yield).

$^1$H NMR (400 MHz, $CDCl_3$): δ 7.18 (d, *J* = 5.2 Hz, 1 H), 6.51 (d, *J* = 5.2 Hz, 1 H), 5.13 (dd, *J* = 8.0, 4.0 Hz, 1 H), 3.96 (d, *J* = 11.6 Hz, 1 H), 3.77 - 3.70 (m, 1 H), 3.67-3.57 (m, 2 H), 1.06 - 0.75 (m, 4 H).

LC-MS $[M+H]^+$: 258.9.

**Step b: synthesis of 1-((5'H,7'H-spiro[cyclopropane-1,4'-thieno[2,3-c]pyran]-7'-yl)methyl)azetidine**

Potassium carbonate (4.2 g, 30.2 mmol) was added to a N,N-dimethylformamide (20mL) solution of 7'-(bromomethyl)-5'H,7'H-spiro[cyclopropane-1,4'-thieno[2,3-c]pyran (2.6g, 10.1 mmol) and azetidine (0.69g, 12.1mmol), stirring at 120°C for 4 hours; after the reaction was completed, cooling to room temperature, diluting with water (200 mL), extracting with ethyl acetate (200mL × 3); the extract solution was washed successively with water (200mL × 3) and saturated salt solution (200mL × 3), drying with anhydrous sodium sulfate, filtering and concentrating, then the crude product was purified by column chromatography and preparative liquid chromatography to obtain the target product

(41.8 mg, 1.8% yield).

$^1$H NMR (400 MHz, $CD_3OD$): δ 7.17 (d, *J* = 5.2 Hz, 1 H), 6.52 (d, *J* = 5.2 Hz, 1 H), 4.86 - 4.78 (m, 1 H), 3.84 (dd, *J* = 11.6, 1.2 Hz, 1 H), 3.51 (d, *J* = 11.6 Hz, 1 H), 3.40 - 3.30 (m, 4 H), 2.86 - 2.74 (m, 2 H), 2.17 - 2.05 (m, 2 H), 1.04 - 0.72 (m, 4 H).

LC-MS $[M+H]^+$: 236.1.

**Example 24: 5'H-dispiro[cyclopropane-1,4'-thieno[2,3-c]pyran-7',3 "-pyrrolidine]**

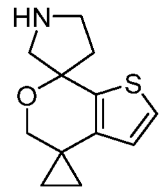

Synthesis route 1:

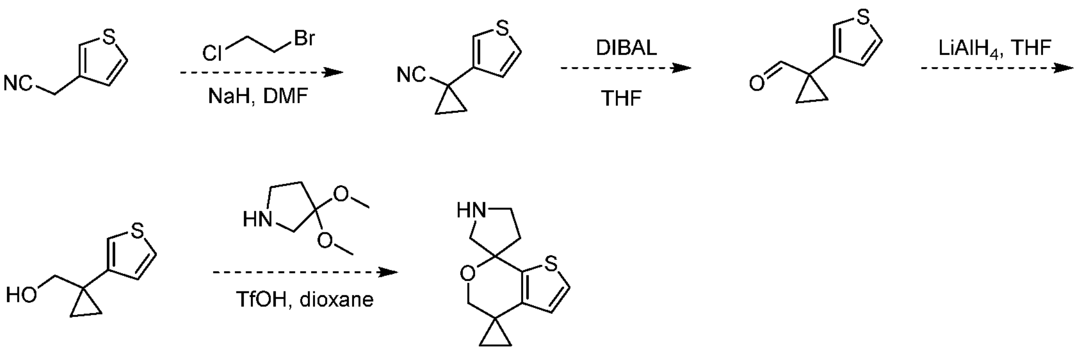


Synthesis route 2:

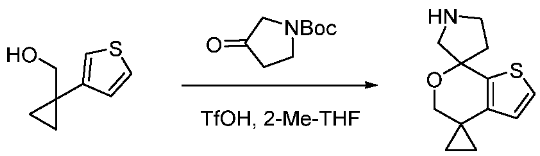


Tert-butyl 3-oxopyrrolidine-1-carboxylate (540.4mg, 1.95mmol) and trifluoromethanesulfonic acid (438.3mg, 1.95mmol) were added to a 2-methyltetrahydrofuran (12mL) solution of (1-(thiophen-3-yl)cyclopropyl)methanol (300.0mg, 1.95mmol), stirring at room temperature for 4 hours; after the reaction was completed, the pH of the reaction solution was adjusted to 13 with 15% sodium hydroxide aqueous solution, then diluting with water (10mL); the mixture was extracted with ethyl acetate (10mL × 3), and the extract phase was concentrated to obtain the crude product, which was then separated and purified by the preparative liquid chromatography (water/formic acid/acetonitrile) to get the target product (6.2mg, 1.2% yield).

$^1$H NMR (400 MHz, $CD_3OD$): δ 8.54 (s, 1 H), 7.36 (d, *J* = 5.2 Hz, 1 H), 6.60 (d, *J* = 5.2 Hz, 1 H), 3.82 (d, *J* = 12.0 Hz, 1 H), 3.76 - 3.65 (m, 2 H), 3.64 - 3.47 (m, 2 H), 3.37 (d, *J* = 12.4 Hz, 1 H), 2.64 - 2.48 (m, 1 H), 2.41 - 2.27 (m, 1 H), 1.06 - 0.86 (m, 4 H).

LC-MS $[M+H]^+$: 222.1.

**Example 25: N-methyl-5'H-dispiro[cyclobutane-1,7'-thieno[2,3-c]pyran-4',1" -cyclopropane]-2-amine**

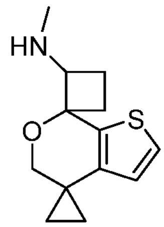

Synthesis route:

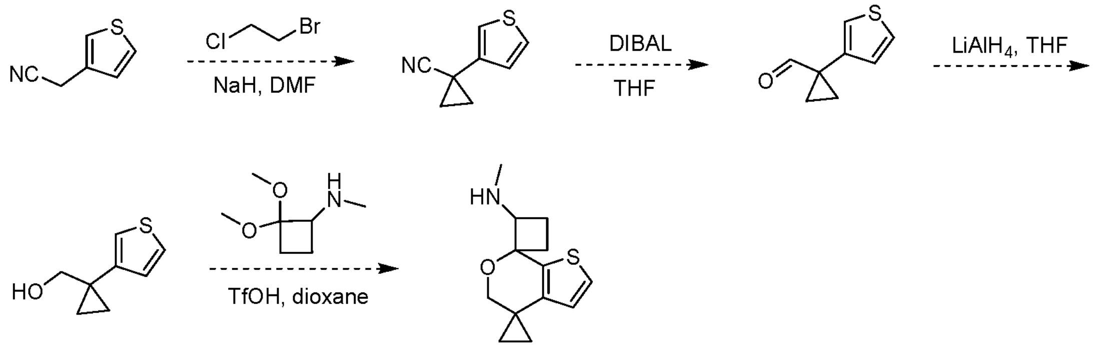


LC-MS $[M+H]^+$: 236.1.

**Example 26: N-methyl-1-(4'H,6'H-spiro[cyclopropane-1,7'-pyrano[4,3-d]-th iazole]-4'-yl)methanamine**

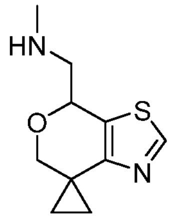

Synthesis route:

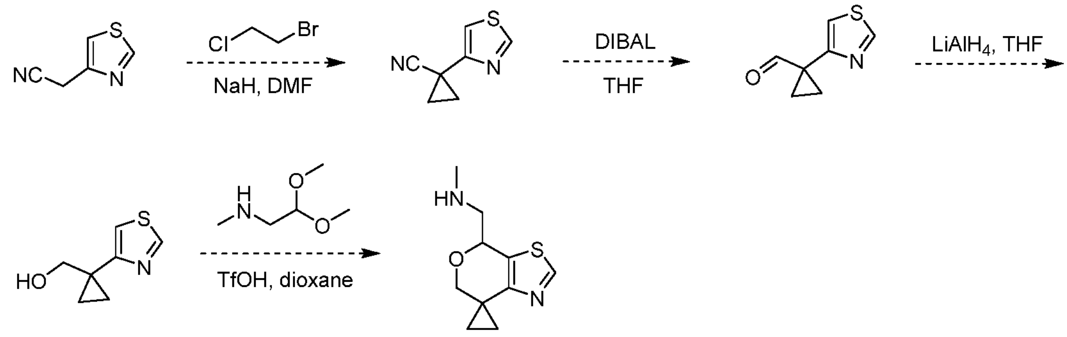


LC-MS $[M+H]^+$: 211.1.

**Example 27: 1-(5',6'-dihydro-8'H-spiro[cyclopropane-1,4'-thieno[2,3-c]oxepine]-8'-yl)-N-methylmethylamine**

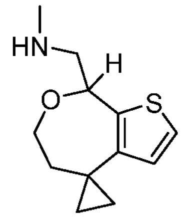

Synthesis scheme:

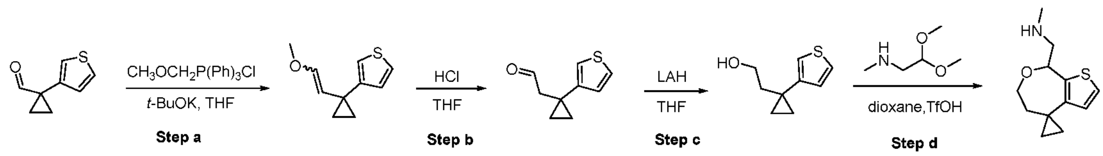


**Step a: synthesis of 3-(1-(2-methoxyvinyl)cyclopropyl)thiophene**

(Methoxymethyl) triphenylphosphine chloride (9.01g, 26.28mmol) was dissolved in tetrahydrofuran (80mL), then a tetrahydrofuran (20mL) solution of potassium tert-butanol (2.95g, 26.28mmol) was slowly added dropwise in an ice bath, reacting for half an hour, then a THF (20mL) solution of 1-(thiophen-3-yl)cyclopropane formaldehyde (2 g, 13.14 mmol) was added dropwise. After one hour of reaction, water (250mL) was added to quench the reaction, extracting with ethyl acetate (200mL × 2), combining the extract phase, drying with anhydrous sodium sulfate and filtering, then the solvent was spin-dried to obtain the crude product, which was separated and purified by column chromatography (petroleum ether: ethyl acetate=10:1) to obtain the target compound, which was a cis-trans mixture (1.8 g, 76.0% yield).

Cis product: $^1H$ NMR ($CDCl_3$, 600 MHz): δ 7.20 - 7.17 (m, 1 H), 6.94 - 6.92 (m, 1 H), 6.85 - 6.82 (m, 1 H), 5.92 (d, *J* = 6.0 Hz, 1 H), 4.69 (d, *J* = 6.6 Hz, 1 H), 3.59 (s, 3 H), 1.15 - 1.11 (m, 2 H), 1.04 - 1.01 (m, 2 H).

Trans product: $^1H$ NMR ($CDCl_3$, 600 MHz): δ 7.24 - 7.21 (m, 1 H), 6.97 - 6.95 (m, 1 H), 6.90 - 6.87 (m, 1 H), 6.29 (d, *J* = 12.6 Hz, 1 H), 5.07 (d, *J* = 12.6 Hz, 1 H), 3.52 (s, 3 H), 1.00 - 0.92 (m, 4H).

**Step b: synthesis of 2-(1-(thiophen-3-yl)cyclopropyl)acetaldehyde**

3-(1-(2-methoxyvinyl)cyclopropyl)thiophene (1.8g, 9.99mmol) was dissolved in tetrahydrofuran (20mL), then hydrochloric acid solution (20mL, 2M) was added, reacting at room temperature for 15 hours; then the reaction solution was poured into water (60 mL), extracting twice with ethyl acetate (100mL × 2), combining the organic phase and washing with saturated sodium bicarbonate aqueous solution (50 mL), then drying with anhydrous sodium sulfate, filtering and concentrating to obtain the target crude compound (1.6 g, 96.3% yield), which was directly used for the next reaction without purification.

**Step c: synthesis of 2-(1-(thiophen-3-yl)cyclopropyl)ethanol**

2-(1-(thiophen-3-yl)cyclopropyl)acetaldehyde (1.6g, 9.62mmol) was dissolved in tetrahydrofuran (30mL), and lithium aluminum hydride (0.37g, 9.62mmol) was added in batches in an ice bath, reacting for 10 minutes, then anhydrous sodium sulfate (25g) was added to the reaction solution in an ice bath, and then ice water was slowly added to quench the reaction, filtering and concentrating to obtain the crude product, which was then separated and purified by column chromatography (petroleum ether : ethyl acetate=4:1) to obtain the target compound (1.4 g, 86.5% yield).

$^{1}$H NMR ($CDCl_3$, 600 MHz): δ 7.25 - 7.23 (m, 1 H), 6.98 - 6.96 (m, 2 H), 3.69 (t, *J* = 6.6 Hz, 2 H), 1.88 (t, *J* = 6.6 Hz, 2 H), 1.30 (brs, 1 H), 0.85 - 0.81 (m, 2 H), 0.75-0.72 (m, 2 H).

**Step d: synthesis of 1-(5',6'-dihydro-8'H-spiro[cyclopropane-1,4'-thieno[2, 3-c]oxepine]-8'-yl)-N-methylmethylamine**

2-(1-(thiophen-3-yl)cyclopropyl)ethanol (800.0mg, 4.75mmol) and 2,2-dimethoxy-N-methylethylamine (566.0mg, 4.75mmol) were dissolved in dioxane (20mL), then trifluoromethanesulfonic acid (712.9mg, 4.75mmol) was added dropwise at room temperature, followed by microwave reaction at 110°C for 2 hours. After the reaction was completed, the reaction solution was poured into water (90mL), alkalizing with saturated aqueous sodium bicarbonate, extracting with ethyl acetate (80mL×2); then the organic phase was combined, drying with anhydrous sodium sulfate, filtering and concentrating to obtain the crude product, which was then separated and purified by column chromatography (dichloromethane:methanol=10:1) and preparative liquid chromatography to obtain the target product (22.0 mg, 11.4% yield).

$^{1}$H NMR ($CDCl_3$, 600 MHz): δ 7.02 (d, *J* = 5.4 Hz, 1 H), 6.74 (d, *J* = 4.8 Hz, 1 H), 5.17 (dd, *J* = 11.4, 3.0 Hz, 1 H), 4.28 - 4.22 (m, 1 H), 4.04 - 3.97 (m, 1 H), 3.61 - 3.56 (m, 1 H), 3.39 - 3.31 (m, 1 H), 2.85 (s, 3 H), 2.18 - 2.08 (m, 1 H), 1.20 - 1.10 (m, 2 H), 0.82 - 0.68 (m, 3 H).

**Example 28: N-methyl-1-(7'-methyl-5'H,7'H-spiro[cyclopropane-1,4'-thieno [2,3-c]pyran]-7'-yl)methylamine**

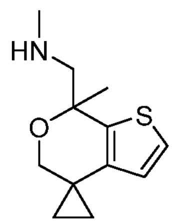

Synthesis route:

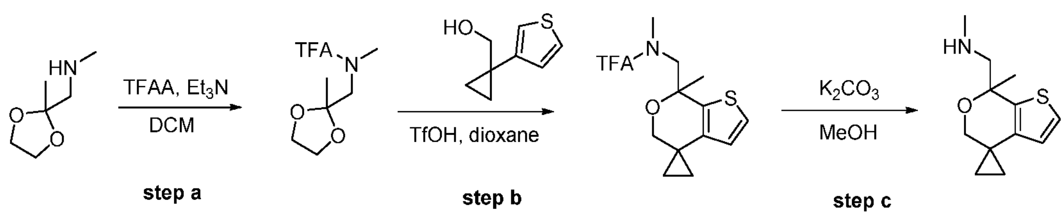


**Step** a: **synthesis of 2,2,2-trifluoro-N-methyl-N-((2-methyl-1,3-dioxolan-2-y l)methyl)acetamide**

N-methyl-1-(2-methyl-1,3-dioxolan-2-yl)methylamine (300.0mg, 2.29 mmol) was dissolved in dichloromethane (10mL), then triethylamine (578.6mg, 5.72mmol) was added, and trifluoroacetic anhydride (624.5mg, 2.97mmol) was added dropwise at 0°C, then slowly heating to room temperature and stirring overnight. The next day, the mixture was poured into saturated aqueous sodium bicarbonate (50mL), extracting twice with dichloromethane (30mL × 2); the collected organic phase was dried with anhydrous sodium sulfate, then filtering and concentrating to obtain the crude product of the target compound (500 mg, 96.1% yield).

**Step b: synthesis of 2,2,2-trifluoro-N-methyl-N-((7'-methyl-5'H,7'H-spiro[c yclopropane-1,4'-thieno[2,3-c]pyran]-7'-yl)methyl)acetamide**

(1-(thiophene-3-yl)cyclopropyl)methanol (100.0mg, 0.65mmol) and 2,2,2-trifluoro-N-methyl-N-((2-methyl-1,3-dioxolan-2-yl)methyl)acetamide (221.0 mg, 0.97mmol)were dissolved in dioxane (10mL), and trifluoromethanesulfonic acid (291.9mg, 1.95mmol) was slowly added dropwise at 0°C, then reacting overnight at room temperature. The next day, the reaction solution was poured into a sodium bicarbonate aqueous solution (30mL), extracting twice with ethyl acetate (30mL × 2), the collected organic phase was dried with anhydrous sodium sulfate, then filtering and concentrating to obtain the crude product, which was separated and purified by column chromatography (petroleum ether: ethyl acetate=10:1) to obtain the target compound (53 mg, 25.6% yield).

LC-MS $[M+H]^+$: 319.9.

**Step c: synthesis of N-methyl-1-(7'-methyl-5'H,7'H-spiro[cyclopropane-1,4 '-thieno[2,3-c]pyran]-7'-yl)methylamine**

2,2,2-trifluoro-N-methyl-N-((7'-methyl-5'H,7'H-spiro[cyclopropane-1,4'-thieno[2 ,3-c]pyran]-7'-yl)methyl)acetamide (53.0mg, 0.17mmol) was dissolved in a mixed solvent of methanol and water (3mL/0.3mL), and potassium carbonate (45.9mg, 0.33mmol) was added, then stirring at 70°Cfor three hours; after that, the mixture was filtered and concentrated to obtain the crude product, which was then separated and purified by preparative liquid chromatography to obtain the target compound (30 mg, 53.6% yield).

$^1$H NMR (600MHz,DMSO-$d_6$) δ 7.47 (d, $J$ = 4.8 Hz, 1 H), 6.66 (d, $J$ = 4.8 Hz, 1 H), 3.76 (d, $J$ = 12.0 Hz, 1 H), 3.68 (d, $J$ = 12.0 Hz, 1 H), 3.43 (d, $J$ = 13.2 Hz, 1 H), 3.36 (d, $J$ = 13.2 Hz, 1 H), 2.58 (s, 3 H), 1.57 (s, 3 H), 1.00 - 0.87(m, 4 H).

LC-MS $[M+H]^+$: 223.95.

**Example 29: N-((5'H,7'H-spiro[cyclopropane-1,4'-thieno[2,3-c]pyran]-7'-yl) methyl)-2,2,2-trifluoroethyl-1-amine**

**Synthesis route:**

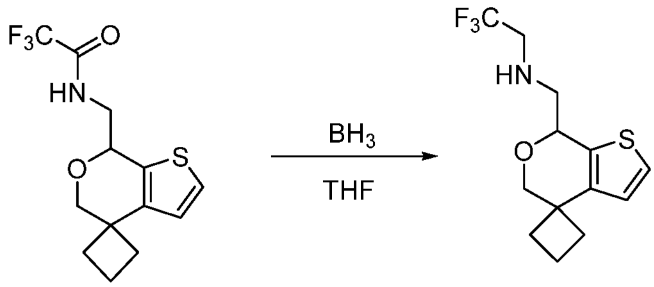


N-(5'H,7'H-spiro[cyclopropane-1,4'-thieno[2,3-c]pyran]-7'-yl)methyl)-2,2,2-amm onium trifluoroacetate (200.0mg, 0.65mmol) was dissolved in tetrahydrofuran (10mL), then tetrahydrofuran solution of borane(1.98mL, 1.98mmol, 1M in THF)was added dropwise, reacting at 70°C for two hours after addition, cooling to room temperature and concentrating to obtain the crude product, which was then separated and purified by preparative liquid chromatography to obtain the target product (32.9mg, 17.2% yield).

$^1$H NMR (400 MHz, $CD_3OD$) δ 7.31 (dd, $J$ = 5.2 Hz, 1H), 7.24 (d, $J$ = 5.2 Hz, 1H), 4.88 - 4.82 (m, 1H), 4.21 (d, $J$ = 11.2 Hz, 1H), 3.66 (d, $J$ = 11.2 Hz, 1H), 3.41-3.25 (m, 2H), 3.00 (d, $J$ = 6.0 Hz, 2H), 2.52 - 2.39 (m, 1H), 2.29 - 1.92 (m, 5H).

LC-MS $[M+H]^+$: 292.1.

**Example 30: 1-(3'-bromo-5'H,7'H-spiro[cyclopropane-1,4'-thieno[2,3-c]pyran]-7'-yl)-N-methylmethylamine**

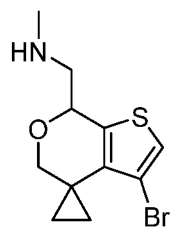

**Synthesis route:**

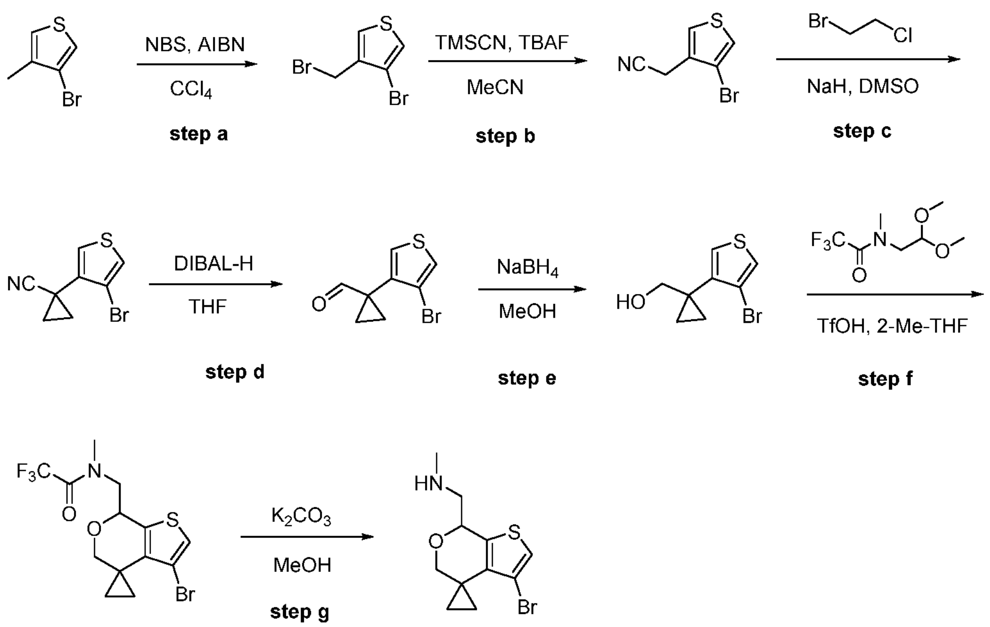


**Step a: synthesis of 3-bromo-4-(bromomethyl)thiophene**

3-bromo-4-methylthiophene (2.0g, 11.3mmol), N-bromosuccinimide (2.02g, 11.36mmol) and azobisisobutyronitrile (93.0mg, 0.57mmol) were dissolved in carbon tetrachloride (30mL), the reaction solution was refluxed for 2 hours under nitrogen protection. The reaction mixture was cooled to room temperature, filtering and concentrating; then the residue was purified by column chromatography to obtain the product (2.3g, 79.5% yield).
$^1H$ NMR (400 MHz, $CDCl_3$): δ 7.40 (d, *J* = 4.4 Hz, 1 H), 7.29 (d, *J* = 4.4 Hz, 1 H), 4.48 (s, 2 H).

**Step b: synthesis of 2-(4-bromothiophene-3-yl)acetonitrile**

3-bromo-4-(bromomethyl)thiophene (2.3g, 8.99mmol) was dissolved in acetonitrile (30mL), trimethylsilylcyanide (2.7g, 27.2mmol) and tetrabutylammonium fluoride (2.7mL, 27.2mmol) were slowly added, stirring overnight at room temperature; after that, the reaction mixture was cooled to 0°C, water (100mL) was added, then extracting with ethyl acetate (100mL × 3). The extract phases were combined, washing with water (100mL × 2) and salt solution (100 mL), drying with anhydrous sodium sulfate, filtering and concentrating in vacuo; then the crude product was purified by column chromatography (petroleum ether:ethyl acetate=10:1) to obtain the product (1.25g, 68.8% yield).
$^1H$ NMR (400 MHz, $CDCl_3$): δ 7.40 (d, *J* = 4.4 Hz, 1 H), 7.34 (d, *J* = 4.4 Hz, 1 H), 3.68 (s, 2 H).

**Step c: synthesis of 1-(4-bromothiophene-3-yl)cyclopropanenitrile**

Sodium hydride (597.0mg, 14.93mmol) was slowly added to dimethyl sulfoxide (20mL), then 2-(4-bromothiophene-3-yl)acetonitrile (1.2g, 5.97mmol) and 1-bromo-2-chloroethane (2.12g, 14.93mmol) were slowly added to the mixture. The reaction mixture was stirred overnight at room temperature. After cooling to 10°C, water (100mL) was added, then extracting with ethyl acetate (100mL × 3). The extract phases were combined, washing with water (100mL

× 2) and salt solution (100mL), drying with anhydrous sodium sulfate, filtering and concentrating; then the residue was purified by column chromatography (petroleum ether: ethyl acetate=10:1) to obtain the product (1.13 g, 82.9% yield).

$^1$H NMR (400 MHz, $CDCl_3$): δ 7.30 (d, *J* = 4.4 Hz, 1 H), 7.22 (d, *J* = 4.0 Hz, 1 H), 1.75 - 1.63 (m, 2 H), 1.37 - 1.23 (m, 2 H).

**Step d: synthesis of 1-(4-methylthiophen-3-yl)cyclopropane formaldehyde**

1-(4-bromothiophen-3-yl)cyclopromethonitrile (1.1g, 4.85mmol) was dissolved in tetrahydrofuran (15mL), and diisobutyl aluminum hydride (1M in hexane, 9.8 mL, 9.8 mmol) was slowly added to the reaction solution under nitrogen protection at 0°C; after the addition was completed, the reaction mixture was heated to room temperature and stirred overnight. The reaction mixture was cooled to 0°C, adding water (50 mL) and extracting with ethyl acetate (50mL × 3). The extract phases were combined, washing with water (50mL × 3) and salt solution (50mL), drying with anhydrous sodium sulfate, filtering and concentrating to obtain the product (930mg, 83.8% yield).

$^1$H NMR (400 MHz, $CDCl_3$): δ 9.21 (s, 1 H), 7.32 (d, *J* = 4.4 Hz, 1 H), 7.15 (d, *J* = 4.4 Hz, 1 H), 1.72 -1.60 (m, 2 H), 1.42 - 1.30 (m, 2 H).

**Step e: synthesis of (1-(4-bromothiophene-3-yl)cyclopropyl)methanol**

1-(4-bromothiophen-3-yl)cyclopropane formaldehyde (930.0mg, 4.04mmol) was dissolved in methanol (10mL), sodium borohydride (169.0mg, 4.45mmol)was slowly added at 0°C, and the reaction mixture was heated to room temperature and stirred for 3 hours. Water (50mL) was added to the reaction mixture, extracting with ethyl acetate (30mL × 3). The combined extract phases were washed with water (30mL × 3) and salt solution (30mL), drying with anhydrous sodium sulfate, filtering and concentrating; then the obtained residue was purified by column chromatography (petroleum ether: ethyl acetate=1:1) to obtain the target product (800.0 mg, 84.9% yield).

$^1$H NMR (400 MHz, $CDCl_3$): δ 7.27 (d, *J* = 3.2 Hz, 1 H), 7.18 (d, *J* = 4.0 Hz, 1 H), 3.66 (s, 2 H), 0.89 (d, *J* = 14.4 Hz, 4 H).

**Step f: N-((3'-bromo-5'H,7'H-spiro[cyclopropane-1,4'-thieno[2,3-c]pyran]-7 '-yl)methyl)-2,2,2-trifluoro-N-methylacetamide**

(1-(4-bromothiophen-3-yl)cyclopropyl)methanol (800.0mg, 3.45mmol) and N-(2,2-dimethoxyethyl)-2,2,2-trifluoro-N-methylacetamide (1.3g, 6.21mmol) were dissolved in 2-methyltetrahydrofuran (10mL), and trifluoromethanesulfonic acid (3.1g, 20.7mmol) was slowly added to the solution at 0°C; the reaction mixture was heated to room temperature and stirred for 2 hours, then cooling to 0°C, diluting with dichloromethane (50mL), washing with 5% sodium hydroxide (50mL × 3) and salt solution (50mL × 3), drying with anhydrous sodium sulfate, filtering and concentrating in vacuo. The residue was separated and purified by column chromatography (petroleum ether: ethyl acetate=10:1) to obtain the product (500.0 mg, 37.6% yield).

LC-MS $[M+H]^+$: 384.2.

**Step g: synthesis of 1-(3'-bromo-5'H,7'H-spiro[cyclopropane-1,4'-thieno[2, 3-c]pyran]-7'-yl)-N-methylmethylamine**

N-((3'-bromo-5'H,7'H-spiro [cyclopropane-1,4'-thieno [2,3-c]pyran]-7'-yl)methyl)-2,2,2-trifluoro-N-methylacetamide (250.0mg, 0.65mmol) was dissolved in methanol (10mL), and potassium carbonate (270.0mg, 1.95mmol) was added to the solution; the reaction mixture was stirred at room temperature for 3 hours, then the reaction mixture was filtered and concentrated to obtain the residue, which was separated and purified by preparative liquid chromatography (water/formic acid/acetonitrile) to obtain the target product (62.4 mg, 28.8% yield).

$^1$H NMR (400 MHz, DMSO-$d_6$): δ 8.24 (s, 1 H), 7.50 (s, 1 H), 4.95 - 4.85 (m, 1 H), 3.85 (d, *J* = 15.6 Hz, 1 H), 3.38 (d, *J* = 15.6 Hz, 1 H), 3.06 - 2.78 (m,2 H), 2.41 (s, 3 H), 1.95 - 1.80 (m,1 H), 1.40 - 1.30 (m,1 H), 0.90 - 0.76 (m,1 H), 0.65 - 0.50 (m,1 H).

LC-MS $[M+H]^+$: 288.0.

**Example 32: N-((4',6'-dihydrospiro[cyclopropane-1,7'-thieno[3,2-c]pyran]-4' -yl)methyl)methyl-$D_3$-amine**

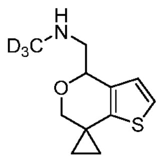

**Synthesis route:**

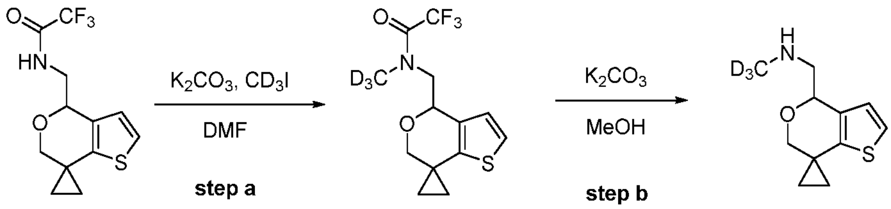


**Step a: synthesis of N-((4',6'-dihydrospiro[cyclopropane-1,7'-thieno[3,2-c] pyran]-4'-yl)methyl)2,2,2-trifluoro-N-(methyl-$D_3$)acetamide**

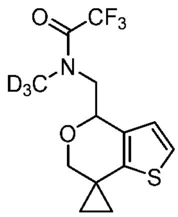

Potassium carbonate (950mg, 6.86mmol) and deuterated iodomethane (1.5g, 10.3mmol) were successively added to a N,N-dimethylformamide solution (10mL) ofN-((4',6'-dihydrospiro [cyclopropane-1,7'-thieno [3,2-c]pyran]-4'-yl)methyl)2,2,2-trif l-uoroacetamide (1.0g, 3.43mmol), stirring at room temperature for 4 hours; after the reaction was completed, the reaction solution was poured into a saturated ammonium chloride aqueous solution (70mL), extracting with ethyl acetate (30mL × 3), and concentrating the extract phase to obtain the crude product, which was then separated and purified by column chromatography (petroleum ether: ethyl acetate=10:1) to obtain the target compound (600mg, 56% yield).

LC-MS $[M+H]^+$: 308.9.

$^1H$ NMR (600 MHz, $CDCl_3$): δ 7.02 (d, *J* = 4.8 Hz, 1 H), 6.83 (d, *J* = 5.4 Hz, 1 H), 5.06 (dd, *J* = 9.0, 2.4 Hz, 1H), 4.12 (dd, *J* = 14.4, 2.4 Hz, 1H), 3.81 (d, *J* = 11.4 Hz, 1 H), 3.61 (d, *J* = 11.4 Hz, 1 H), 3.54 - 3.48 (m, 1 H), 1.07 - 0.93(m, 4 H).

**Step b: synthesis of N-((4',6'-dihydrospiro[cyclopropane-1,7'-thieno[3,2-c] pyran]-4'-yl)methyl)methyl-$D_3$-amine**

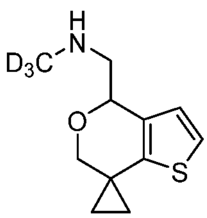

Potassium carbonate (540mg, 3.9mmol) was added to a methanol (6mL) solutionofN-(4',6'-dihydrospiro[cyclopropane-1,7'-thieno[3,2-c]pyran]-4'-yl)methyl)2, 2,2-trifluoro-N-(methyl-$D_3$)acetamide (600mg, 1.95mmol), stirring at room temperature for 2 hours; after the reaction was completed, the reaction solution was filtered and concentrated, and the crude product was separated and purified by preparative liquid chromatography (acetonitrile: water=25:75) to

obtain the target product (340 mg, 53.5% yield).

LC-MS $[M+H]^+$: 212.9.

$^1H$ NMR (600 MHz, $CDCl_3$): $\delta$ 7.00 (d, *J* = 4.8 Hz, 1 H), 6.75 (d, *J* = 4.8 Hz, 1 H), 4.91 (dd, *J* = 9.0, 3.0 Hz, 1 H), 3.93 (d, *J* = 11.4 Hz, 1 H), 3.56 (d, *J* = 11.4 Hz, 1 H), 3.01 (dd, *J* = 12.6, 3.0 Hz, 1 H), 2.92 (dd, *J* = 12.6, 9.0 Hz, 1 H), 1.06 - 0.89 (m, 4 H).

**Example 32-S and Example 32-R:**

**(*S*)-N-((4',6'-dihydrospiro[cyclopropane-1,7'-thieno[3,2-c]pyran]-4'-yl)methy l)methyl-$D_3$-amine and (*R*)-N-((4',6'-dihydrospiro[cyclopropane-1,7'-thieno[3,2-c]pyran]-4'-yl)methyl)methyl-$D_3$-amine**

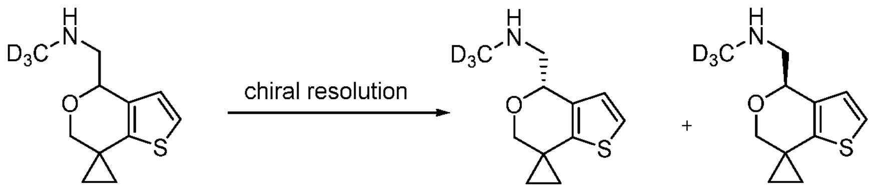


32 32-S 32-R

Example 32 was resolved by chiral chromatography to obtain Example 32-S and Example 32-R. Liquid chromatography analysis method: chromatographic column: DAICEL CHIRALPAK IG column; mobile phase A: supercritical $CO_2$, mobile phase B: methanol (containing 0.1% diethylamine); detection wavelength: 214nm; flow rate: 1.5mL/min; column temperature: 35°C; background column pressure: 1800psi.Mobile phase gradient:

| time(min) | A(%V/V) | B(%V/V) |
|---|---|---|
| 0.00 | 95 | 5 |
| 0.50 | 95 | 5 |
| 5.50 | 60 | 40 |
| 10.00 | 60 | 40 |

Example 32-S:

RT: 3.236 min; $[\alpha]D^{23}$ = 63 (c 0.10, MeOH); LC-MS $[M+H]^+$: 212.9.

$^1H$ NMR (600 MHz, $CDCl_3$): $\delta$ 7.00 (d, *J* = 5.4 Hz, 1 H), 6.75 (d, *J* = 5.4 Hz, 1 H), 4.91 (dd, *J* = 9.0, 3.0 Hz, 1 H), 3.93 (dd, *J* = 11.4, 1.2 Hz, 1 H), 3.56 (d, *J* = 11.4 Hz, 1 H), 3.01 (dd, *J*=12.6, 3.0 Hz,1H), 2.92 (dd, *J* = 12.6, 9.0 Hz, 1 H), 1.06-0.90 (m, 4 H).

Example 32-R:

RT: 3.624 min; $[\alpha]D^{23}$ = -60 (c 0.11, MeOH); LC-MS $[M+H]^+$: 212.9.

$^1H$ NMR (600 MHz, $CDCl_3$): $\delta$ 7.00 (d, *J* = 4.8 Hz, 1 H), 6.75 (d, *J* = 4.8 Hz, 1 H), 4.91 (dd, *J* = 8.4, 3.0 Hz, 1 H), 3.93 (d, *J* = 11.4 Hz, 1 H), 3.56 (d, *J* = 11.4 Hz, 1 H), 3.00 (dd, *J* = 12.6, 3.0 Hz, 1 H), 2.91 (dd, *J* = 12.6, 8.4 Hz, 1 H), 1.06 - 0.90 (m, 4 H).

**BIOLOGY TEST EVALUATION**

The following further describes and explains the present application in combination with test examples, but these examples are not meant to limit the scope of the present application.

The following SEP-363856 of the present application is as follows, and it was prepared with reference to the method of Example 129 of patent PCT/US2010/058884,

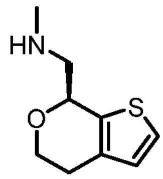

.

The following comparative example 1 of the present application is as follows, and it was prepared with reference to the method of Example 89 of patent PCT/US2010/058884,

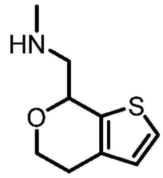

.

**Test example 1: test method of TAAR1 receptor cAMP agonist**

1.1 Experimental materials:

The cAMP detection kit was purchased from Cisbio; the HE3K293 cell line stably expressing TAAR1 receptor was constructed by Shanghai Shujing Biotechnology Co., Ltd.; IBMX was purchased from Sigma; Phenethylamine (PEA); ProxiPlate-384 well plate was purchased from PerkinElmer; HBSS was purchased from Thermo Fisher Scientific Company. PerkinElmer Envision 2105 multifunctional microplate reader, Agilent Bravo liquid workstation, Countstar BioTech cell counter.

1.2 Experimental method:

(1) Preparation of experimental buffer: 5× stimulation buffer wasdiluted to 1× with dd$H_2O$, adding IBMX with a final concentration of 0.5mM, mixing well for later use.
(2) The frozen cells were thawed quickly in a 37°C water bath, the cell suspension was washed with HBSS buffer, centrifuging at 200×g to remove the frozen storage solution; the pellet was resuspended with an appropriate amount of experimental buffer, taking 20 μL for counting with a cell counter, and diluting to $1.5\times10^6$ cells/mL.
(3) 5 μL of cell suspension ($1.5\times10^4$ cells per well) was added to the ProxiPlate-384 well plate.
(4) The compound to be tested was diluted in the experimental buffer by gradient, and 5 μL was transferred to the reaction plate using Bravo. 5 μL experimental buffer was put in each negative control well, and 5 μL PEA (final concentration $10^{-2}$M) was put in each positive control well.
(5) Incubating at 37°C for 1 hour.
(6) 10 μL of detection reagent was added to the reaction plate, incubating at room temperature for 1 hour in dark.
(7) Envision 2105 multifunctional microplate reader was used for detection with excitation light 340nm, emission light 620nm and 665nm.The ratio of 665nm/620nm for each test well was calculated.

$$\text{Activation rate (activity\%)}=(\text{negative control ratio}-\text{compound ratio})/(\text{negative control ratio}-\text{positive control ratio})\times100\%$$

The $EC_{50}$ values of the compounds were calculated using the four-parameter fitting model log(agonist)vs.response-- Variable slope(four parameters) in GraphPad Prism.

1.3 Experimental results:

The specific results of the compounds of the present application in the TAAR1 receptor agonist activity test through the above scheme are shown in Table 1.

Table 1: the results of the agonistic activity of the compounds of the present application on TAAR1 receptors

| Example number | TAAR1 | |
|---|---|---|
| | $EC_{50}$ (nM) | Max Act% |
| Example 8 | 1632 | 96.6% |
| Example 8-S | 704 | 96.1% |
| Example 9 | 1666 | 95.5% |
| Example 9-S | 795 | 95.8% |
| Example 9-R | 1549 | 95.5% |
| Example 15 | 1726 | 93.3% |
| Example 32 | 1324 | 97.7% |
| Example 32-S | 806 | 97.1% |
| Comparative example 1 | 1646 | 100.5% |
| SEP-363856 | 925 | 108.3% |

**1.4 Experimental conclusion:**

The in vitro experimental results indicate that the compounds of the present application have an agonist effect on the TAAR1 receptors.

**Test example 2: test method of 5-$HT_{1A}$ receptor cAMP agonist**

2.1 Experimental materials:

The cAMP detection kit was purchased from Cisbio; the HEK293 cell line stably expressing 5-$HT_{1A}$ receptor was constructed by Shanghai Shujing Biotechnology Co., Ltd.; Serotonin, Forskolin and IBMX were purchased from Sigma; ProxiPlate-384-well plates was purchased from PerkinElmer; HBSS was purchased from Thermo Fisher Scientific. PerkinElmer Envision 2105 multifunctional microplate reader, Tecan D300e picoliter micro-dosing system, Agilent Bravo liquid workstation, Countstar BioTech cell counter.

2.2 Experimental method:

(1) Preparation of experimental buffer: 5×stimulation buffer was diluted to 1× with $ddH_2O$, adding IBMX with a final concentration of 0.5mM, mixing well for later use.
(2) The cultured cells were digested with trypsin, and the cell suspension was washed with HBSS buffer after the digestion was terminated, centrifuging to remove the culture medium at 200×g; the precipitate was resuspended with an appropriate amount of experimental buffer, taking 20 μL for counting with a cell counter, and diluting to 0.4 × $10^6$ cells/mL.
(3) 5 μL cell suspension (2x$10^3$ cells per well) was added to ProxiPlate-384 well plate.
(4) The compound to be tested was diluted in the experimental buffer by gradient, and 5 μL was transferred to the reaction plate using Bravo. 5 μL experimental buffer was put in each negative control well, and 5 μL Serotonin (final concentration $10^{-6}$M) was put in each positive control well.
(5) Incubating at room temperature for 15 minutes.
(6) Forskolin (final concentration 1.5×$10^{-7}$M) was added to the reaction plate using TecanD300e picoliter micro-dosing system.
(7) Incubating at room temperature for 45 minutes.
(8) 10 μL detection reagent was added to the reaction plate, incubating at room temperature for 1 hour in dark.
(9) Envision 2105 multifunctional microplate reader was used for detection with excitation light 340nm, emission light 620nm and 665nm.The ratio of665nm/620nm for each test well was calculated.

Activation rate (activity%)=(negative control ratio-compound ratio)/(negative control ratio-positive control ratio)×100%

The values of the compounds were calculated using the four-parameter fitting model log(agonist)vs. response--Variable slope(four parameters) in Graph Pad Prism.

2.3 Experimental results:

The specific results of the compounds of the present application in the 5-$HT_{1A}$ receptor agonist activity test through the above scheme are shown in Table 2.

Table 2: the results of the agonistic activity of the compounds of the present application on 5-$HT_{1A}$ receptors

| **Example number** | **5-$HT_{1A}$** | |
|---|---|---|
| | **$EC_{50}$ (nM)** | **Max Act%** |
| Example 2 | 257.4 | 108.9% |
| Example 7 | 393.9 | 93.4% |
| Example 8 | 1542 | 91.6% |
| Example 8-S | 903 | 90.7% |
| Example 9 | 2438 | 92.7% |
| Example 9-R | 1039 | 95.2% |
| Example 15 | 2684 | 93.7% |
| Example 16 | 1375 | 115.6% |
| Example 23 | 5510 | 75.5% |
| Example 32 | 1884 | 80.5% |
| Example 32-R | 1670 | 86.3% |
| Comparative example 1 | 47746 | 59.5% |
| SEP-363856 | 13130 | 82.4% |

2.4 Experimental conclusion:

The results of in vitro experiments show that the compounds of the present application have agonistic effect on 5-$HT_{1A}$ receptors, suggesting that the compounds of the present application can improve negative symptoms and cognitive injury.

**Test example 3: inhibition experiments of the compounds of the present application on high spontaneous activity induced by MK-801 in mice**

3.1 Experimental materials:

Compounds to be tested: compounds in the examples of the present application, self-made.
(+)-MK-801 bimaleate: purchased from Sigma-Aldrich Company, article number: M107-50MG.
Experimental animals: 18-22g male C57B1/6J mice, purchased from Shanghai Slack Experimental Animal Co., Ltd.

3.2 Experimental method:

3.2.1 Grouping of animals: before the start of the experiment, animals were randomly grouped according to body weight.

3.2.2 Animal adaptation: before the experiment, the mice were first adapted to the experimental environment for at least 1 hour. That is, the animals were transferred from the breeding room to the laboratory and moved freely in the cage.

3.2.3 Administration:

Drug preparation: taking the compound to be tested, adding pure water and performing ultrasonication.

The animals were randomly divided into blank group, model group, and administration group according to body weight, 9 mice /group, and the detailed administration information is shown in the following table:

| Group | Dosage of administration (mg/kg, p.o.) | MK-801 modeling dose(mg/kg, i.p.) | Number of groups | Number of animals (9 mice /group, ♂) |
|---|---|---|---|---|
| Blank group | 0 | 0 | 1 | 9 |
| Model group | 0 | 0.3 | 1 | 9 |
| Example 8-S | 0.3, 1, 3, 10 | 0.3 | 4 | 36 |
| Example 9-R | 0.3, 1, 3, 10, 30 | 0.3 | 5 | 45 |
| SEP-363856 | 0.3, 1, 3, 10, 30 | 0.3 | 5 | 45 |

T-30min oral administration of compound: compound to be tested or solvent (pure water) was administered orally by gavage, and put into test box (test box size length * width * height=27 * 27 * 40cm) immediately after administration, recording the spontaneous activity of the mice within 30 minutes.

T0 min intraperitoneal injection of modeling drug: 30 minutes after compound administration, the mice were taken out, and administered withMK-801 (0.3 mg/kg) by intraperitoneal injection. The blank control group was injected with normal saline. After the administration of MK-801, the animals were immediately put back into the test box, and the test was continued for 150 minutes.

3.2.4 Data recording and analysis.

The activities of these animals (movement distance in the test box) were automatically recorded by the camera, and analyzed with ANY MAZE software, and the $ED_{50}$values were calculated.

3.3 Experimental results: as shown in Table 3.

Table 3: Experimental results of the inhibitory effect of the compounds of the present application on high spontaneous activity induced by MK-801 in mice

| **Group** | **$ED_{50}$ (mg/kg)** | **MED (mg/kg)** |
|---|---|---|
| Example 8-S | 0.36 | 0.3 |
| Example 9-R | 4.48 | 10 |
| SEP-363856 | 10.35 | 30 |
| Note: $ED_{50}$ is the half effective dose, and MED is the minimum effective dose. | | |

3.4 Experimental conclusion:

Through the above scheme, it can be concluded that the compounds of the present application can significantly inhibit the high spontaneous activity induced by MK-801 in mice, and the inhibitory effect is gradually enhanced with the increase of the compound dosage, and there is a good dose-dependent relationship. Compared to SEP-363856, the compounds of the present application have a lower minimum effective dose and stronger inhibitory effect.

**Test example 4: inhibition experiments of the compounds of the present application on high spontaneous activity induced by PCP in mice**

4.1 Experimental materials:

Compounds to be tested: compounds of the examples of the present application, self-made.
Phencyclidine hydrochloride (PCP): purchased from Shanghai Yuansi Standard Science Technology Co., Ltd., specification: 5g.
Experimental animals: 18-22g male C57B1/6J mice, purchased from Shanghai Slack Experimental Animal Co., Ltd.

4.2 Experimental method:

4.2.1 Grouping of animals: before the start of the experiment, animals were randomly grouped according to body weight.
4.2.2 Animal adaptation: before the experiment, the mice were first adapted to the experimental environment for at least 1 hour. That is, animals were transferred from the feeding room to the laboratory, and moved freely in the cage.
4.2.3 Administration:
Drug preparation: taking the compound to be tested, adding pure water and performing ultrasonication.
The animals were randomly divided into blank group, model group, and administration group according to body weight, 9 mice /group, and the detailed administration information is shown in the following table:

| Group | Dosage of administration ( mg/kg, p.o.) | PCP modeling dose (mg/kg, i.p.) | Number of groups | Number of animals (9 mice /group, ♂) |
|---|---|---|---|---|
| Blank group | 0 | 0 | 1 | 9 |
| Model group | 0 | 5 | 1 | 9 |
| Example 8-S | 0.1, 0.3, 1, 3, 10 | 5 | 5 | 45 |
| Example 9-R | 0.1, 0.3, 1, 3, 10 | 5 | 5 | 45 |
| SEP-363856 | 0.1, 0.3, 1, 3, 10 | 5 | 5 | 45 |

T-30min oral administration of compound: compound to be tested or solvent (pure water) was administered orally by gavage, and put into test box (test box size length * width * height=27 * 27 * 40cm) immediately after administration, recording the spontaneous activity of the mice within 30 minutes.
T0 min intraperitoneal injection of modeling drug: 30 minutes after compound administration, the mice were taken out, and administered with PCP (5 mg/kg) by intraperitoneal injection. The blank control group was injected with pure water. After the administration of PCP, the animals were immediately put back into the test box, and the test was continued for 60 minutes.
4.2.4 Data recording and analysis.
The activities of these animals (movement distance in the test box) were automatically recorded by the camera, and analyzed with ANY MAZE software, and the ED50 values were calculated.
4.3 Experimental results: as shown in Table 4.

Table 4: results of the inhibitory effect of the compounds of the present application on high spontaneous activity induced by PCP in mice

| Group | $ED_{50}$ (mg/kg) | MED (mg/kg) |
|---|---|---|
| Example 8-S | 1.97 | 0.3 |
| Example 9-R | 0.45 | 0.1 |
| SEP-363856 | 1.84 | 3 |
| Note: $ED_{50}$ is the half effective dose, and MED is the minimum effective dose. | | |

4.4 Experimental conclusion:

Through the above scheme, it can be concluded that the compounds of the present application can significantly inhibit the high spontaneous activity induced by PCP in mice, and the inhibitory effect is gradually enhanced with the increase of the compound dosage, and there is a good dose-dependent relationship. Compared to SEP-363856, the compounds of the present application have a lower minimum effective dose and stronger inhibitory effect.

## Claims

**1.** A compound represented by general formula (I), a stereoisomer or a pharmaceutically acceptable salt thereof,

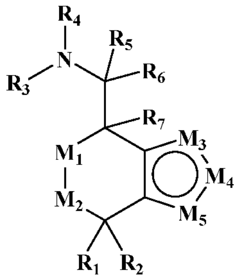

(I) ,

wherein

$M_1$ and $M_2$ are each independently selected from $-(CR_AR_B)_n-$, O or S;
$M_3$ is selected from $CR_a$, N or S;
$M_4$ is selected from $CR_b$, N or S;
$M_5$ is selected from $CR_c$, N or S;
$R_A$, $R_B$, $R_a$, $R_b$ and $R_c$ are each independently selected from hydrogen, deuterium, halogen, hydroxyl, cyano, amino, $C_{1-6}$ alkyl, $C_{1-6}$ deuterated alkyl, $C_{1-6}$ haloalkyl, $C_{1-6}$ alkoxy or $C_{1-6}$ alkylamine;
$R_1$ and $R_2$ together with the carbon atom to which they are connected are linked to form a $C_{3-8}$ cycloalkyl or a 3-8 membered heterocyclyl;
$R_3$ and $R_4$ are each independently selected from hydrogen, deuterium, halogen, hydroxyl, cyano, $C_{1-6}$ alkyl, $C_{1-6}$ deuterated alkyl or $C_{1-6}$ haloalkyl;
alternatively, $R_3$ and $R_4$ together with the nitrogen atom to which they are connected are linked to form a 3-8 membered nitrogen-containing heterocyclyl;
$R_5$ and $R_6$ are each independently selected from hydrogen, deuterium, halogen, hydroxyl, cyano, $C_{1-6}$ alkyl, $C_{1-6}$ deuterated alkyl or $C_{1-6}$ haloalkyl;
alternatively, $R_5$ and $R_6$ together with the carbon atom to which they are connectedare linked to form a $C_{3-8}$ cycloalkyl or a 3-8 membered heterocyclyl;
alternatively, $R_5$ or $R_6$ is linked with $R_3$ or $R_4$ to form a 3-8 membered nitrogen-containing heterocyclyl;
$R_7$ is selected from hydrogen, deuterium, halogen, hydroxyl, cyano, $C_{1-6}$ alkyl, $C_{1-6}$ deuterated alkyl or $C_{1-6}$ haloalkyl;
alternatively, $R_7$ is linked with $R_3$ or $R_4$ to form a 4-8 membered nitrogen-containing heterocyclyl;
alternatively, $R_7$ is linked with $R_5$ or $R_6$ to form a $C_{3-8}$ cycloalkyl or a 3-8 membered heterocyclyl;
the cycloalkyl or heterocyclyl formed by linking $R_1$ and $R_2$ together with the carbon atom to which they are connected, the nitrogen-containing heterocyclyl formed by linking $R_3$ and $R_4$ together with the nitrogen atom to which they are connected, the cycloalkyl or heterocyclyl formed by linking $R_5$ and $R_6$ together with the carbon atom to which they are connected, the nitrogen-containing heterocyclyl formed by linking $R_5$ or $R_6$ with $R_3$ or $R_4$, the nitrogen-containing heterocyclyl formed by linking $R_7$ with $R_3$ or $R_4$, or the cycloalkyl or heterocyclyl formed by linking $R_7$ with $R_5$ or $R_6$, optionally further substituted by one or more substituents selected from the group consisting of: hydrogen, deuterium, halogen, hydroxyl, cyano, nitro, amino, $C_{1-6}$ alkyl, $C_{1-6}$ alkoxy, $-(CH_2)_{n1}OR_{aa}$, $-(CH_2)_{n1}SR_{aa}$, $-(CH_2)_{n1}NR_{aa}R_{bb}$, $-(CH_2)_{n1}NR_{aa}C(O)R_{bb}$, $-(CH_2)_{n1}C(O)NR_{aa}R_{bb}$, $-(CH_2)_{n1}C(O)R_{aa}$, $-(CH_2)_{n1}C(O)OR_{aa}$, $-(CH_2)_{n1}S(O)_2R_{aa}$, $-(CH_2)_{n1}NR_{aa}S(O)_2R_{bb}$ and$-(CH_2)_{n1}S(O)_2NR_{aa}R_{bb}$;
$R_{aa}$ and $R_{bb}$ are each independently selected from hydrogen, deuterium, halogen, hydroxyl, cyano, nitro, amino, $C_{1-6}$ alkyl, $C_{1-6}$ deuterated alkyl or $C_{1-6}$ haloalkyl;

n is an integer from 0 to 2; and
n1 is an integer from 0to2.

2. The compound represented by the general formula (I), a stereoisomer or a pharmaceutically acceptable salt thereof according to claim 1, wherein

$M_1$ and $M_2$ are each independently selected from $-(CR_AR_B)_n-$, O or S;
$M_3$ is selected from $CR_a$, N or S;
$M_4$ is selected from $CR_b$;
$M_5$ is selected from $CR_c$, N or S;
$R_A$, $R_B$, $R_a$, $R_b$ and $R_c$ are each independently selected from hydrogen, deuterium, halogen, hydroxyl, cyano, amino, $C_{1-6}$ alkyl, $C_{1-6}$ deuterated alkyl, $C_{1-6}$ haloalkyl, $C_{1-6}$ alkoxy or $C_{1-6}$alkylamine;
$R_1$ and $R_2$ together with the carbon atom to whichthey are connected are linked to form a $C_{3-8}$ cycloalkyl or a 3-8 membered heterocyclyl;
$R_3$ and $R_4$ are each independently selected from hydrogen, deuterium, halogen, hydroxyl, cyano, $C_{1-6}$ alkyl, $C_{1-6}$ deuterated alkyl or $C_{1-6}$ haloalkyl;
alternatively, $R_3$ and $R_4$ together with the nitrogen atom to which they are connected are linked to form a 3-8 membered nitrogen-containing heterocyclyl;
$R_5$ and $R_6$ are each independently selected from hydrogen, deuterium, halogen, hydroxyl, cyano, $C_{1-6}$ alkyl, $C_{1-6}$ deuterated alkyl or $C_{1-6}$ haloalkyl;
alternatively, $R_5$ and $R_6$ together with the carbon atom to which they are connected are linked to form a $C_{3-8}$ cycloalkyl or a 3-8 membered heterocyclyl;
alternatively, $R_5$ or $R_6$ is linked with $R_3$ or $R_4$ to form a 3-8 membered nitrogen-containing heterocyclyl;
$R_7$ is selected from hydrogen, deuterium, halogen, hydroxyl, cyano, $C_{1-6}$ alkyl, $C_{1-6}$ deuterated alkyl or $C_{1-6}$ haloalkyl;
alternatively, $R_7$ is linked with $R_3$ or $R_4$ to form a 4-8 membered nitrogen-containing heterocyclyl;
alternatively, $R_7$ is linked with $R_5$ or $R_6$ to form a $C_{3-8}$ cycloalkyl or a 3-8 membered heterocyclyl;
the cycloalkyl or heterocyclyl formed by linking $R_1$ and $R_2$ together with the carbon atom to which they are connected, the nitrogen-containing heterocyclyl formed by linking $R_3$ and $R_4$ together with the nitrogen atom to which they are connected, the cycloalkyl or heterocyclyl formed by linking $R_5$ and $R_6$ together with the carbon atom to which they are connected, the nitrogen-containing heterocyclyl formed by linking $R_5$ or $R_6$ with $R_3$ or $R_4$, the nitrogen-containing heterocyclyl formed by linking $R_7$ with $R_3$ or $R_4$, or the cycloalkyl or heterocyclyl formed by linking $R_7$ with $R_5$ or $R_6$, optionally further substituted by one or more substituents selected from the group consisting of: hydrogen, deuterium, halogen, hydroxyl, cyano, nitro, amino, $C_{1-6}$ alkyl, $C_{1-6}$ alkoxy, $-(CH_2)_{n1}0R_{aa}$, $-(CH_2)_{n1}SR_{aa}$, $-(CH_2)_{n1}NR_{aa}R_{bb}$, $-(CH_2)_{n1}NR_{aa}C(O)R_{bb}$, $-(CH_2)_{n1}C(O)NR_{aa}R_{bb}$, $-(CH_2)_{n1}C(O)R_{aa}$, $-(CH_2)_{n1}C(O)OR_{aa}$, $-(CH_2)_{n1}S(O)_2R_{aa}$, $-(CH_2)_{n1}NR_{aa}S(O)_2R_{bb}$ and $-(CH_2)_{n1}S(O)_2NR_{aa}R_{bb}$;
$R_{aa}$ and $R_{bb}$ are each independently selected from hydrogen, deuterium, halogen, hydroxyl, cyano, nitro, amino, $C_{1-6}$ alkyl, $C_{1-6}$ deuterated alkyl or $C_{1-6}$ haloalkyl;
n is an integer from 0 to 2; and
n1 is an integer from 0to 2.

3. The compound represented by the general formula (I), a stereoisomer or a pharmaceutically acceptable salt thereof according to claim 1 or 2, wherein it satisfies one or more of the following conditions:

(1) $R_A$, $R_B$, $R_a$, $R_b$ and $R_c$ are each independently selected from hydrogen, halogen or $C_{1-6}$ alkyl, preferably hydrogen, fluorine, chlorine, bromine or $C_{1-3}$ alkyl, further preferably hydrogen, fluorine, chlorine, bromine or methyl;
(2) $R_1$ and $R_2$ together with the carbon atom to which they are connected are linked to form a $C_{3-6}$ cycloalkyl or a 3-6 membered heterocyclyl, preferably a $C_{3-4}$ cycloalkyl or a 3-4 membered heterocyclyl, further preferably cyclopropyl, cyclobutyl or oxetanyl;
(3) $R_3$ and $R_4$ are each independently selected from hydrogen, $C_{1-6}$ alkyl, $C_{1-6}$ deuterated alkyl or $C_{1-6}$ haloalkyl, preferably hydrogen, $C_{1-3}$ alkyl, $C_{1-3}$ deuterated alkyl or $C_{1-3}$ haloalkyl, further preferably hydrogen, methyl, ethyl, isopropyl, $-CD_3$ or $-CH_2CF_3$;
(4) $R_3$ and $R_4$ together with the nitrogen atom to which they are connected are linked to form a 4-6 membered nitrogen-containing heterocyclyl, further preferably azetidinyl;
(5) $R_5$ and $R_6$ are each independently selected from hydrogen or $C_{1-6}$ alkyl, preferably hydrogen or $C_{1-3}$ alkyl, further preferably hydrogen or methyl;
(6) Rs and $R_6$ together with the carbon atom to which they are connected are linked to form a $C_{3-5}$ cycloalkyl

or a 3-5 membered heterocyclyl, preferably cyclopropyl;

(7) Rs or $R_6$ is linked with $R_3$ or $R_4$ to form a 4-6 membered nitrogen-containing heterocyclyl, preferably tetrahydropyrrolyl;

(8) $R_7$ is selected from hydrogen or $C_{1-6}$ alkyl, more preferably hydrogen or $C_{1-3}$ alkyl, further preferably hydrogen or methyl;

(9) $R_7$ is linked with $R_3$ or $R_4$ to form a 5-6 membered nitrogen-containing heterocyclyl, further preferably tetrahydropyrrolyl;

(10) $R_7$ is linked with $R_5$ or $R_6$ to form a $C_{4-6}$ cycloalkyl or a 4-6 membered heterocyclyl, further preferably cyclobutyl;

(11) $R_{aa}$ and $R_{bb}$ are each independently selected from hydrogen, deuterium, halogen, hydroxyl, cyano, nitro, amino, $C_{1-3}$ alkyl, $C_{1-3}$ deuterated alkyl or $C_{1-3}$ haloalkyl;

(12) the compound represented by the general formula (I) is selected from:

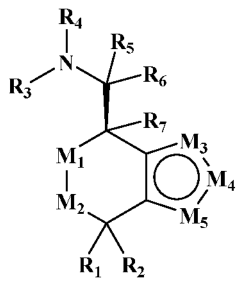

(I a) ,

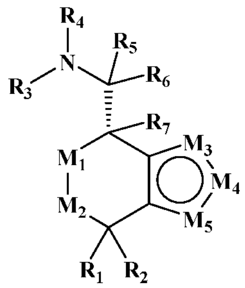

(I b)

or a mixture thereof.

4. The compound represented by general formula (I), a stereoisomer or a pharmaceutically acceptable salt thereof according to claim 1 or 2, wherein it does not comprise the following compounds:

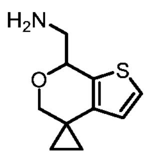 , 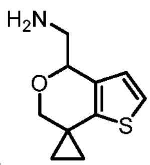 , 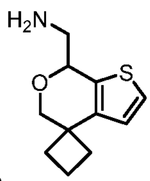 , 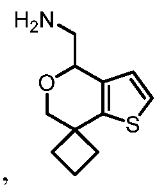 , 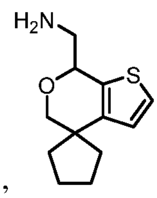 , 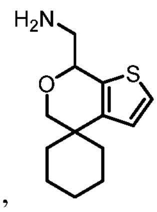 , 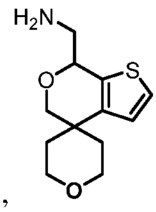 .

5. The compound represented by general formula (I), a stereoisomer or a pharmaceutically acceptable salt thereof according to claim 1 or 2, wherein $M_3$ is selected from S; $M_4$ is selected from $CR_b$ or N; Ms is selected from $CR_c$ or N.

6. The compound represented by general formula (I), a stereoisomer or a pharmaceutically acceptable salt thereof according to claim 1 or 2, wherein $M_3$ is selected from $CR_a$ or N; $M_4$ is selected from S; Ms is selected from $CR_c$ or N.

7. The compound represented by general formula (I), a stereoisomer or a pharmaceutically acceptable salt thereof according to claim 1 or 2, wherein $M_3$ is selected from $CR_a$ or N; $M_4$ is selected from $CR_b$ or N; Ms is selected from S.

8. The compound represented by general formula (I), a stereoisomer or a pharmaceutically acceptable salt thereof according to claim 1 or 2, wherein

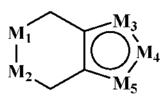

in general formula (I) is selected from the following groups:

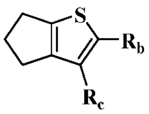 , 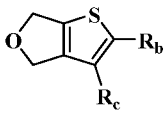 , 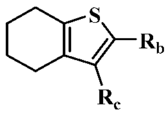 , 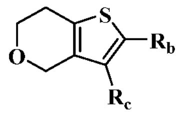 , 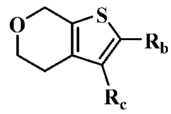 ,

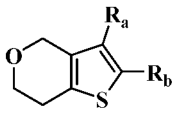 , 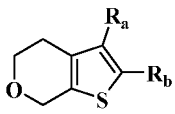 , 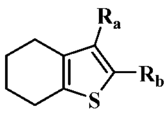 , 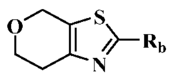 or 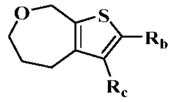 ,

preferably

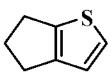 , 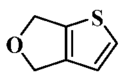 , 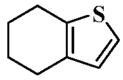 , 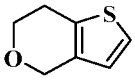 , 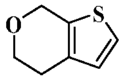 , 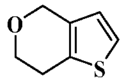 ,

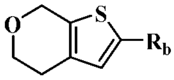 , 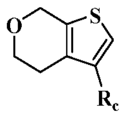 , 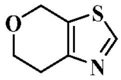 or 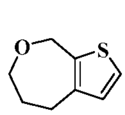 ;

wherein $R_a$, $R_b$ and $R_c$ are each independently selected from hydrogen, deuterium, halogen, hydroxyl, cyano, amino, $C_{1-6}$ alkyl, $C_{1-6}$ deuterated alkyl, $C_{1-6}$ haloalkyl, $C_{1-6}$ alkoxy or $C_{1-6}$ alkylamine, preferably hydrogen, halogen or $C_{1-6}$ alkyl, more preferably hydrogen, fluorine, chlorine, bromine or $C_{1-3}$ alkyl, further preferably hydrogen, fluorine, chlorine, bromine or methyl.

**9.** The compound represented by general formula (I), a stereoisomer or a pharmaceutically acceptable salt thereof according to claim 1 or 2, wherein the general formula (I) is further represented by general formula (II-A):

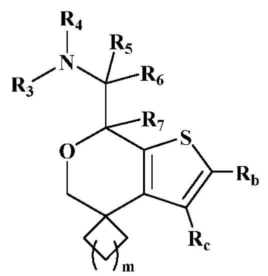

(II-A)

wherein $R_3$ and $R_4$ are each independently selected from hydrogen, $C_{1-6}$ alkyl, $C_{1-6}$ deuterated alkyl or $C_{1-6}$ haloalkyl, preferably hydrogen, $C_{1-3}$ alkyl, $C_{1-3}$ deuterated alkyl or $C_{1-3}$ haloalkyl, more preferably hydrogen, methyl, ethyl, isopropyl, $-CD_3$ or $-CH_2CF_3$;

alternatively, $R_3$ and $R_4$ together with the nitrogen atom to which they are connected are linked to form a 4-6 membered nitrogen-containing heterocyclyl, preferably azetidinyl;

Rs and $R_6$ are each independently selected from hydrogen or $C_{1-6}$ alkyl, preferably hydrogen or $C_{1-3}$ alkyl, more preferably hydrogen or methyl;

alternatively, $R_5$ and $R_6$ together with the carbon atom to which they are connected are linked to form a $C_{3-5}$ cycloalkyl or a 3-5 membered heterocyclyl, preferably a cyclopropyl;

alternatively, $R_5$ or $R_6$ is linked with $R_3$ or $R_4$ to form a 4-6 membered nitrogen-containing heterocyclyl, preferably tetrahydropyrrolyl;

$R_7$ is selected from hydrogen or $C_{1-6}$ alkyl, preferably hydrogen or $C_{1-3}$ alkyl, more preferably hydrogen or methyl;

alternatively, $R_7$ is linked with $R_3$ or $R_4$ to form a 5-6 membered nitrogen-containing heterocyclyl, preferably tetrahydropyrrolyl;

alternatively, $R_7$ is linked with $R_5$ or $R_6$ to form a $C_{4-6}$ cycloalkyl or a 4-6 membered heterocyclyl, preferably cyclobutyl;

$R_b$ and $R_c$ are each independently selected from hydrogen, halogen or $C_{1-6}$ alkyl, preferably hydrogen, fluorine, chlorine, bromine or $C_{1-3}$ alkyl, more preferably hydrogen, fluorine, chlorine, bromine or methyl; and

m is 0 or 1.

**10.** The compound represented by general formula (I), a stereoisomer or a pharmaceutically acceptable salt thereof according to claim 1 or 2, wherein the general formula (I) is further represented by general formula (II):

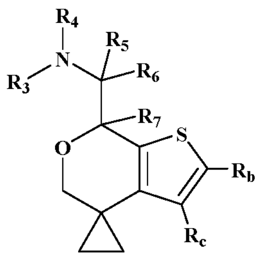

( II )

wherein $R_b$, $R_c$, $R_3$, $R_4$, $R_5$, $R_6$ and $R_7$ are as described in claim 1 or 2.

**11.** The compound represented by general formula (I), a stereoisomer or a pharmaceutically acceptable salt thereof according to claim 1 or 2, wherein the general formula (I) is further represented by general formula (III):

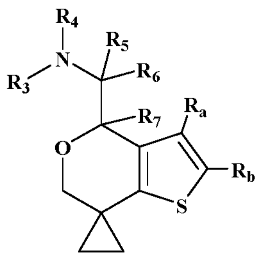

( III )

wherein $R_a$, $R_b$, $R_3$, $R_4$, $R_5$, $R_6$ and $R_7$ are as described in claim 1 or 2.

**12.** The compound represented by general formula (I), a stereoisomer or a pharmaceutically acceptable salt thereof according to any one of claims 1-11, wherein $R_3$ and $R_4$ are not hydrogen at the same time.

**13.** The compound represented by general formula (I), a stereoisomer or a pharmaceutically acceptable salt thereof according to claim 12, wherein $R_3$ is hydrogen; and $R_4$ is selected from $C_{1-3}$ alkyl, $C_{1-3}$ deuterated alkyl or $C_{1-3}$ haloalkyl, preferably methyl, ethyl, isopropyl, $-CD_3$ or $-CH_2CF_3$, more preferably methyl or $-CD_3$.

**14.** The compound represented by general formula (I), a stereoisomer or a pharmaceutically acceptable salt thereof according to any one of claims 1-13, wherein it is selected from the group consisting of:

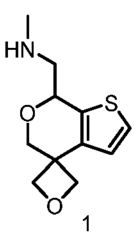 1 , 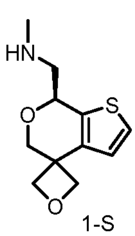 1-S , 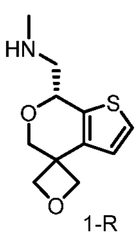 1-R , 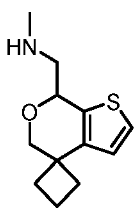 2 , 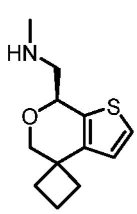 2-S , 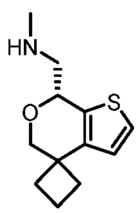 2-R , 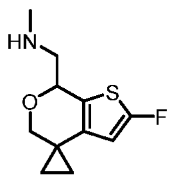 3 ,

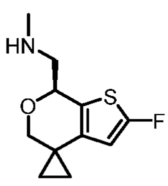 3-S , 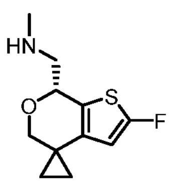 3-R , 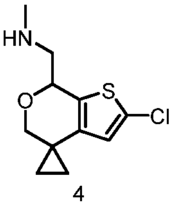 4 , 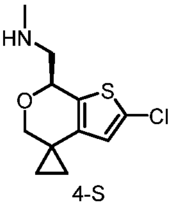 4-S , 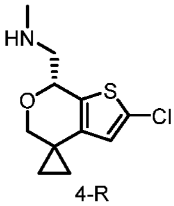 4-R , 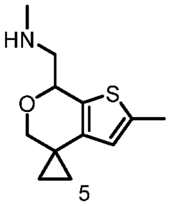 5 ,

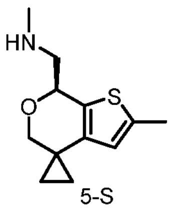 5-S , 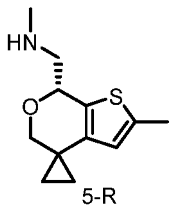 5-R , 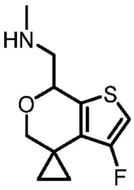 6 , 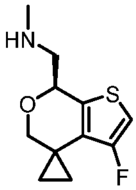 6-S , 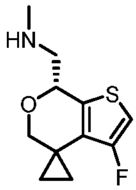 6-R , 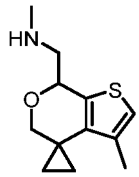 7 , 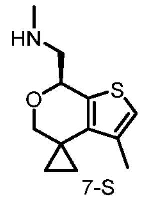 7-S ,

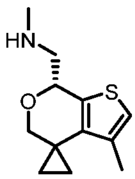 7-R , 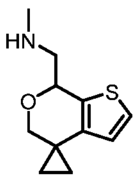 8 , 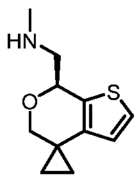 8-S , 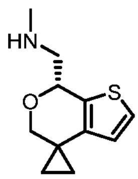 8-R , 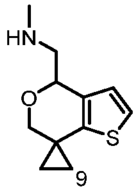 9 , 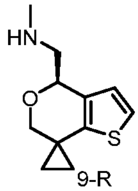 9-R , 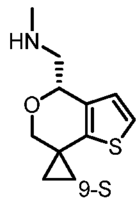 9-S ,

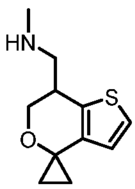 10 , 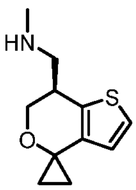 10-R , 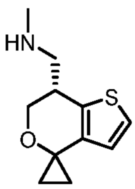 10-S , 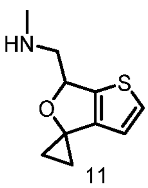 11 , 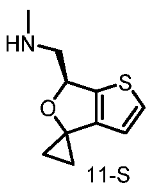 11-S , 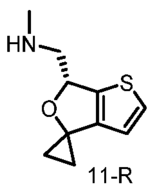 11-R , 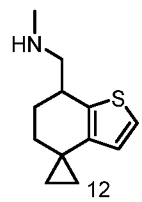 12 ,

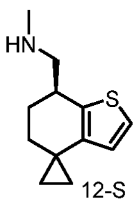 12-S , 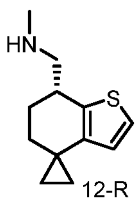 12-R , 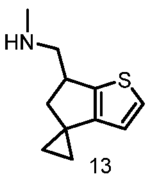 13 , 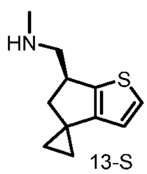 13-S , 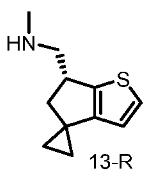 13-R , 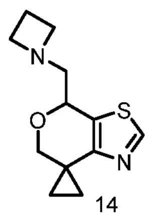 14 , 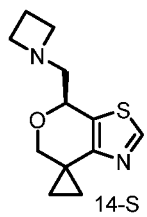 14-S ,

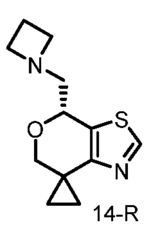 14-R , 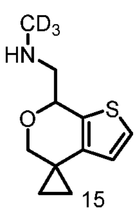 15 , 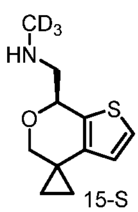 15-S , 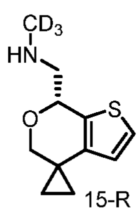 15-R , 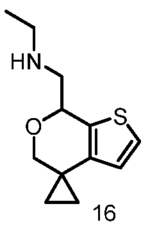 16 , 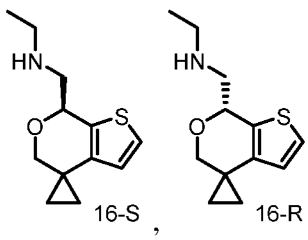 16-S , 16-R ,

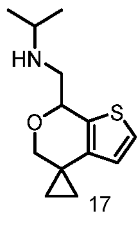 17 , 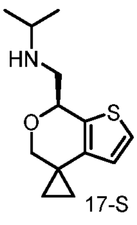 17-S , 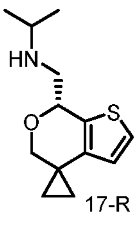 17-R , 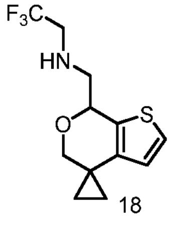 18 , 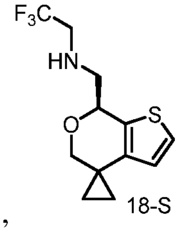 18-S , 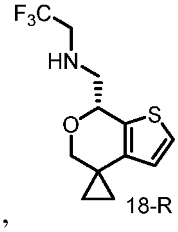 18-R , 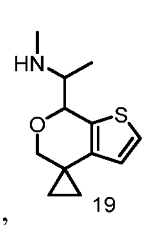 19 ,

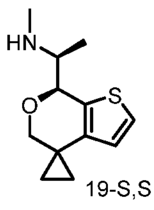 19-S,S , 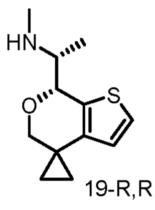 19-R,R , 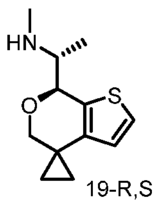 19-R,S , 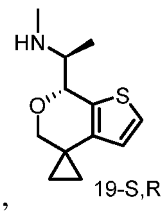 19-S,R , 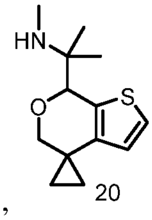 20 , 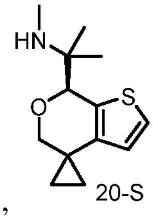 20-S , 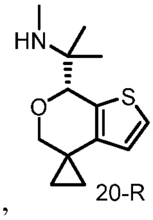 20-R ,

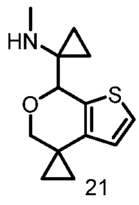 21 , 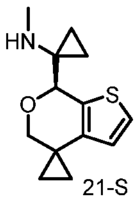 21-S , 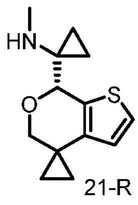 21-R , 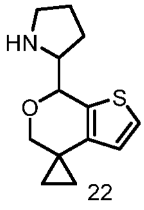 22 , 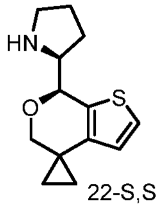 22-S,S , 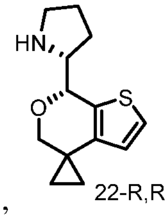 22-R,R , 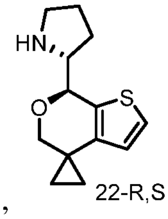 22-R,S ,

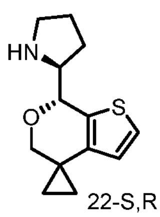 22-S,R , 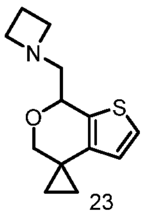 23 , 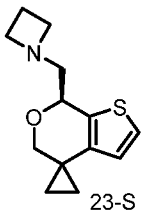 23-S , 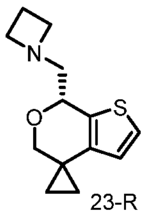 23-R , 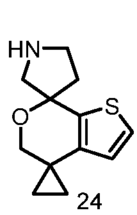 24 , 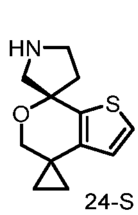 24-S , 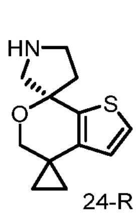 24-R ,

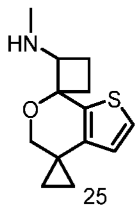 25 , 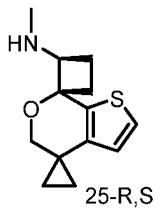 25-R,S , 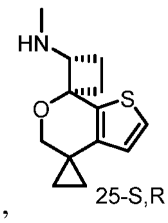 25-S,R , 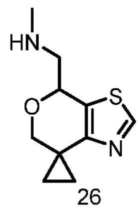 26 , 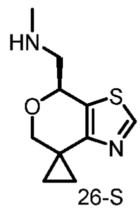 26-S , 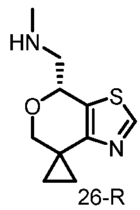 26-R , 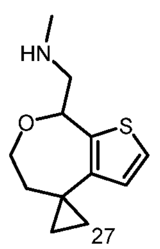 27 ,

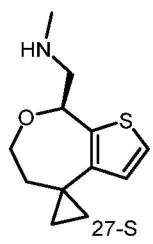 27-S , 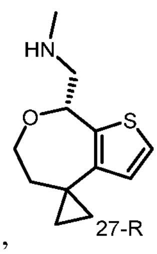 27-R , 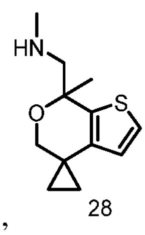 28 , 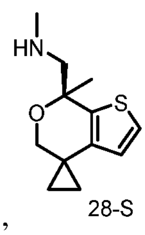 28-S , 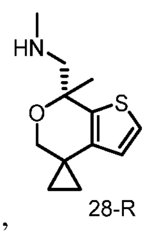 28-R , 29 , 29-S ,

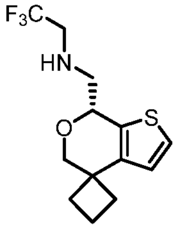 29-R, 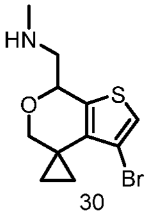 30, 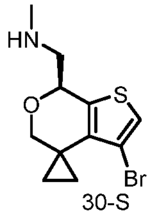 30-S, 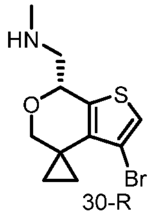 30-R, 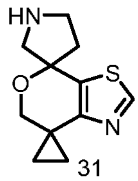 31, 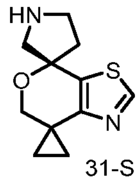 31-S, 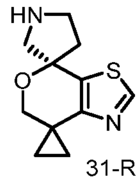 31-R,

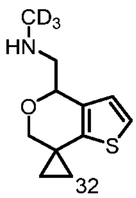 32, 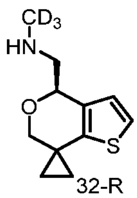 32-R, 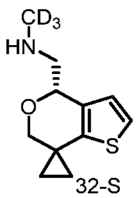 32-S, 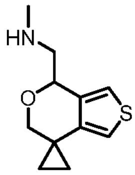 33, 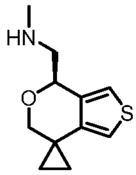 33-R, 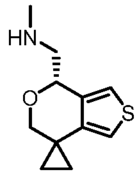 33-S, 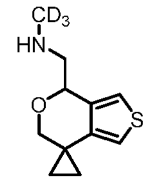 34,

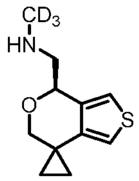 34-R, 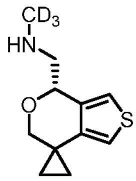 34-S, 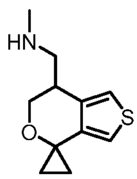 35, 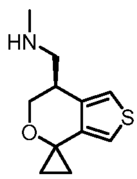 35-R, 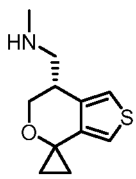 35-S, 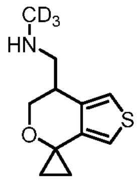 36, 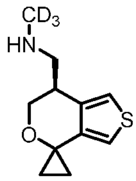 36-R

and

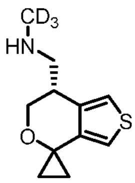 36-S.

**15.** A method for preparing the compound represented by general formula (II-A), a stereoisomer or a pharmaceutically acceptable salt thereof according to claim 9, comprising:

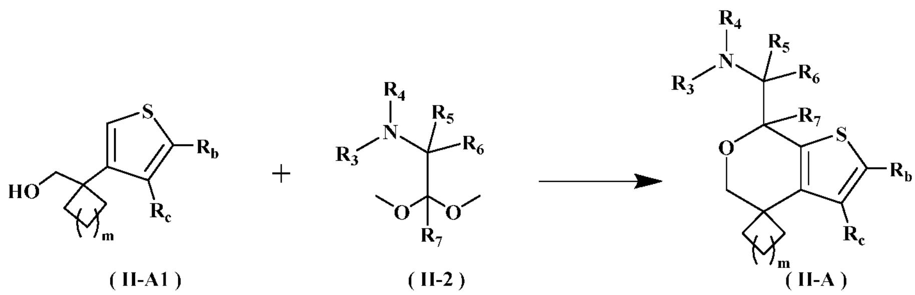

( II-A1 ) ( II-2 ) ( II-A )

reacting a compound of the general formula (II-A1) with a compound of the general formula (II-2) to prepare the compound represented by the general formula (II-A), a stereoisomer or a pharmaceutically acceptable salt thereof;

wherein $R_b$, $R_c$, m, $R_3$, $R_4$, $R_5$, $R_6$ and $R_7$ are as described in claim 9.

**16.** A method for preparing the compound represented by general formula (II), a stereoisomer or a pharmaceutically acceptable salt thereof according to claim 10, comprising:

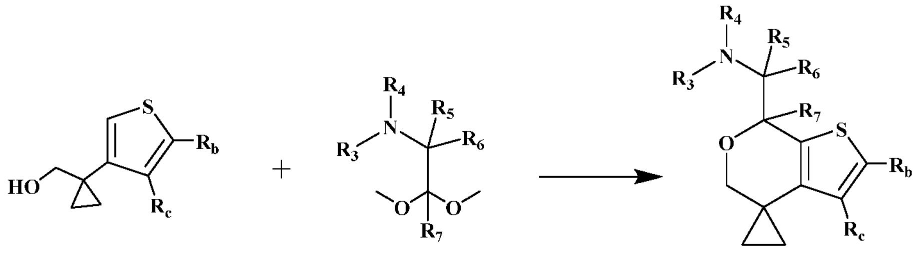

( II-1 ) ( II-2 ) ( II )

reacting a compound of the general formula (II-1) with a compound of the general formula (II-2) to prepare the compound represented by the general formula (II), a stereoisomer or a pharmaceutically acceptable salt thereof; wherein $R_b$, $R_c$, $R_3$, $R_4$, $R_5$, $R_6$ and $R_7$ are as described in claim 10.

**17.** A method for preparing the compound represented by general formula (III), a stereoisomer or a pharmaceutically acceptable salt thereof according to claim 11, comprising:

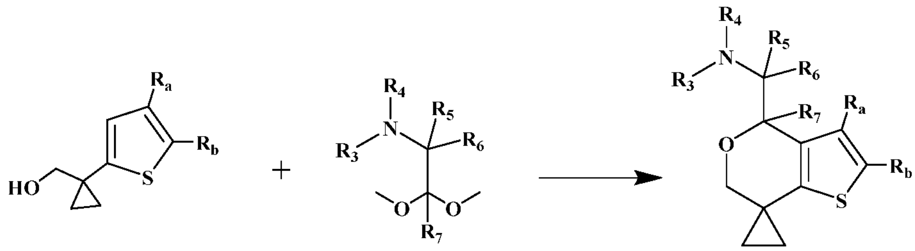

( III-1 ) ( II-2 ) ( III )

reacting a compound of the general formula (III-l) with a compound of the general formula (II-2) to prepare the compound represented by the general formula (III), a stereoisomer or a pharmaceutically acceptable salt thereof; wherein $R_a$, $R_b$, $R_3$, $R_4$, $R_5$, $R_6$ and $R_7$ are as described in claim 11.

**18.** A pharmaceutical composition comprising a therapeutically effective amount of the compound, a stereoisomer or a pharmaceutically acceptable salt thereof according to any one of claims 1-14 and one or more pharmaceutically acceptable carriers.

**19.** Use of the compound, a stereoisomer or a pharmaceutically acceptable salt thereof according to any one of claim 1-14, or the pharmaceutical composition according to claim 18 in the preparation of a medicament for preventing and/or treating a neuropsychiatric disease in mammals; the neuropsychiatric disease is preferably central nervous system disease related to 5-hydroxytryptamine receptors, and/or trace amine-associated receptors, and/or dopamine receptors.

**20.** The use according to claim 19, wherein the neuropsychiatric disease is one or more diseases selected from the group consisting of: schizophrenia, schizophrenia spectrum disease, acute schizophrenia, chronic schizophrenia, NOS schizophrenia, schizoid personality disorder, schizotypal personality disorder, delusional disorder, psychosis, psychotic disorder, brief psychotic disorder, shared psychotic disorder, psychotic disorder due to a physical disease, drug-induced psychosis, psychoaffective disorder, aggression, delirium, Parkinson's psychosis, excitative psychosis, Tourette's syndrome, organic or NOS psychosis, seizure, agitation, post-traumatic stress disorder, behavior disorder, neurodegenerative disease, Alzheimer's disease, Parkinson's disease, dyskinesias, Huntington's disease, dementia, mood disorder, anxiety, affective disorder, depression, major depressive disorder, dysthymia, bipolar disorder, manic disorder, seasonal affective disorder, attention deficit disorder, attention deficit hyperactivity disorder, obsessive-compulsive disorder, vertigo, epilepsy, pain, neuropathic pain, sensitization accompanying neuropathic pain, inflammatory pain, fibromyalgia, migraine, cognitive impairment, movement disorder, restless leg syndrome, multiple sclerosis, sleep disorder, sleep apnea, narcolepsy, excessive daytime sleepiness, jet lag, drowsy side effect of medications, insomnia, substance abuse or dependence, addiction, eating disorder, sexual dysfunction, hypertension, emesis, Lesche-Nyhane disease, Wilson's disease, autism, Huntington's chorea, and premenstrual dysphoria.

## INTERNATIONAL SEARCH REPORT

International application No.
**PCT/CN2022/083485**

### A. CLASSIFICATION OF SUBJECT MATTER

C07D 495/06(2006.01)i； C07D 491/052(2006.01)i； C07D 493/04(2006.01)i； A61K 31/35(2006.01)i； A61K 31/381(2006.01)i； A61P 25/18(2006.01)i； A61P 25/20(2006.01)i； A61P 25/22(2006.01)i； A61P 25/24(2006.01)i； A61P 25/28(2006.01)i

According to International Patent Classification (IPC) or to both national classification and IPC

### B. FIELDS SEARCHED

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, WPI, EPODOC, ISI Web of Knowledge, STN(REGISTRY, MARPAT, CAPLUS): 上海枢境生物科技有限公司, 噻吩, 吡喃, 螺, +thiophen+, +pyran+, +spiro+, structural formula search

### C. DOCUMENTS CONSIDERED TO BE RELEVANT

| Category* | Citation of document, with indication, where appropriate, of the relevant passages | Relevant to claim No. |
|---|---|---|
| X | WO 2021027304 A1 (SHANGHAI HAIYAN PHARMACEUTICAL TECHNOLOGY CO., LTD. et al.) 18 February 2021 (2021-02-18) see embodiments 1-3, 21, 33, 35, 37 | 1-3, 5, 7-11, 15-17 |
| A | CN 102762575 A (SUNOVION PHARMACEUTICALS INC et al.) 31 October 2012 (2012-10-31) abstract, and claims 1-62 | 1-20 |
| A | CN 103068379 A (HOFFMANN LA ROCHE) 24 April 2013 (2013-04-24) claims 1-18 | 1-20 |
| A | US 2010029589 A1 (GALLEY GUIDO et al.) 04 February 2010 (2010-02-04) abstract, claims 1-45 | 1-20 |
| A | CN 101528709 A (HOFFMANN LA ROCHE et al.) 09 September 2009 (2009-09-09) abstract, and claims 1-17 | 1-20 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 June 2022** | **27 June 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/083485**

| Patent document cited in search report | Publication date (day/month/year) | Patent family member(s) | Publication date (day/month/year) |
|---|---|---|---|
| WO 2021027304 A1 | 18 February 2021 | CN 112789276 A | 11 May 2021 |
| CN 102762575 A | 31 October 2012 | CA 2781716 A1 | 09 June 2011 |
| | | JP 6028310 B1 | 16 November 2016 |
| | | IL 243019 A | 30 August 2018 |
| | | RU 2012127770 A | 10 January 2014 |
| | | KR 20150090269 A | 05 August 2015 |
| | | NZ 600008 A | 31 October 2014 |
| | | JP 5710064 B1 | 30 April 2015 |
| | | HK 1212969 A1 | 24 June 2016 |
| | | CN 104193761 A | 10 December 2014 |
| | | IN 1373MUMNP2012 A | 11 October 2013 |
| | | EP 2507245 A2 | 10 October 2012 |
| | | WO 2011069063 A2 | 09 June 2011 |
| | | KR 20120127581 A | 22 November 2012 |
| | | ES 2625330 T3 | 19 July 2017 |
| | | NZ 731621 A | 25 January 2019 |
| | | ZA 201203533 B | 28 August 2013 |
| | | SG 181498 A1 | 30 July 2012 |
| | | KR 20200013071 A | 05 February 2020 |
| | | US 2013109677 A1 | 02 May 2013 |
| | | AU 2010325925 A1 | 07 June 2012 |
| | | US 2020323819 A1 | 15 October 2020 |
| | | JP 2017031198 A | 09 February 2017 |
| | | US 2016083399 A1 | 24 March 2016 |
| | | KR 20170100047 A | 01 September 2017 |
| | | RU 2018100098 A | 09 July 2019 |
| | | IL 243015 A | 31 October 2018 |
| | | IL 220173 D0 | 31 July 2012 |
| | | CN 111560025 A | 21 August 2020 |
| | | KR 20210120133 A | 06 October 2021 |
| | | RU 2641648 C1 | 19 January 2018 |
| | | SG 10201510665 W A | 28 January 2016 |
| | | HK 1247911 A1 | 05 October 2018 |
| | | IL 243018 A | 31 May 2020 |
| | | PT 2507245 T | 22 May 2017 |
| | | JP 2020164541 A | 08 October 2020 |
| | | MX 2012006326 A | 09 October 2012 |
| | | BR 112012013431 A2 | 05 April 2016 |
| | | AU 2016200448 A1 | 18 February 2016 |
| | | IL 243021 A | 30 April 2018 |
| | | JP 2018104454 A | 05 July 2018 |
| | | AU 2017248551 A1 | 09 November 2017 |
| | | JP 2013512926 A | 18 April 2013 |
| | | EP 3252057 A2 | 06 December 2017 |
| | | NZ 626068 A | 24 June 2016 |
| | | JP 2015227348 A | 17 December 2015 |
| | | NZ 711802 A | 30 June 2017 |
| | | US 2021267938 A1 | 02 September 2021 |
| | | SG 10201401661 R A | 30 July 2014 |
| | | IL 272376 D0 | 31 March 2020 |

Form PCT/ISA/210 (patent family annex) (January 2015)

**INTERNATIONAL SEARCH REPORT**
**Information on patent family members**

International application No.
**PCT/CN2022/083485**

| Patent document cited in search report | Publication date (day/month/year) | Patent family member(s) | Publication date (day/month/year) |
|---|---|---|---|
| CN 103068379 A | 24 April 2013 | KR 20130070629 A | 27 June 2013 |
| | | AU 2011287701 A1 | 10 January 2013 |
| | | NZ 604592 A | 27 February 2015 |
| | | MY 166955 A | 25 July 2018 |
| | | MA 34458 B1 | 01 August 2013 |
| | | ZA 201300431 B | 25 September 2013 |
| | | TW 201206912 A | 16 February 2012 |
| | | CR 20120663 A | 01 April 2013 |
| | | EA 201291476 A1 | 28 June 2013 |
| | | EC SP13012414 A | 28 March 2013 |
| | | CL 2013000313 A1 | 03 May 2013 |
| | | JP 2015145408 A | 13 August 2015 |
| | | SG 187125 A1 | 28 February 2013 |
| | | CA 2802935 A1 | 09 February 2012 |
| | | PE 20130497 A1 | 22 April 2013 |
| | | US 2013345201 A1 | 26 December 2013 |
| | | BR 112013002518 A2 | 31 May 2016 |
| | | MX 2013000745 A | 05 March 2013 |
| | | JP 2013536181 A | 19 September 2013 |
| | | WO 2012016879 A1 | 09 February 2012 |
| | | EP 2600853 A1 | 12 June 2013 |
| | | UA 112527 C2 | 26 September 2016 |
| | | AR 082458 A1 | 12 December 2012 |
| | | CO 6761298 A2 | 30 September 2013 |
| | | US 2012028964 A1 | 02 February 2012 |
| US 2010029589 A1 | 04 February 2010 | US 2011144333 A1 | 16 June 2011 |
| | | US 8357712 B2 | 22 January 2013 |
| CN 101528709 A | 09 September 2009 | WO 2008046756 A1 | 24 April 2008 |
| | | ES 2340223 T3 | 31 May 2010 |
| | | RU 2009112495 A | 27 November 2010 |
| | | MX 2009003887 A | 22 April 2009 |
| | | EP 2076496 A1 | 08 July 2009 |
| | | AU 2007312389 A1 | 24 April 2008 |
| | | AR 063326 A1 | 21 January 2009 |
| | | CL 2007002970 A1 | 30 May 2008 |
| | | JP 2010506874 A | 04 March 2010 |
| | | BR PI0718059 A2 | 05 November 2013 |
| | | KR 20090055640 A | 02 June 2009 |
| | | CA 2665255 A1 | 24 April 2008 |
| | | IL 197872 D0 | 24 December 2009 |
| | | ZA 200902279 B | 31 March 2010 |
| | | TW 200835494 A | 01 September 2008 |
| | | PE 20080984 A1 | 19 July 2008 |
| | | DE 602007005671 D1 | 12 May 2010 |
| | | AT 462696 T | 15 April 2010 |
| | | US 2008096906 A1 | 24 April 2008 |
| | | NO 20091480 L | 30 April 2009 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2021103384291
- CN 2021111517346
- CN 2021114966347
- US 2010058884 W